# EUROPEAN PATENT SPECIFICATION

(11) **EP 4 232 060 B1**
(45) Date of publication and mention of the grant of the patent: **01.07.2026**
(21) Application number: 21883760.7
(22) Date of filing: 20.10.2021
(51) Int. Cl.: A61K 9/51, A61K 38/16, A61K 38/17, A61P 7/02, A61P 9/00, A61K 31/428, C07K 14/775

(54) **COMPOSITIONS AND METHODS FOR TREATING CARDIOVASCULAR RELATED DISORDERS**
ZUSAMMENSETZUNGEN UND VERFAHREN ZUR BEHANDLUNG VON HERZ-KREISLAUF-ERKRANKUNGEN
COMPOSITIONS ET MÉTHODES DE TRAITEMENT DE TROUBLES CARDIOVASCULAIRES

(30) Priority: 20.10.2020 US 202063093839 P
(43) Date of publication of application: 30.08.2023
(73) Proprietor: The Regents of the University of Michigan, Ann Arbor, MI 48109 (US)
(72) Inventor: ADILI, Reheman, Ann Arbor, Michigan 48109 (US); HE, Hongliang, Ann Arbor, Michigan 48109 (US); SCHWENDEMAN, Anna, Ann Arbor, Michigan 48109 (US); HOLINSTAT, Michael, Ann Arbor, Michigan 48109 (US)
(74) Representative: Algemeen Octrooi- en Merkenbureau B.V.
(86) International application number: PCT/US2021/055736
(87) International publication number: WO 2022/087058

(56) References cited:
- WO-A1-2015/054662
- WO-A2-2016/154542
- WO-A2-2017/223085
- US-B2- 10 752 581

## Description

### STATEMENT REGARDING FEDERALLY SPONSORED RESEARCH OR DEVELOPMENT

This invention was made with government support under GM131835 awarded by the National Institutes of Health. The government has certain rights in the invention.

### FIELD OF THE INVENTION

The present invention relates to nanoparticles associated with N-2-benzothiazolyl-4-[[(2-hydroxy-3-methoxyphenyl)methyl]amino]-benzenesulfonamide (ML355) configured for treating cardiovascular related disorders. In particular, the present invention is directed to compositions comprising synthetic HDL (sHDL) nanoparticles associated with ML355 configured for treating cardiovascular related disorders (e.g., inhibit platelet aggregation; inhibit thrombosis formation; inhibit vessel occlusion; inhibit platelet associated 12-LOX activity, as well as systems and methods utilizing such sHDL nanoparticles (e.g., therapeutic settings).

### BACKGROUND OF THE INVENTION

Thrombosis is a serious health problem underlying several cardiovascular pathologies, including stroke and myocardial infarction (*1*). Current antithrombotic therapies include 1) anticoagulants, which limit blood clotting; 2) antiplatelets, which inhibit platelet activation and aggregation (*2, 3*); and 3) thrombolytics, which work to dissolve the thrombus once formed (*4-6*). However, adverse effects associated with antithrombotic agents including high risk of hemorrhagic bleeding, slow absorption, poor bioavailability, short half-life, and narrow therapeutic windows, have severely limited the agents' broad application in the clinic (*7-10*). Patent document WO 2015/054662 A1 discloses 4-((2-hydroxy-3-methoxybenzyl)amino) benzenesulfonamide derivatives, useful for treating or preventing 12-lipoxygenase mediated diseases.

With an estimated 48% of U.S. citizens living with cardiovascular disease (*11*), safer and more effective antithrombotic therapies are needed.

The present invention addresses this need.

### SUMMARY

Nanoparticle-based therapies have received notable attention as they offer rapid and targeted delivery of antithrombotic agents to the site of injury and promise to overcome many of the clinical obstacles faced by conventional therapeutic approaches (*12*, *13*). Among them, several thrombus-targeted nanoparticles have been developed, including iron oxide (*14, 15*), polymer-based (*16-18*), and cell membrane-coated (*19*) nanoparticles, to encapsulate a variety of antithrombotic drugs, including antiplatelet agents (*20-22*), anticoagulants (*23, 24*), and thrombolytics (*14, 16, 25, 26*). Despite these advances, most of the existing platforms suffer from particle heterogeneity and complicated fabrication processes that are difficult to scale (*27, 28*).

Experiments conducted during the course of developing embodiments for the present invention developed and utilized synthetic high-density lipoprotein (sHDL) nanoparticles as a drug delivery vehicle (*29*, *30*). Compared to other types of nanoparticles, sHDL offers the advantage of proven clinical safety and established large-scale manufacturing processes (*28, 31*). Furthermore, in addition to their pleiotropic atheroprotective effects (*32-35*), much preclinical and clinical evidence have shown that both native HDL and sHDL have direct effects on attenuating platelet reactivity and inhibiting thrombus formation (*36-38*). Among them, HDL₃, the major HDL subfraction in the blood, was proven to inhibit thrombin-induced platelet aggregation (*39*). The infusion of the plasma purified HDL, CSL-111 (80 mg/kg) to individuals with type 2 diabetes mellitus resulted in a widespread attenuation of platelet activation and a 50% reduction in thrombus formation under flow (*40*). Similarly, the administration of a recombinant ApoA1 Milano-based sHDL, ETC-216, reduced platelet aggregation and thrombus formation on an occlusive platelet-fibrin-rich thrombus rat model (*38*). These findings suggest that sHDL holds promise in acting as an effective antithrombotic vehicle for delivery and exerting a synergistic inhibitory effect on platelet hyperactivity when delivering antiplatelet agents.

Such experiments investigated the ability of encapsulating ML355 (N-2-benzothiazolyl-4-[[(2-hydroxy-3-methoxyphenyl)methyl]amino]-benzenesulfonamide; 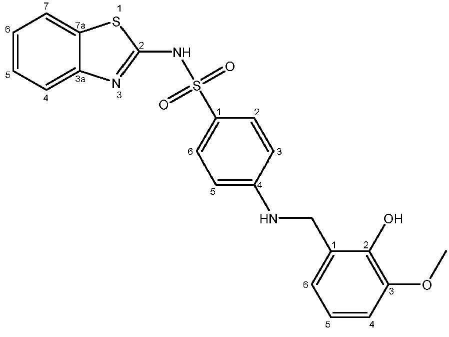 ) in nano-based delivery systems to achieve targeted drug delivery at the site of vascular injury to enhance the drug's therapeutic index and to further expand its broader therapeutic applications against thrombosis and other diseases (*41, 42*).

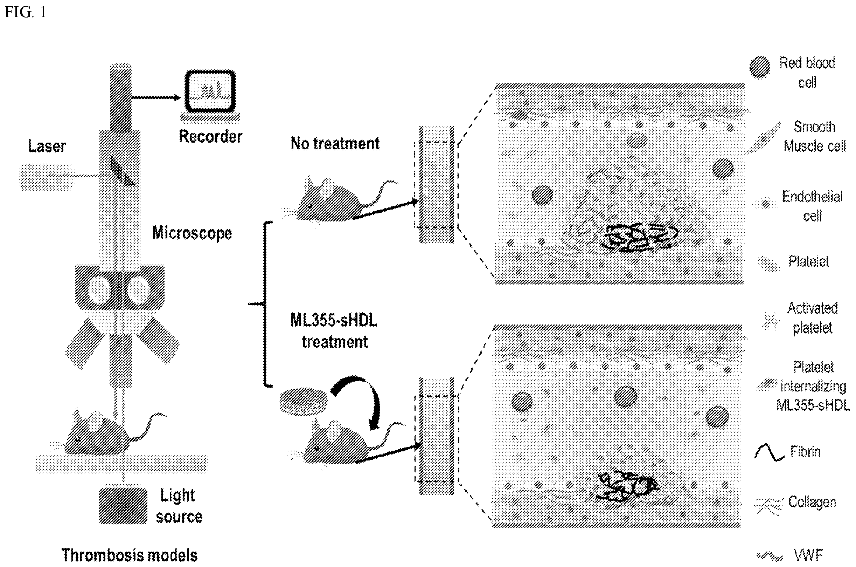

To fulfill this aim, experiments were conduced that developed sHDL nanoparticles that consist of apoA1 mimetic peptide 22A and phospholipids. The sHDL platform exhibited sustained release of ML355 *in vitro* and a superior pharmacokinetic profile *in vivo.* Incubation of sHDL with isolated human platelets resulted in robust uptake of sHDL by platelets and attenuation of platelet function *in vitro.* Intravenous administration of sHDL blank or loaded with ML355 in mice resulted in rapid uptake of sHDL by platelets and retained within platelets *in vivo* for up to 72 hours following intravenous administration. Given the intrinsic antithrombotic properties of endogenous HDL, it was hypothesized that sHDL would also possess antithrombotic properties. Indeed, it was found that sHDL alone was capable of attenuating platelet thrombosis *in vivo* and that our newly generated ML355-sHDL exhibited synergetic antithrombotic effects of both sHDL and ML355 as examined in a laser-induced thrombosis mouse model, without impairing the normal hemostatic property of platelets. Together, these results demonstrate the entrapment of an antithrombotic agent by sHDL provides an effective, feasible and rapidly translatable strategy for the prevention of thrombotic events (see, Fig. 1).

Accordingly, the present invention relates to a composition according to claim 1 and a composition for use according to claim 6. In particular, the present invention is directed to compositions comprising synthetic HDL (sHDL) nanoparticles carrying ML355 configured for treating cardiovascular related disorders (e.g., inhibit platelet aggregation; inhibit thrombosis formation; inhibit vessel occlusion; inhibit platelet associated 12-LOX activity, as well as systems and methods utilizing such sHDL nanoparticles (e.g., therapeutic settings).

In some embodiments, the sHDL-ML355 is configured to treat a cardiovascular disorder. In some embodiments, the sHDL-ML355 is configured to inhibit platelet aggregation. In some embodiments, the sHDL-ML355 is configured to inhibit thrombosis formation. In some embodiments, the sHDL-ML355 is configured to inhibit vessel occlusion. In some embodiments, the sHDL-ML355 is configured to inhibit platelet associated 12-LOX activity.

In some embodiments, the molar ratio of the HDL apolipoprotein component to the lipid component is about 2:1 to 200:1.

In some embodiments, the lipid component comprises a combination of one or any combination of sphingomyelin (SM), D-erythrose-sphingomyelin, D-erythrose dihydrosphingomyelin, palmitoylsphingomyelin, lysophospholipids, galactocerebroside, gangliosides, cerebrosides, glycerides, triglycerides, diglycerides, small alkyl chain phospholipids, phosphatidylcholine, egg phosphatidylcholine, soybean phosphatidylcholine, dipalmitoylphosphatidylcholine (DPPC), dimyristoylphosphatidylcholine, 1-palmitoyl-2-oleoyl-phosphatidylcholine (POPC), 1,2-dimyristoyl-sn-glycero-3-phosphatidylcholine (DMPC), 1,2-distearoyl-sn-glycero-3-phosphatidylcholine (DSPC), distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, dioleoylphosphatidylcholine dioleophosphatidylethanolamine, dilauroylphosphatidylglycerol phosphatidylcholine, phosphatidylserine, phosphatidylethanolamine, phosphatidylinositol, phosphatidylglycerols, diphosphatidylglycerols such as dimyristoylphosphatidylglycerol, dipalmitoylphosphatidylglycerol, distearoylphosphatidylglycerol, dioleoylphosphatidylglycerol, dimyristoylphosphatidic acid, dipalmitoylphosphatidic acid, dimyristoylphosphatidylethanolamine, dipalmitoylphosphatidylethanolamine, ceramides, a phosphatidylserine, dimyristoylphosphatidylserine, dipalmitoylphosphatidylserine, brain phosphatidylserine, brain sphingomyelin, egg sphingomyelin, milk sphingomyelin, palmitoyl sphingomyelin, phytosphingomyelin, dipalmitoylsphingomyelin, distearoylsphingomyelin, dipalmitoylphosphatidylglycerol salt, phosphatidic acid, galactocerebroside, gangliosides, cerebrosides, dilaurylphosphatidylcholine, (1,3)-D-mannosyl-(1,3)diglyceride, aminophenylglycoside, 3-cholesteryl-6'-(glycosylthio)hexyl ether glycolipids, and cholesterol and its derivatives, lyso-phosphotydyl choline, lyso-sphingomyelin, dioleoyl-sn-glycero-3-phosphoethanolamine-N-[3-(2-pyridyldithio) propionate] (DOPE-PDP), 1,2-dipalmitoyl-sn-glycero-3-phosphothioethanol, 1,2-di-(9Z-octadecenoyl)-*sn*-glycero-3-phosphoethanolamine-N-[4-(p-maleimidophenyl)butyramide], 1,2-dihexadecanoyl-*sn*-glycero-3-phosphoethanolamine-N-[4-(p-maleimidophenyl)butyramide], 1,2-dihexadecanoyl-*sn-*glycero-3-phosphoethanolamine-N-[4-(p-maleimidomethyl)cyclohexane-carboxamide], 1,2-di-(9Z-octadecenoyl)-*sn*-glycero-3-phosphoethanolamine-N-[4-(p-maleimidomethyl)cyclohexane-carboxamide], Lyso phoshphatidic acid, Lyso phosphatidylcholine, OA-NO₂ (nitrated oleic acid 9- and 10- nitro-*cis*-octedecenolic acids), LNO₂ (nitrated linoleic Acid 9-, 10-, 12-and 13-nitro-*cis*-octedecadienoic acids), AA-NO₂ (nitrated Arachidonic Acid 5-, 6-, 8-, 9-, 11-, 12-, 14,-and 15-nitro-cis-eicosatetraenoic acids), CLNO₂ (nitrated cholesteryl linoleate cholestaryl-9-, 10-, 12- and 13-nitro-*cis-*octedecadiencates), fatty acid, omega-3 polyunsaturated fatty acids, hexadecatrienoic acid (HTA; 16:3 (n-3); all-cis-7,10,13-hexadecatrienoic acid), α-Linolenic acid (ALA; 18:3 (n-3); all-cis-9,12,15-octadecatrienoic acid), stearidonic acid (SDA; 18:4 (n-3); all-cis-6,9,12,15-octadecatetraenoic acid), eicosatrienoic acid (ETE; 20:3 (n-3); all-cis-11,14,17-eicosatrienoic acid), eicosatetraenoic acid (ETA; 20:4 (n-3); all-cis-8,11,14,17-eicosatetraenoic acid), eicosapentaenoic acid (EPA; 20:5 (n-3); all-cis-5,8,11,14,17-eicosapentaenoic acid), heneicosapentaenoic acid (HPA; 21:5 (n-3); all-cis-6,9,12,15,18-heneicosapentaenoic acid); docosapentaenoic acid (DPA; clupanodonic acid; 22:5 (n-3); all-cis-7,10,13,16,19-docosapentaenoic acid), docosahexaenoic acid (DHA; 22:6 (n-3); all-cis-4,7,10,13,16,19-docosahexaenoic acid), tetracosapentaenoic acid; 24:5 (n-3); all-cis-9,12,15,18,21-tetracosapentaenoic acid), tetracosahexaenoic acid (Nisinic acid; 24:6 (n-3), all-cis-6,9,12,15,18,21-tetracosahexaenoic acid), sphingosine-1-phosphate analogs, sphingosine-1-phosphate antagonists, sphingosine-1-phosphate agonists, sphingosine-1-phosphate receptor agonists, sphingosine-1-phosphate receptor antagonists, and sphingosine-1-phosphate receptor analogs.

In some embodiments, the lipid component comprises neutral phospholipids, negatively charged phospholipids, positively charged phospholipids, or a combination thereof. In some embodiments, the fatty acid chains on the phospholipids are preferably from 12 to 26 or 16 to 26 carbons in length and can vary in degree of saturation from saturated to mono-unsaturated.

The HDL apolipoprotein component is selected from the group consisting of apolipoprotein A-I (apo A-I), apolipoprotein A-II (apo A-II), apolipoprotein A-II xxx (apo A-II-xxx), apolipoprotein A4 (apo A4), apolipoprotein Cs (apo Cs), apolipoprotein E (apo E), apolipoprotein A-I milano (apo A-I-milano), apolipoprotein A-I paris (apo A-I-paris), apolipoprotein M (apo M), an HDL apolipoprotein mimetic, preproapoliprotein, preproApoA-I, proApoA I, preproApoA-II, proApoA II, preproApoA-IV, proApoA-IV, ApoA-V, preproApoE, proApoE, preproApoA I_{Milano}, proApoA-I_{Milano}, preproApoA-I_{Paris}, proApoA-I_{Paris}, and mixtures thereof.

The ApoA-I mimetic is described by any of SEQ ID NOs: 1-336 and WDRVKDLATVYVDVLKDSGRDYVSQF (SEQ ID NO: 337), LKLLDNWDSVTSTFSKLREOL (SEQ ID NO: 338), PVTOEFWDNLEKETEGLROEMS (SEQ ID NO: 339), KDLEEVKAKVQ (SEQ ID NO: 340), KDLEEVKAKVO (SEQ ID NO: 341), PYLDDFQKKWQEEMELYRQKVE (SEQ ID NO: 342), PLRAELQEGARQKLHELOEKLS (SEQ ID NO: 343), PLGEEMRDRARAHVDALRTHLA (SEQ ID NO: 344), PYSDELRQRLAARLEALKENGG (SEQ ID NO: 345), ARLAEYHAKATEHLSTLSEKAK (SEQ ID NO: 346), PALEDLROGLL (SEQ ID NO: 347), PVLESFKVSFLSALEEYTKKLN (SEQ ID NO: 348), PVLESFVSFLSALEEYTKKLN (SEQ ID NO: 349), PVLESFKVSFLSALEEYTKKLN (SEQ ID NO: 350), TVLLLTICSLEGALVRRQAKEPCV QTVTDYGKDLME (SEQ ID NO: 351), KVKSPELOAEAKSYFEKSKE (SEQ ID NO: 352), VLTLALVAVAGARAEVSADOVATV (SEQ ID NO: 353), NNAKEAVEHLOKSELTOOLNAL (SEQ ID NO: 354), LPVLVWLSIVLEGPAPAOGTPDVSS (SEQ ID NO: 355), LPVLVVVLSIVLEGPAPAQGTPDVSS (SEQ ID NO: 356), ALDKLKEFGNTLEDKARELIS (SEQ ID NO: 357), VVALLALLASARASEAEDASLL (SEQ ID NO: 358), HLRKLRKRLLRDADDLQKRLAVYOA (SEQ ID NO: 359), AQAWGERLRARMEEMGSRTRDR (SEQ ID NO: 360), LDEVKEQVAEVRAKLEEQAQ (SEQ ID NO: 361), DWLKAFYDKVAEKLKEAF (SEQ ID NO: 362), DWLKAFYDKVAEKLKEAFPDWAKAAYDKAAEKAKEAA (SEQ ID NO: 363), PVLDLFRELLNELLEALKQKL (SEQ ID NO: 364), PVLDLFRELLNELLEALKQKLA (SEQ ID NO: 365), PVLDLFRELLNELLEALKQKLK (SEQ ID NO: 366), PVLDLFRELLNELLEALKQKLA (SEQ ID NO: 367), PVLDLFRELLNELLEALKKLLK (SEQ ID NO: 368), PVLDLFRELLNELLEALKKLLA (SEQ ID NO: 369), and PLLDLFRELLNELLEALKKLLA (SEQ ID NO: 370).

In some embodiments, the ratio of HDL apolipoprotein component to lipid component is at or between 1:1 to 1:4 wt/wt. In some embodiments, the ratio of HDL apolipoprotein component to lipid component is at or between 1:1.5 to 1:3 wt/wt. In some embodiments, the ratio of HDL apolipoprotein component to lipid component is 1:2 wt/wt.

In some embodiments, the sHDL nanoparticle has less than 5% free lipid component impurity. In some embodiments, the sHDL nanoparticle has less than 20% free HDL apolipoprotein component impurity.

In some embodiments, approximately 25% of the lipid component is cholesterol and/or cholesterol ester. In some embodiments, approximately 10% of the lipid component is cholesterol and/or cholesterol ester. In some embodiments, approximately 5% of the lipid component is cholesterol and/or cholesterol ester. In some embodiments, approximately 1% of the lipid component is cholesterol and/or cholesterol ester.

In some embodiments, the composition comprising sHDL is at least 90%, at least 92.5%, at least 95%, at least 96%, at least 97% or at least 98% pure.

In some embodiments, the composition comprising sHDL is at least 80%, at least 85%, at least 90% or at least 95% homogeneous, as reflected by a single peak in gel permeation chromatography.

In some embodiments, at least 80%, at least 85%, at least 90% or at least 95% of the sHDL nanoparticles range 4 nm to 12 nm in size, 6 nm to 12 nm in size, or 8 nm to 12 nm in size, as measured by GPC or DLS.

In some embodiments, at least 95%, at least 96%, at least 97%, at least 98% or at least 99% of the HDL apolipoprotein component is in complexes.

The sHDL-ML355 nanoparticles are not limited to a particular size. In some embodiments, the average particle size of the sHDL-ML355 nanoparticle is between 6-20 nm (e.g., 6-14) (e.g., 8-10 nm).

In some embodiments, an imaging agent (e.g., a lipophilic near infrared fluorescent dye or a nuclear imaging agent) is contained within the sHDL-ML355 mixture of at least one phospholipid, ML355, and at least one HDL apolipoprotein. In some embodiments, the lipophilic near infrared fluorescent dye is DiD.

In certain embodiments, the present invention provides compositions for use in methods of preventing, attenuating or treating thrombosis in a subject having or at risk for having conditions and symptoms caused by thrombosis, comprising administering to the subject such a composition comprising one or more sHDL-ML355 moieties. In some embodiments, administration of the composition results in, for example, reduction of platelet activity, reduction of platelet aggregation, prevention of thrombus formation, reduction of vessel occlusion, and reduction of platelet associated 12-LOX activity.

In some embodiments, the conditions and symptoms caused by thrombosis are related to a venous thrombosis. In some embodiments, the conditions and symptoms caused by thrombosis are related to an arterial thrombosis.

In some embodiments, thrombosis is a feature of an underlying disease or condition. Non-limiting examples of such disease or condition include acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, thromboembolic stroke, systemic embolism, ischemic stroke, venous thromboembolism, atrial fibrillation, non-valvular atrial fibrillation, atrial flutter, transient ischemic attacks, venous thrombosis, deep venous thrombosis, pulmonary embolus, coagulopathy, disseminated intravascular coagulation, thrombotic thrombocytopenic purpura, thromboanglitis obliterans, thrombotic disease associated with heparin-induced thrombocytopenia, thrombotic complications associated with extracorporeal circulation, thrombotic complications associated with instrumentation, thrombotic complications associated with the fitting of prosthetic devices, occlusive coronary thrombus formation resulting from either thrombolytic therapy or percutaneous transluminal coronary angioplasty, thrombus formation in the venous vasculature, disseminated intravascular coagulopathy, a condition wherein there is rapid consumption of coagulation factors and systemic coagulation which results in the formation of life-threatening thrombi occurring throughout the microvasculature leading to widespread organ failure, hemorrhagic stroke, renal dialysis, blood oxygenation, and cardiac catheterization.

In some embodiments, the conditions and symptoms caused by thrombosis are selected from the group consisting of embolic stroke, thrombotic stroke, venous thrombosis, deep venous thrombosis, acute coronary syndrome, and myocardial infarction.

In some embodiments for preventing, attenuating or treating a subject having or at risk for having conditions and symptoms caused by thrombosis, the composition comprising one or more sHDL-ML355 moeities is co-administered with one or more of the following therapeutic agents: heparin; tPA; anistreplase; streptokinase; urokinase; a coumadin; warfarin; idraparinux; fondaparinux; aspririn; an adenosine diphosphate receptor inhibitor; a phosphodiesterase inhibitor; a glycoprotein IIB/IIA inhibitor; an adenosine reuptake inhibitor; and a thromboxane receptor antagonist.

In such embodiments for preventing, attenuating, and/or treating thrombosis in a subject, the administering to the subject a therapeutically effective amount of a composition comprising one or more sHDL-ML355 moeities comprises a continuous infusion of the composition and/or a non-continuous infusion of the composition.

In some embodiments, the subject is a human being.

In certain embodiments, the present invention provides compositions for use in methods of preventing, attenuating or treating a subject (e.g., a human subject) having a cardiovascular related disorder, comprising administering to the subject a therapeutically effective amount of such a composition comprising one or more sHDL-ML355 moieties.

In certain embodiments, the present invention provides compositions for use in methods of preventing, attenuating or treating increased platelet activity in a subject (e.g., a human subject) having or at risk for having increased platelet activity, comprising administering to the subject such a composition comprising one or more sHDL-ML355 moieties.

In certain embodiments, the present invention provides compositions for use in methods of preventing, attenuating or treating platelet aggregation in a subject (e.g., a human subject) having or at risk for having platelet aggregation, comprising administering to the subject such a composition comprising one or more sHDL-ML355 moieties.

In certain embodiments, the present invention provides compositions for use in methods of preventing, attenuating or treating vessel occlusion (e.g., arterial and/or venous) in a subject (e.g., a human subject) having or at risk for having vessel occlusion, comprising administering to the subject such a composition comprising one or more sHDL-ML355 moieties.

In certain embodiments, the present invention provides compositions for use in methods of preventing, attenuating or treating increased platelet associated 12-LOX activity in a subject (e.g., a human subject) having or at risk for having increased platelet associated 12-LOX activity, comprising administering to the subject such a composition comprising one or more sHDL-ML355 moieties.

Additional embodiments will be apparent to persons skilled in the relevant art based on the teachings contained herein.

### BRIEF DESCRIPTION OF THE DRAWINGS

FIG. 1: Schematic demonstrating that encapsulation of antiplatelet agent ML355 into sHDL (ML355-sHDL) exerts synergistic antithrombotic effects of both ML355 and sHDL, thus efficiently inhibiting thrombus formation.

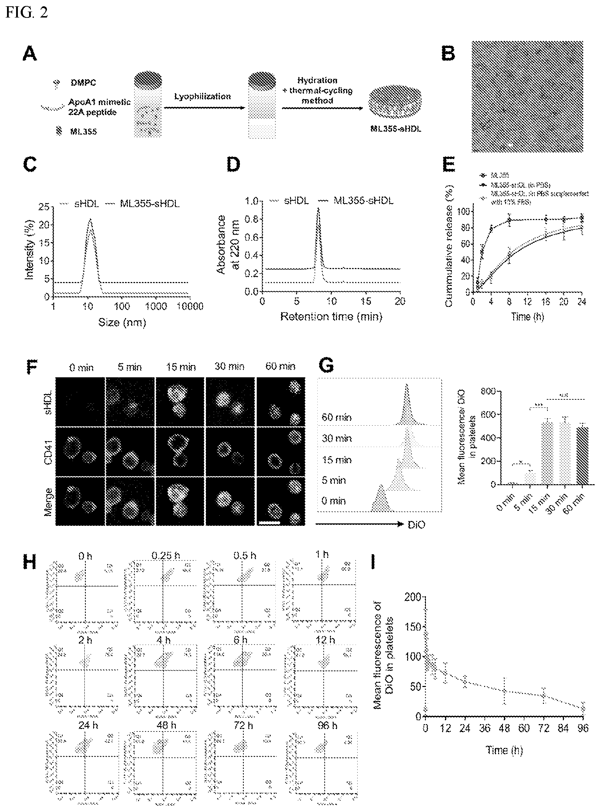

FIG. 2: Preparation and characterization of ML355-sHDL. (**A**) Illustration of the synthesis of ML355-sHDL composed of an ApoA1 mimetic 22-mer peptide (22A), 1,2-dimyristoyl-sn-glycero-3-phosphocholine (DMPC) and ML355 using the thermal-cycling method. (**B**) Negative-stained transmission electron microscopy image of ML355-sHDL, scale bar is 20 nm. (**C**) Dynamic light scattering of blank sHDL and ML355-sHDL. (**D**) Gel permeation chromatography of blank sHDL and ML355-sHDL. (**E**) Release of ML355 from sHDL at PBS and PBS supplemented with 10% Fetal Bovine Serum (FBS). Data represents mean ± SD (n = 3). (**F**) Platelet uptake and intracellular distribution of DiO-sHDL in platelets imaged by confocal microscopy (scale bar = 2 µm). Washed mouse platelets were suspended in 1 mL Tyrode's buffer at 3×10⁶ platelets/mL and incubated with DiO-sHDL (sHDL at 50 µg/mL and DiO at 2.5 µg/mL) for indicated lengths of time (5, 15, 30 and 60 minutes) at 37 °C. Platelets were then washed with Tyrode's buffer twice to remove free DiO-sHDL and then fixed with 4% paraformaldehyde, followed by staining with Alexa Fluor 647 conjugated anti-mouse CD41 antibody. Platelet membrane is shown in red and sHDL particle accumulation in platelets are shown in green. (**G**) Representative flow cytometry histograms and data analysis of mean DiO fluorescent intensity in platelets by flow cytometry. Data shown as mean ± SD (n = 3). **P*<0.05 and N.S. (no significant difference). (**H**) CD41-PE positive platelets in the blood versus DiO-sHDL. (**I**) Quantification of DiO-sHDL uptake by CD41-PE positive platelets in blood. Data are represented as mean ± SD (n = 4).

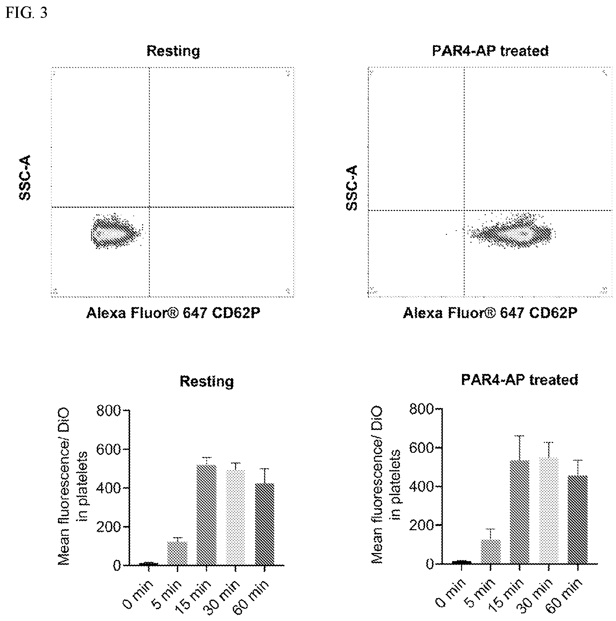

FIG. 3: The comparison of sHDL uptake by washed mouse platelets (resting) and activated mouse platelets treated with PAR4-AP. Both unstimulated washed mouse platelets and activated mouse platelets pre-treated with PAR4-AP (50 µM) were incubated with DiO-sHDL (sHDL at 50 µg/mL and DiO at 2.5 µg/mL) for indicated lengths of time (5, 15, 30 and 60 minutes) at 37 °C. Platelets were then washed with Tyrode's buffer twice to remove free DiO-sHDL and then fixed with 4% paraformaldehyde, followed by staining with Alexa Fluor 647 conjugated anti-mouse CD41 antibody. Platelet membrane is shown in red and sHDL particle accumulation in platelets is shown in green. Representative flow cytometry scatterplots for Alexa Fluor^{®} 647 CD62P fluorescent intensity in platelets and data analysis of mean DiO fluorescent intensity in platelets by flow cytometry.

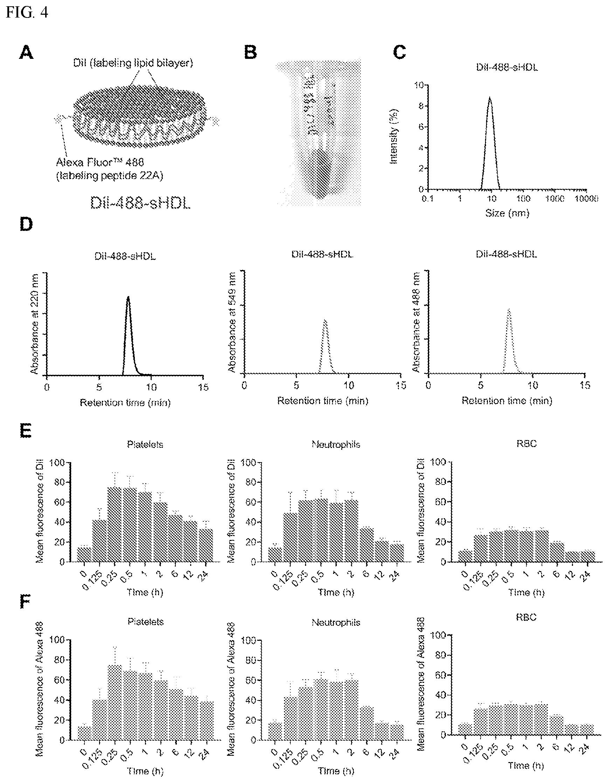

FIG. 4: The distribution of Dil-488-sHDL in major blood cells. **A.** Illustration of dual labeled Dil-488-sHDL was prepared by labeling 22A peptide in sHDL with AlexaFluor 488 dye using Invitrogen protein labeling kit and labeling lipid bilayer in sHDL with cell-labeling fluorophore Dil. (**B**) Photo, (**C**) dynamic light scattering and (**D**) gel permeation chromatography of Dil-488-sHDL at multiple absorbance wavelength 220, 488 and 561 nm, respectively. Photo Credit of Dil-488-sHDL: Hongliang He, The University of Michigan. For investigation of biodistribution of Dil-488-sHDL over time course among major blood cell types, including platelets, neutrophils and red blood cells in mouse, both male and female C57BL/6J mice were intravenously dosed with Dil-488-sHDL (Dil at 0.5 mg/kg and Alexa Fluor 488 at 0.5 mg/kg). At different time points (from 5 minutes up to 24 hours), whole blood were collected and mean fluorescent intensity of both lipid tracer Dil (**E**) and peptide tracer Alexa Fluor 488 (**F**) in each cell type were analyzed by flow cytometry. Data shown as mean ± SD (n = 4).

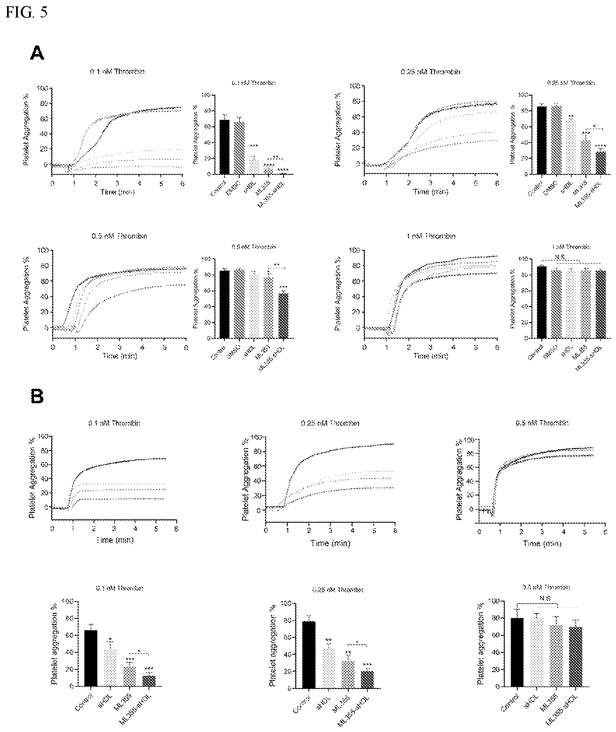

FIG. 5: ML355-sHDL treatment inhibits both human and mouse platelet aggregation. (**A**) Washed human platelets were incubated with different ML355 formulations for 15 minutes, including DMSO (equivalent amount used to dissolve ML355), ML355 (10 µM), sHDL (100 µg/mL) or ML355-sHDL (sHDL at 100 µg/mL and ML355 at 10 µM). Untreated platelets were included as control. After different treatments, platelet aggregation was measured by the addition of thrombin at various concentrations. Compared to both control and DMSO group, both ML355 and sHDL treatment inhibited platelet aggregation. Furthermore, encapsulation of ML355 into sHDL exhibited an improved inhibitory effect on human platelet aggregation at multiple thrombin concentrations compared to treatment with either ML355 or sHDL. Inhibition of aggregation was overwhelmed for all formulations by a high concentration (1 nM) of thrombin. Data shown as mean ± SD (n = 3). **P* < 0.05, ***P <* 0.01, ****P* < 0.001, and N.S. (no significant difference) relative to control or as otherwise indicated. (**B**) Mice were intravenously administered with saline control, ML355 (1.5 mg/kg), sHDL (50 mg/kg), or ML355-sHDL (sHDL at 50 mg/kg and ML355 at 1.5 mg/kg) for 24 hours followed by platelet isolation. Washed mouse platelets were subjected to aggregation analysis by incubation with various concentrations of thrombin. Platelets isolated from mice treated with ML355-sHDL exhibited less aggregation following incubation with thrombin (0.1 and 0.25 nM thrombin) than platelets from the mice treated with other formulations. Inhibition of aggregation was overwhelmed for all formulations by a high concentration (0.5 nM) of thrombin. Data shown as mean ± SD (n = 4). **P* < 0.05, ***P* < 0.01, ****P* < 0.001 and N.S. (no significant difference) relative to control or as otherwise indicated.

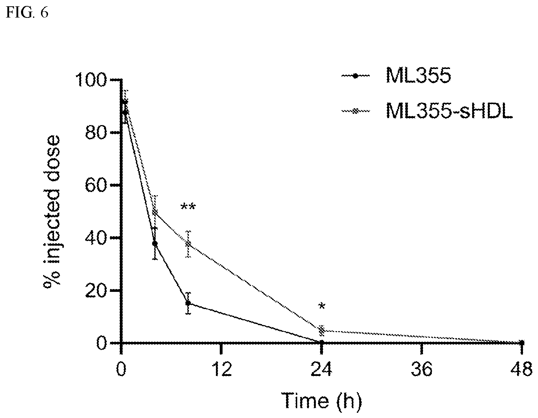

FIG. 6: ML355-sHDL increased the blood circulation of ML355 in mice. A single intravenous injection of ML355 (3 mg/kg) or ML355-sHDL (sHDL at 100 mg/kg and ML355 at 3 mg/kg) in mice. Data shown as mean ± SD (n = 4). **P* < 0.05 and ***P* < 0.01.

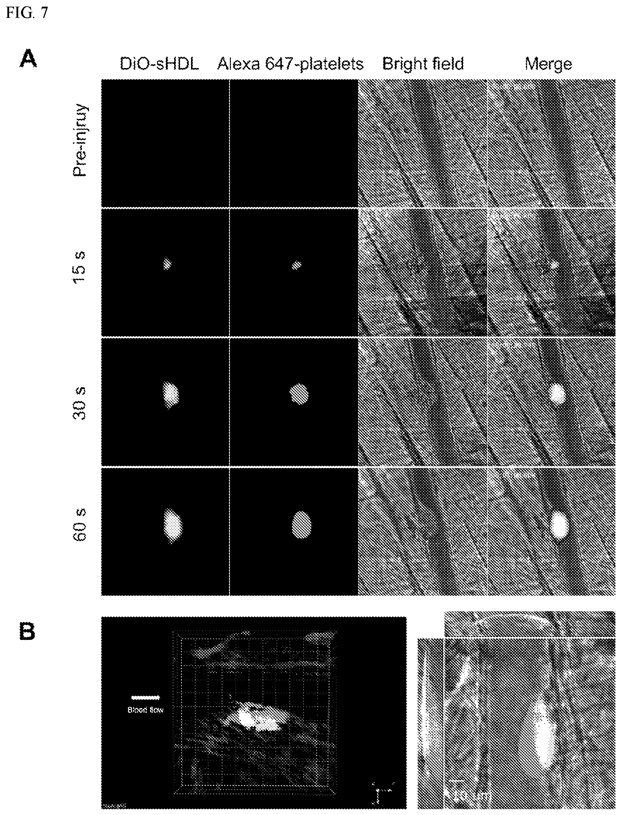

FIG. 7: sHDL targets thrombus *in vivo.* Male mice were pretreated with DiO-sHDL via IV at 50 mg/kg of sHDL and 2.5 mg/kg of DiO. After 24 hours, mice were anesthetized and surgically prepared as described in detail in the method section. DyLight 647-conjugated rat anti-mouse platelet GP1bβ antibody (0.1 µg/g, X649; EMFRET Analytics) was administered by jugular vein cannula prior to vascular injury. Multiple independent thrombi (8-10) were induced in the arterioles (30-50 µm diameter) per mouse (three mice in each group) by a laser ablation system as described in the method section. Images of thrombus formation at the site of injured arterioles were acquired in real-time under 63X water-immersion objective with a Zeiss Axio Examiner Z1 fluorescent microscope. (**A**) Sequence of intravital fluorescence microscopic images recorded over 1 minute showing accumulation of fluorescently labeled DiO-sHDL in a forming thrombus indicated by fluorescently labeled platelets. (**B**) Left panel: Representative three-dimensional confocal images of DiO-sHDL (green) and platelet accumulation (red) in stable thrombi recorded under confocal intravital microscopy. Right panel: Representative middle section of thrombus shown under three direction view.

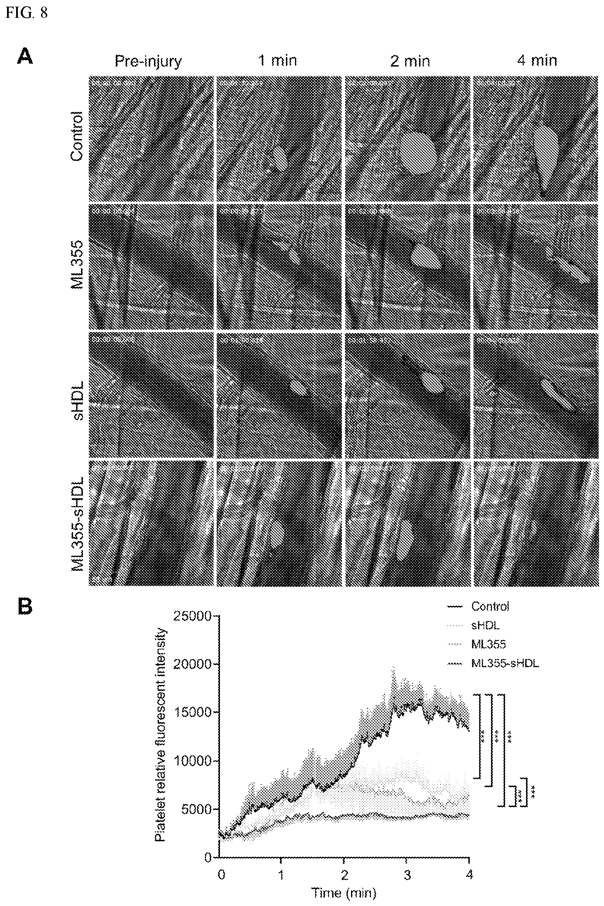

FIG. 8: ML355-sHDL efficiently inhibits thrombus formation in laser-induced cremaster arteriole thrombosis models. Male mice (n = 4) were pretreated IV with saline control, sHDL (50 mg/kg), ML355 (1.5 mg/kg), or ML355-sHDL (sHDL at 50 mg/kg and ML355 at 1.5 mg/kg). After 24 hours, mice were anesthetized and surgically prepared as described in detail in the methods section. Multiple independent thrombi were induced and recorded as described above. (**A**) Representative captured thrombi from each group at different time points. (**B**) Dynamics of platelet accumulation in thrombi assessed by relative mean fluorescence intensity of platelet averaged by 8-10 thrombi per mouse, three mice in each group. All thrombi were statistically analyzed for the change of fluorescent intensity over the course of thrombus formation after subtracting fluorescent background defined on an uninjured section of the vessel using the Slidebook program. Data were represented as mean ± SD and compared by two-way ANOVA analysis for statistical difference. ****P* < 0.001.

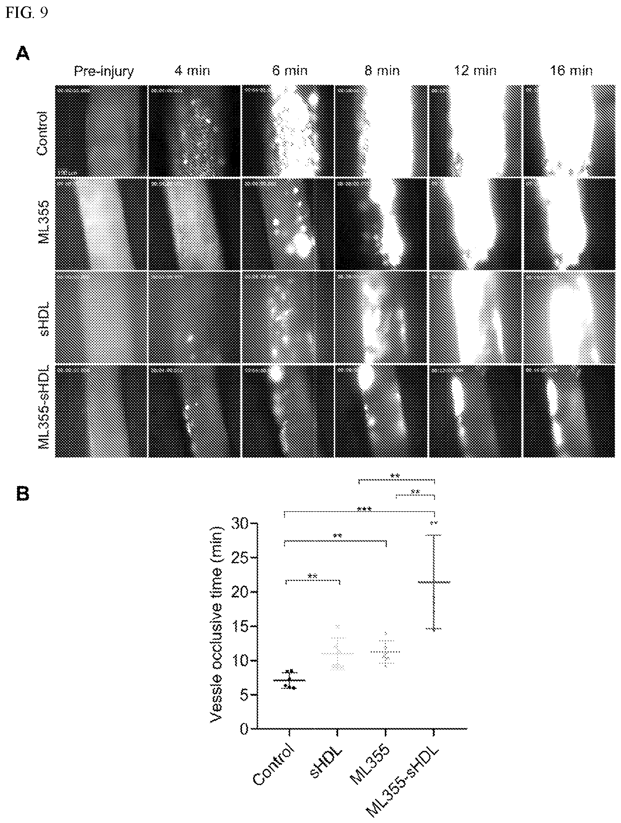

FIG. 9: ML355-sHDL efficiently delays vessel occlusion in FeCl₃- induced carotid artery thrombosis model. Both female and male mice (n = 6) were pretreated IV with saline control, sHDL (50 mg/kg), ML355 (1.5 mg/kg), or ML355-sHDL (sHDL at 50 mg/kg and ML355 at 1.5 mg/kg). After 24 hours, mice were anesthetized and surgically prepared as described in detail in the method section. (**A**) Representative images of thrombosis in carotid artery in response to FeCl₃ injury. 10% FeCl₃ was topically applied on the right carotid artery for 3 minutes and images of thrombi were recorded in real-time and vessel occlusion was determined by the secession of blood flow. (**B**) Carotid artery vessel occlusion time in each group. The mean vessel occlusion time in the control mice, ML355, sHDL and ML355-sHDL were 7.1 ± 1.2 min, 11.0 ± 2.3 min, 11.3 ± 1.3 min, and 21.4 ± 6.4 min, respectively. Data shown as mean ± SD (n = 6). ***P* < 0.01, ****P* < 0.001.

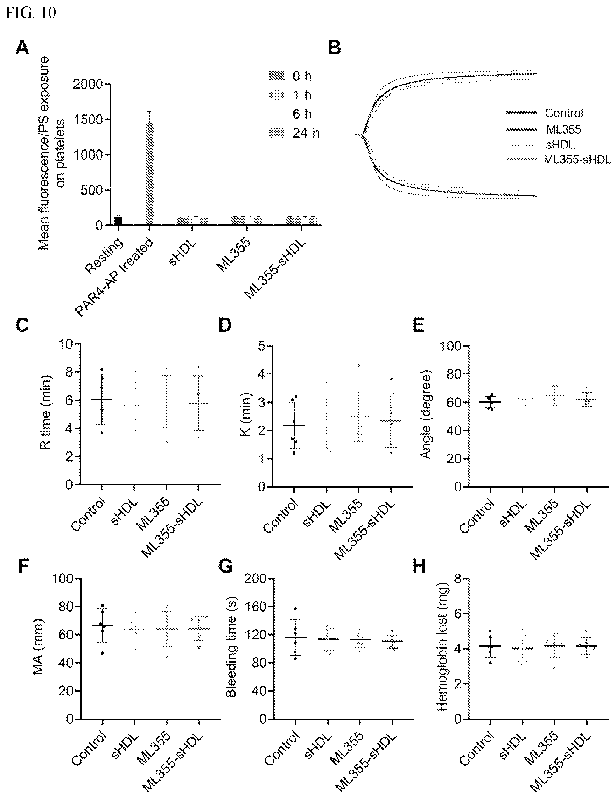

FIG. 10: ML355-sHDL does not affect the phosphatidylserine exposure on platelet surface, coagulation system, and hemostatic action. Both female and male mice (n = 6) were pretreated IV with saline control, sHDL (50 mg/kg), ML355 (1.5 mg/kg), or ML355-sHDL (sHDL at 50 mg/kg and ML355 at 1.5 mg/kg). All treated group were subjected to the different following examinations, respectively. (**A**) At different time points post administration (1, 6 and 24 hours), the phosphatidylserine-exposure over the time course in platelets from different groups were quantified by flow cytometry. Blood collected from untreated mice was taken as resting platelets (negative control) and blood stimulated with PAR4-activating peptide (200µM) was used as activated platelets (positive control). (**B**) Representative tracings indicating different treatments were presented. Some major coagulation parameters were analyzed. (**C**) R time, time to formation of the initial fibrin threads (min). (**D**) K time, the time until the clot reaches a certain strength (min). (**E**) Alpha (α) angle, the rapidity with which the clot forms (degree). (**F**) Maximum amplitude (MA), clot's maximum strength (mm). (**G**) Bleeding times and (**H**) blood loss (quantified as milligrams of hemoglobin) were quantified.

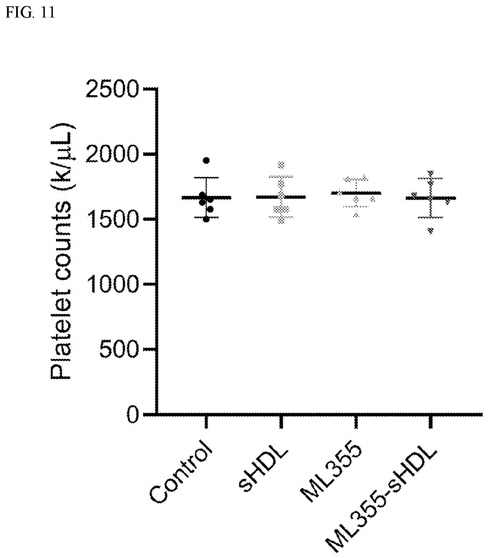

FIG. 11: ML355-sHDL treatment do not impact the platelet counts in mice. Both female and male mice (n = 6) were pretreated IV with saline control, sHDL (50 mg/kg), ML355 (1.5 mg/kg), or ML355-sHDL (sHDL at 50 mg/kg and ML355 at 1.5 mg/kg). After 24 hours, mice were anesthetized by intraperitoneal injection of ketamine/xylazine as described in the methods section. Blood were collected from mice using the lateral saphenous vein. Complete blood counts were performed using a Hemavet 950 analyzer (Drew Scientific Inc., Oxford, CT, USA).

### DEFINITIONS

To facilitate an understanding of the present invention, a number of terms and phrases are defined below:
As used here, the term "lipids" refer to fatty substances that are insoluble in water and include fats, oils, waxes, and related compounds. They may be either made in the blood (endogenous) or ingested in the diet (exogenous). Lipids are essential for normal body function and whether produced from an exogenous or endogenous source, they must be transported and then released for use by the cells. The production, transportation and release of lipids for use by the cells is referred to as lipid metabolism. While there are several classes of lipids, two major classes are cholesterol and triglycerides. Cholesterol may be ingested in the diet and manufactured by the cells of most organs and tissues in the body, primarily in the liver. Cholesterol can be found in its free form or, more often, combined with fatty acids as what is called cholesterol esters.

As used herein the term, "lipoproteins" refer to spherical compounds that are structured so that water-insoluble lipids are contained in a partially water-soluble shell. Depending on the type of lipoprotein, the contents include varying amounts of free and esterified cholesterol, triglycerides and apoproteins or apolipoproteins. There are five major types of lipoproteins, which differ in function and in their lipid and apoprotein content and are classified according to increasing density: (i) chylomicrons and chylomicron remnants, (ii) very low density lipoproteins ("VLDL"), (iii) intermediate-density lipoproteins ("IDL"), (iv) low-density lipoproteins ("LDL"), and (v) high-density lipoproteins ("HDL"). Cholesterol circulates in the bloodstream as particles associated with lipoproteins.

As used herein, the term "HDL" or "high density lipoprotein" refers to high-density lipoprotein. HDL comprises a complex of lipids and proteins in approximately equal amounts that functions as a transporter of cholesterol in the blood. HDL is mainly synthesized in and secreted from the liver and epithelial cells of the small intestine. Immediately after secretion, HDL is in a form of a discoidal particle containing apolipoprotein A-I (also called apoA-I) and phospholipid as its major constituents, and also called nascent HDL. This nascent HDL receives, in blood, free cholesterol from cell membranes of peripheral cells or produced in the hydrolysis course of other lipoproteins, and forms mature spherical HDL while holding, at its hydrophobic center, cholesterol ester converted from said cholesterol by the action of LCAT (lecithin cholesterol acyltransferase). HDL plays an extremely important role in a lipid metabolism process called "reverse cholesterol transport", which takes, in blood, cholesterol out of peripheral tissues and transports it to the liver. High levels of HDL are associated with a decreased risk of atherosclerosis and coronary heart disease (CHD) as the reverse cholesterol transport is considered one of the major mechanisms for HDL's prophylactic action on atherosclerosis.

As used herein, the terms "synthetic HDL," "sHDL," "reconstituted HDL", or "rHDL" refer to a particle structurally analogous to native HDL, composed of a lipid or lipids in association with at least one of the proteins of HDL, preferably Apo A-I or a mimetic thereof, and which exhibits all of the known physiological functions of HDL. Typically, the components of sHDL may be derived from blood, or produced by recombinant technology.

As used herein, the term "subject" refers to any animal (*e.g*., a mammal), including, but not limited to, humans, non-human primates, rodents, and the like, which is to be the recipient of a particular treatment. Typically, the terms "subject" and "patient" are used interchangeably herein in reference to a human subject.

As used herein, the term "sample" is used in its broadest sense. In one sense, it is meant to include a specimen or culture obtained from any source, as well as biological and environmental samples. Biological samples may be obtained from animals (including humans) and encompass fluids, solids, tissues, and gases. Biological samples include blood products, such as plasma, serum and the like. Environmental samples include environmental material such as surface matter, soil, water, crystals and industrial samples. Such examples are not however to be construed as limiting the sample types applicable to the present invention.

As used herein, the term "drug" or "therapeutic agent" is meant to include any molecule, molecular complex or substance administered to an organism for diagnostic or therapeutic purposes, including medical imaging, monitoring, contraceptive, cosmetic, nutraceutical, pharmaceutical and prophylactic applications. The term "drug" is further meant to include any such molecule, molecular complex or substance that is chemically modified and/or operatively attached to a biologic or biocompatible structure.

As used herein, the term "solvent" refers to a medium in which a reaction is conducted. Solvents may be liquid but are not limited to liquid form. Solvent categories include but are not limited to nonpolar, polar, protic, and aprotic.

### DETAILED DESCRIPTION OF THE INVENTION

Antiplatelet agents offer a desirable approach to thrombosis prevention through the reduction of platelet reactivity. However, major bleeding events greatly attenuate the clinical outcomes of most antithrombotic agents. Therefore, the development of safer and more effective strategies to prevent vascular occlusion and avoid bleeding is urgently needed. A reconstituted nanoparticle, synthetic HDL (sHDL), which mimics the native high-density lipoprotein (HDL), has been established as clinically safe and tolerable at high doses and is easily manufactured on a large scale.

Experiments conducted during the course of developing embodiments for the present invention demonstrated delivery of antiplatelet drug ML355, a selective inhibitor of 12(S)-lipoxygenase (12-LOX), by sHDL efficiently inhibits thrombosis via targeting ML355 to the intended site of action, improving the pharmaceutical profile and harnessing the innate antithrombotic efficacy of the sHDL carrier. Such results indicate that ML355-sHDL exhibits more potent inhibition on thrombus formation in both small arterioles and larger arteries in mice without impairing the normal hemostasis *in vivo.*

Accordingly, the present invention relates to nanoparticles associated with N-2-benzothiazolyl-4-[[(2-hydroxy-3-methoxyphenyl)methyl]amino]-benzenesulfonamide (ML355) configured for treating cardiovascular related disorders. In particular, the present invention is directed to compositions according to claim 1 comprising synthetic HDL (sHDL) nanoparticles associated with ML355 configured for treating cardiovascular related disorders (e.g., inhibit platelet aggregation; inhibit thrombosis formation; inhibit vessel occlusion; inhibit platelet associated 12-LOX activity, as well as systems and methods utilizing such sHDL nanoparticles (e.g., therapeutic settings).

HDL apolipoproteins include, for example apolipoprotein A-I (apo A-I), apolipoprotein A-II (apo A-II), apolipoprotein A4 (apo A4), apolipoprotein Cs (apo Cs), and apolipoprotein E (apo E). Preferably, the carrier particles are composed of Apo A-I or Apo A-II, however the use of other lipoproteins including apolipoprotein A4, apolipoprotein Cs or apolipoprotein E may be used alone or in combination to formulate carrier particle mixtures for delivery of therapeutic agents. In some embodiments, the HDL apolipoprotein is selected from preproapoliprotein, preproApoA-I, proApoA-I, ApoA-I, preproApoA-II, proApoA-II, ApoA-II, preproApoA-IV, proApoA-IV, ApoA-IV, ApoA-V, preproApoE, proApoE, ApoE, preproApoA-IMilano, proApoA-IMilano ApoA-IMilano preproApoA-IParis , proApoA-IParis, and ApoA-IParis and peptide mimetics of these proteins mixtures thereof. In some embodiments, mimetics of such HDL apolipoproteins are used.

ApoA-I is synthesized by the liver and small intestine as preproapolipoprotein which is secreted as a proprotein that is rapidly cleaved to generate a mature polypeptide having 243 amino acid residues. ApoA-I consists mainly of 6 to 8 different 22 amino acid repeats spaced by a linker moiety which is often proline, and in some cases consists of a stretch made up of several residues. ApoA-I forms three types of stable complexes with lipids: small, lipid-poor complexes referred to as pre-beta-1 HDL; flattened discoidal particles containing polar lipids (phospholipid and cholesterol) referred to as pre-beta-2 HDL; and spherical particles containing both polar and nonpolar lipids, referred to as spherical or mature HDL (HDL₃ and HDL₂). Most HDL particles in the circulating population contain both ApoA-I and ApoA-II (the second major HDL protein). However, the fraction of HDL containing only ApoA-I (referred to herein as the Al-HDL fraction) is more effective in reverse cholesterol transport.

In some embodiments, ApoA-I agonists or mimetics are provided. In some embodiments, such ApoA-I mimetics are capable of forming amphipathic α-helices that mimic the activity of ApoA-I, and have specific activities approaching or exceeding that of the native molecule. In some, the ApoA-I mimetics are peptides or peptide analogues that: form amphipathic helices (in the presence of lipids), bind lipids, form pre-β-like or HDL-like complexes, activate lecithin:cholesterol acyltransferase (LCAT), increase serum levels of HDL fractions, and promote cholesterol efflux.

The present invention is not limited to use of a particular ApoA-I mimetic. In some embodiments, any of the ApoA-I mimetics described in Srinivasa, et al., 2014 Curr. Opinion Lipidology Vol. 25(4): 304-308 are utilized. In some embodiments, any of the ApoA-I mimetics described in U.S. Patent Application Publication Nos. 20110046056 and 20130231459 are utilized.

In some embodiments, the "22A" ApoA-I mimetic is used (PVLDLFRELLNELLEALKQKLK) (SEQ ID NO: 4) (see, e.g., U.S. Patent No. 7,566,695). In some embodiments, any of the following ApoA-I mimetics shown in Table 1 as described in U.S. Patent No. 7,566,695 are utilized:

**Table 1. ApoA-I mimetics**

| **SEQ ID NO** | **AMINO ACID SEQUENCE** |
|---|---|
| (SEQ ID NO:1) | PVLDLFRELLNELLEZLKQKLK |
| (SEQ ID NO:2) | GVLDLFRELLNELLEALKQKLKK |
| (SEQ ID NO:3) | PVLDLFRELLNELLEWLKQKLK |
| (SEQ ID NO:4) | PVLDLFRELLNELLEALKQKLK |
| (SEQ ID NO:5) | pVLDLFRELLNELLEALKQKLKK |
| (SEQ ID NO:6) | PVLDLFRELLNEXLEALKQKLK |
| (SEQ ID NO:7) | PVLDLFKELLNELLEALKQKLK |
| (SEQ ID NO:8) | PVLDLFRELLNEGLEALKQKLK |
| (SEQ ID NO:9) | PVLDLFRELGNELLEALKQKLK |
| (SEQ ID NO:10) | PVLDLFRELLNELLEAZKQKLK |
| (SEQ ID NO:11) | PVLDLFKELLQELLEALKQKLK |
| (SEQ ID NO:12) | PVLDLFRELLNELLEAGKQKLK |
| (SEQ ID NO:13) | GVLDLFRELLNEGLEALKQKLK |
| (SEQ ID NO:14) | PVLDLFRELLNELLEALOQOLO |
| (SEQ ID NO:15) | PVLDLFRELWNELLEALKQKLK |
| (SEQ ID NO:16) | PVLDLLRELLNELLEALKQKLK |
| (SEQ ID NO:17) | PVLELFKELLQELLEALKQKLK |
| (SEQ ID NO:18) | GVLDLFRELLNELLEALKQKLK |
| (SEQ ID NO:19) | pVLDLFRELLNEGLEALKQKLK |
| (SEQ ID NO:20) | PVLDLFREGLNELLEALKQKLK |
| (SEQ ID NO:21) | pVLDLFRELLNELLEALKQKLK |
| (SEQ ID NO:22) | PVLDLFRELLNELLEGLKQKLK |
| (SEQ ID NO:23) | PLLELFKELLQELLEALKQKLK |
| (SEQ ID NO:24) | PVLDLFRELLNELLEALQKKLK |
| (SEQ ID NO:25) | PVLDFFRELLNEXLEALKQKLK |
| (SEQ ID NO:26) | PVLDLFRELLNELLELLKQKLK |
| (SEQ ID NO:27) | PVLDLFRELLNELZEALKQKLK |
| (SEQ ID NO:28) | PVLDLFRELLNELWEALKQKLK |
| (SEQ ID NO:29) | AVLDLFRELLNELLEALKQKLK |
| (SEQ ID NO:30) | PVLDLPRELLNELLEALKQKLK¹ |
| (SEQ ID NO:31) | PVLDLFLELLNEXLEALKQKLK |
| (SEQ ID NO:32) | XVLDLFRELLNELLEALKQKLK |
| (SEQ ID NO:33) | PVLDLFREKLNELLEALKQKLK |
| (SEQ ID NO:34) | PVLDZFRELLNELLEALKQKLK |
| (SEQ ID NO:35) | PVLDWFRELLNELLEALKQKLK |
| (SEQ ID NO:36) | PLLELLKELLQELLEALKQKLK |
| (SEQ ID NO:37) | PVLDLFREWLNELLEALKQKLK |
| (SEQ ID NO:38) | PVLDLFRELLNEXLEAWKQKLK |
| (SEQ ID NO:39) | PVLDLFRELLEELLKALKKKLK |
| (SEQ ID NO:40) | PVLDLFNELLRELLEALQKKLK |
| (SEQ ID NO:41) | PVLDLWRELLNEXLEALKQKLK |
| (SEQ ID NO:42) | PVLDEFREKLNEXWEALKQKLK |
| (SEQ ID NO:43) | PVLDEFREKLWEXLEALKQKLK |
| (SEQ ID NO:44) | pvldefreklneXlealkqklk |
| (SEQ ID NO:45) | PVLDEFREKLNEXLEALKQKLK |
| (SEQ ID NO:46) | PVLDLFREKLNEXLEALKQKLK |
| (SEQ ID NO:47) | ~VLDLFRELLNEGLEALKQKLK |
| (SEQ ID NO:48) | pvLDLFRELLNELLEALKQKLK |
| (SEQ ID NO:49) | PVLDLFRNLLEKLLEALEQKLK |
| (SEQ ID NO:50) | PVLDLFRELLWEXLEALKQKLK |
| (SEQ ID NO:51) | PVLDLFWELLNEXLEALKQKLK |
| (SEQ ID NO:52) | PVWDEFREKLNEXLEALKQKLK |
| (SEQ ID NO:53) | VVLDLFRELLNELLEALKQKLK |
| (SEQ ID NO:54) | PVLDLFRELLNEWLEALKQKLK |
| (SEQ ID NO:55) | P~~~LFRELLNELLEALKQKLK |
| (SEQ ID NO:56) | PVLDLFRELLNELLEALKQKKK |
| (SEQ ID NO:57) | PVLDLFRNLLEELLKALEQKLK |
| (SEQ ID NO:58) | PVLDEFREKLNEXLEALKQKL~ |
| (SEQ ID NO:59) | LVLDLFRELLNELLEALKQKLK |
| (SEQ ID NO:60) | PVLDLFRELLNELLEALKQ~~~ |
| (SEQ ID NO:61) | PVLDEFRWKLNEXLEALKQKLK |
| (SEQ ID NO:62) | PVLDEWREKLNEXLEALKQKLK |
| (SEQ ID NO:63) | PVLDFFREKLNEXLEALKQKLK |
| (SEQ ID NO:64) | PWLDEFREKLNEXLEALKQKLK |
| (SEQ ID NO:65) | ~VLDEFREKLNEXLEALKQKLK |
| (SEQ ID NO:66) | PVLDLFRNLLEELLEALQKKLK |
| (SEQ ID NO:67) | ~VLDLFRELLNELLEALKQKLK |
| (SEQ ID NO:68) | PVLDEFRELLKEXLEALKQKLK |
| (SEQ ID NO:69) | PVLDEFRKKLNEXLEALKQKLK |
| (SEQ ID NO:70) | PVLDEFRELLYEXLEALKQKLK |
| (SEQ ID NO:71) | PVLDEFREKLNELXEALKQKLK |
| (SEQ ID NO:72) | PVLDLFRELLNEXLWALKQKLK |
| (SEQ ID NO:73) | PVLDEFWEKLNEXLEALKQKLK |
| (SEQ ID NO:74) | PVLDKFREKLNEXLEALKQKLK |
| (SEQ ID NO:75) | PVLDEFREKLNEELEALKQKLK |
| (SEQ ID NO:76) | PVLDEFRELLFEXLEALKQKLK |
| (SEQ ID NO:77) | PVLDEFREKLNKXLEALKQKLK |
| (SEQ ID NO:78) | PVLDEFRDKLNEXLEALKQKLK |
| (SEQ ID NO:79) | PVLDEFRELLNELLEALKQKLK |
| (SEQ ID NO:80) | PVLDLFERLLNELLEALQKKLK |
| (SEQ ID NO:81) | PVLDEFREKLNWXLEALKQKLK |
| (SEQ ID NO:82) | ~~LDEFREKLNEXLEALKQKLK |
| (SEQ ID NO:83) | PVLDEFREKLNEXLEALWQKLK |
| (SEQ ID NO:84) | PVLDEFREKLNELLEALKQKLK |
| (SEQ ID NO:85) | P~LDLFRELLNELLEALKQKLK |
| (SEQ ID NO:86) | PVLELFERLLDELLNALQKKLK |
| (SEQ ID NO:87) | pllellkellqellealkqklk |
| (SEQ ID NO:88) | PVLDKFRELLNEXLEALKQKLK |
| (SEQ ID NO:89) | PVLDEFREKLNEXLWALKQKLK |
| (SEQ ID NO:90) | ~~~DEFREKLNEXLEALKQKLK |
| (SEQ ID NO:91) | PVLDEFRELLNEXLEALKQKLK |
| (SEQ ID NO:92) | PVLDEFRELYNEXLEALKQKLK |
| (SEQ ID NO:93) | PVLDEFREKLNEXLKALKQKLK |
| (SEQ ID NO:94) | PVLDEFREKLNEALEALKQKLK |
| (SEQ ID NO:95) | PVLDLFRELLNLXLEALKQKLK |
| (SEQ ID NO:96) | pvldlfrellneXlealkqklk |
| (SEQ ID NO:97) | PVLDLFRELLNELLE~~~~~~~ |
| (SEQ ID NO:98) | PVLDLFRELLNEELEALKQKLK |
| (SEQ ID NO:99) | KLKQKLAELLENLLERFLDLVP |
| (SEQ ID NO:100) | pvldlfrellnellealkqklk |
| (SEQ ID NO:101) | PVLDLFRELLNWXLEALKQKLK |
| (SEQ ID NO:102) | PVLDLFRELLNLXLEALKEKLK |
| (SEQ ID NO:103) | PVLDEFRELLNEELEALKQKLK |
| (SEQ ID NO:104) | P~~~~~~~LLNELLEALKQKLK |
| (SEQ ID NO:105) | PAADAFREAANEAAEAAKQKAK |
| (SEQ ID NO:106) | PVLDLFREKLNEELEALKQKLK |
| (SEQ ID NO:107) | klkqklaellenllerfldlvp |
| (SEQ ID NO:108) | PVLDLFRWLLNEXLEALKQKLK |
| (SEQ ID NO:109) | PVLDEFREKLNERLEALKQKLK |
| (SEQ ID NO:110) | PVLDEFREKLNEXXEALKQKLK |
| (SEQ ID NO:111) | PVLDEFREKLWEXWEALKQKLK |
| (SEQ ID NO:112) | PVLDEFREKLNEXSEALKQKLK |
| (SEQ ID NO:113) | PVLDEFREKLNEPLEALKQKLK |
| (SEQ ID NO:114) | PVLDEFREKLNEXMEALKQKLK |
| (SEQ ID NO:115) | PKLDEFREKLNEXLEALKQKLK |
| (SEQ ID NO:116) | PHLDEFREKLNEXLEALKQKLK |
| (SEQ ID NO:117) | PELDEFREKLNEXLEALKQKLK |
| (SEQ ID NO:118) | PVLDEFREKLNEXLEALEQKLK |
| (SEQ ID NO:119) | PVLDEFREKLNEELEAXKQKLK |
| (SEQ ID NO:120) | PVLDEFREKLNEELEXLKQKLK |
| (SEQ ID NO:121) | PVLDEFREKLNEELEALWQKLK |
| (SEQ ID NO:122) | PVLDEFREKLNEELEWLKQKLK |
| (SEQ ID NO:123) | QVLDLFRELLNELLEALKQKLK |
| (SEQ ID NO:124) | PVLDLFOELLNELLEALOQOLO |
| (SEQ ID NO:125) | NVLDLFRELLNELLEALKQKLK |
| (SEQ ID NO:126) | PVLDLFRELLNELGEALKQKLK |
| (SEQ ID NO:127) | PVLDLFRELLNELLELLKQKLK |
| (SEQ ID NO:128) | PVLDLFRELLNELLEFLKQKLK |
| (SEQ ID NO:129) | PVLELFNDLLRELLEALQKKLK |
| (SEQ ID NO:130) | PVLELFNDLLRELLEALKQKLK |
| (SEQ ID NO:131) | PVLELFKELLNELLDALRQKLK |
| (SEQ ID NO:132) | PVLDLFRELLENLLEALQKKLK |
| (SEQ ID NO:133) | PVLELFERLLEDLLQALNKKLK |
| (SEQ ID NO:134) | PVLELFERLLEDLLKALNOKLK |
| (SEQ ID NO:135) | DVLDLFRELLNELLEALKQKLK |
| (SEQ ID NO:136) | PALELFKDLLQELLEALKQKLK |
| (SEQ ID NO:137) | PVLDLFRELLNEGLEAZKQKLK |
| (SEQ ID NO:138) | PVLDLFRELLNEGLEWLKQKLK |
| (SEQ ID NO:139) | PVLDLFRELWNEGLEALKQKLK |
| (SEQ ID NO:140) | PVLDLFRELLNEGLEALOQOLO |
| (SEQ ID NO:141) | PVLDFFRELLNEGLEALKQKLK |
| (SEQ ID NO:142) | PVLELFRELLNEGLEALKQKLK |
| (SEQ ID NO:143) | PVLDLFRELLNEGLEALKQKLK* |
| (SEQ ID NO:144) | pVLELFENLLERLLDALQKKLK |
| (SEQ ID NO:145) | GVLELFENLLERLLDALQKKLK |
| (SEQ ID NO:146) | PVLELFENLLERLLDALQKKLK |
| (SEQ ID NO:147) | PVLELFENLLERLFDALQKKLK |
| (SEQ ID NO:148) | PVLELFENLLERLGDALQKKLK |
| (SEQ ID NO:149) | PVLELFENLWERLLDALQKKLK |
| (SEQ ID NO:150) | PLLELFENLLERLLDALQKKLK |
| (SEQ ID NO:151) | PVLELFENLGERLLDALQKKLK |
| (SEQ ID NO:152) | PVFELFENLLERLLDALQKKLK |
| (SEQ ID NO:153) | AVLELFENLLERLLDALQKKLK |
| (SEQ ID NO:154) | PVLELFENLLERGLDALQKKLK |
| (SEQ ID NO:155) | PVLELFLNLWERLLDALQKKLK |
| (SEQ ID NO:156) | PVLELFLNLLERLLDALQKKLK |
| (SEQ ID NO:157) | PVLEFFENLLERLLDALQKKLK |
| (SEQ ID NO:158) | PVLELFLNLLERLLDWLQKKLK |
| (SEQ ID NO:159) | PVLDLFENLLERLLDALQKKLK |
| (SEQ ID NO:160) | PVLELFENLLERLLDWLQKKLK |
| (SEQ ID NO:161) | PVLELFENLLERLLEALQKKLK |
| (SEQ ID NO:162) | PVLELFENWLERLLDALQKKLK |
| (SEQ ID NO:163) | PVLELFENLLERLWDALQKKLK |
| (SEQ ID NO:164) | PVLELFENLLERLLDAWQKKLK |
| (SEQ ID NO:165) | PVLELFENLLERLLDLLQKKLK |
| (SEQ ID NO:166) | PVLELFLNLLEKLLDALQKKLK |
| (SEQ ID NO:167) | PVLELFENGLERLLDALQKKLK |
| (SEQ ID NO:168) | PVLELFEQLLEKLLDALQKKLK |
| (SEQ ID NO:169) | PVLELFENLLEKLLDALQKKLK |
| (SEQ ID NO:170) | PVLELFENLLEOLLDALQOOLO |
| (SEQ ID NO:171) | PVLELFENLLEKLLDLLQKKLK |
| (SEQ ID NO:172) | PVLELFLNLLERLGDALQKKLK |
| (SEQ ID NO:173) | PVLDLFDNLLDRLLDLLNKKLK |
| (SEQ ID NO:174) | pvlelfenllerlldalqkklk |
| (SEQ ID NO:175) | PVLELFENLLERLLELLNKKLK |
| (SEQ ID NO:176) | PVLELWENLLERLLDALQKKLK |
| (SEQ ID NO:177) | GVLELFLNLLERLLDALQKKLK |
| (SEQ ID NO:178) | PVLELFDNLLEKLLEALQKKLR |
| (SEQ ID NO:179) | PVLELFDNLLERLLDALQKKLK |
| (SEQ ID NO:180) | PVLELFDNLLDKLLDALQKKLR |
| (SEQ ID NO:181) | PVLELFENLLERWLDALQKKLK |
| (SEQ ID NO:182) | PVLELFENLLEKLLEALQKKLK |
| (SEQ ID NO:183) | PLLELFENLLEKLLDALQKKLK |
| (SEQ ID NO:184) | PVLELFLNLLERLLDAWQKKLK |
| (SEQ ID NO:185) | PVLELFENLLERLLDALQOOLO |
| (SEQ ID NO:186) | PVLELFEQLLERLLDALQKKLK |
| (SEQ ID NO:187) | PVLELFENLLERLLDALNKKLK |
| (SEQ ID NO:188) | PVLELFENLLDRLLDALQKKLK |
| (SEQ ID NO:189) | DVLELFENLLERLLDALQKKLK |
| (SEQ ID NO:190) | PVLEFWDNLLDKLLDALQKKLR |
| (SEQ ID NO:191) | PVLDLLRELLEELKQKLK* |
| (SEQ ID NO:192) | PVLDLFKELLEELKQKLK* |
| (SEQ ID NO:193) | PVLDLFRELLEELKQKLK* |
| (SEQ ID NO:194) | PVLELFRELLEELKQKLK* |
| (SEQ ID NO:195) | PVLELFKELLEELKQKLK* |
| (SEQ ID NO:196) | PVLDLFRELLEELKNKLK* |
| (SEQ ID NO:197) | PLLDLFRELLEELKQKLK* |
| (SEQ ID NO:198) | GVLDLFRELLEELKQKLK* |
| (SEQ ID NO:199) | PVLDLFRELWEELKQKLK* |
| (SEQ ID NO:200) | NVLDLFRELLEELKQKLK* |
| (SEQ ID NO:201) | PLLDLFKELLEELKQKLK* |
| (SEQ ID NO:202) | PALELFKDLLEELRQKLR* |
| (SEQ ID NO:203) | AVLDLFRELLEELKQKLK* |
| (SEQ ID NO:204) | PVLDFFRELLEELKQKLK* |
| (SEQ ID NO:205) | PVLDLFREWLEELKQKLK* |
| (SEQ ID NO:206) | PLLELLKELLEELKQKLK* |
| (SEQ ID NO:207) | PVLELLKELLEELKQKLK* |
| (SEQ ID NO:208) | PALELFKDLLEELRQRLK* |
| (SEQ ID NO:209) | PVLDLFRELLNELLQKLK |
| (SEQ ID NO:210) | PVLDLFRELLEELKQKLK |
| (SEQ ID NO:211) | PVLDLFRELLEELOQOLO* |
| (SEQ ID NO:212) | PVLDLFOELLEELOQOLK* |
| (SEQ ID NO:213) | PALELFKDLLEEFRQRLK* |
| (SEQ ID NO:214) | pVLDLFRELLEELKQKLK* |
| (SEQ ID NO:215) | PVLDLFRELLEEWKQKLK* |
| (SEQ ID NO:216) | PVLELFKELLEELKQKLK |
| (SEQ ID NO:217) | PVLDLFRELLELLKQKLK |
| (SEQ ID NO:218) | PVLDLFRELLNELLQKLK* |
| (SEQ ID NO:219) | PVLDLFRELLNELWQKLK |
| (SEQ ID NO:220) | PVLDLFRELLEELQKKLK |
| (SEQ ID NO:221) | DVLDLFRELLEELKQKLK* |
| (SEQ ID NO:222) | PVLDAFRELLEALLQLKK |
| (SEQ ID NO:223) | PVLDAFRELLEALAQLKK |
| (SEQ ID NO:224) | PVLDLFREGWEELKQKLK |
| (SEQ ID NO:225) | PVLDAFRELAEALAQLKK |
| (SEQ ID NO:226) | PVLDAFRELGEALLQLKK |
| (SEQ ID NO:227) | PVLDLFRELGEELKQKLK* |
| (SEQ ID NO:228) | PVLDLFREGLEELKQKLK* |
| (SEQ ID NO:229) | PVLDLFRELLEEGKQKLK* |
| (SEQ ID NO:230) | PVLELFERLLEDLQKKLK |
| (SEQ ID NO:231) | PVLDLFRELLEKLEQKLK |
| (SEQ ID NO:232) | PLLELFKELLEELKQKLK* |
| (SEQ ID NO:233) | LDDLLQKWAEAFNQLLKK |
| (SEQ ID NO:234) | EWLKAFYEKVLEKLKELF* |
| (SEQ ID NO:235) | EWLEAFYKKVLEKLKELF* |
| (SEQ ID NO:236) | DWLKAFYDKVAEKLKEAF* |
| (SEQ ID NO:237) | DWFKAFYDKVFEKFKEFF |
| (SEQ ID NO:238) | GIKKFLGSIWKFIKAFVG |
| (SEQ ID NO:239) | DWFKAFYDKVAEKFKEAF |
| (SEQ ID NO:240) | DWLKAFYDKVAEKLKEAF |
| (SEQ ID NO:241) | DWLKAFYDKVFEKFKEFF |
| (SEQ ID NO:242) | EWLEAFYKKVLEKLKELP |
| (SEQ ID NO:243) | DWFKAFYDKFFEKFKEFF |
| (SEQ ID NO:244) | EWLKAFYEKVLEKLKELF |
| (SEQ ID NO:245) | EWLKAEYEKVEEKLKELF* |
| (SEQ ID NO:246) | EWLKAEYEKVLEKLKELF* |
| (SEQ ID NO:247) | EWLKAFYKKVLEKLKELF* |
| (SEQ ID NO:248) | PVLDLFRELLEQKLK* |
| (SEQ ID NO:249) | PVLDLFRELLEELKQK* |
| (SEQ ID NO:250) | PVLDLFRELLEKLKQK* |
| (SEQ ID NO:251) | PVLDLFRELLEKLQK* |
| (SEQ ID NO:252) | PVLDLFRELLEALKQK* |
| (SEQ ID NO:253) | PVLDLFENLLERLKQK* |
| (SEQ ID NO:254) | PVLDLFRELLNELKQK* |

| | |
|---|---|
| * indicates peptides that are N-terminal acetylated and C-terminal amidated; indicates peptides that are N-terminal dansylated; sp indicates peptides that exhibited solubility problems under the experimental conditions; X is Aib; Z is Nal; O is Orn; He (%) designates percent helicity; mics designates micelles; and ^{~} indicates deleted amino acids. | |

In some embodiments, an ApoA-I mimetic having the following sequence as described in U.S. Patent No. 6,743,778 is utilized: Asp Trp Leu Lys Ala Phe Tyr Asp Lys Val Ala Glu Lys Leu Lys Glu Ala Phe (SEQ ID NO: 256).

In some embodiments, any of the following ApoA-I mimetics shown in Table 2 as described in U.S. Patent Application Publication No. 2003/0171277 are utilized:

**Table 2.**

| **SEQ ID NO** | **AMINO ACID SEQUENCE** |
|---|---|
| (SEQ ID NO:256) | D-W-L-K-A-F-Y-D-K-V-A-E-K-L-K-E-A-F |
| (SEQ ID NO:257) | Ac-D-W-L-K-A-F-Y-D-K-V-A-E-K-L-K-E-A-F-NH₂ |
| (SEQ ID NO:258) | Ac-D-W-F-K-A-F-Y-D-K-V-A-E-K-L-K-E-A-F-NH₂ |
| (SEQ ID NO:259) | Ac-D-W-L-K-A-F-Y-D-K-V-A-E-K-F-K-E-A-F-NH₂ |
| (SEQ ID NO:260) | Ac-D-W-F-K-A-F-Y-D-K-V-A-E-K-F-K-E-A-F-NH₂ |
| (SEQ ID NO:261) | Ac-D-W-L-K-A-F-Y-D-K-V-F-E-K-F-K-E-F-F-NH₂ |
| (SEQ ID NO:262) | Ac-D-W-L-K-A-F-Y-D-K-F-F-E-K-F-K-E-F-F-NH₂ |
| (SEQ ID NO:263) | Ac-D-W-F-K-A-F-Y-D-K-F-F-E-K-F-K-E-F-F-NH₂ |
| (SEQ ID NO:264) | Ac-D-W-L-K-A-F-Y-D-K-V-A-E-K-L-K-E-F-F-NH₂ |
| (SEQ ID NO:265) | Ac-D-W-L-K-A-F-Y-D-K-V-F-E-K-F-K-E-A-F-NH₂ |
| (SEQ ID NO:266) | Ac-D-W-L-K-A-F-Y-D-K-V-F-E-K-L-K-E-F-F-NH₂ |
| (SEQ ID NO:267) | Ac-D-W-L-K-A-F-Y-D-K-V-A-E-K-F-K-E-F-F-NH₂ |
| (SEQ ID NO:268) | Ac-D-W-L-K-A-F-Y-D-K-V-F-E-K-F-K-E-F-F-NH₂ |
| (SEQ ID NO:269) | Ac-E-W-L-K-L-F-Y-E-K-V-L-E-K-F-K-E-A-F-NH₂ |
| (SEQ ID NO:270) | Ac-E-W-L-K-A-F-Y-D-K-V-A-E-K-F-K-E-A-F-NH₂ |
| (SEQ ID NO:271) | Ac-E-W-L-K-A-F-Y-D-K-V-A-E-K-L-K-E-F-F-NH₂ |
| (SEQ ID NO:272) | Ac-E-W-L-K-A-F-Y-D-K-V-F-E-K-F-K-E-A-F-NH₂ |
| (SEQ ID NO:273) | Ac-E-W-L-K-A-F-Y-D-K-V-F-E-K-L-K-E-F-F-NH₂ |
| (SEQ ID NO:274) | Ac-E-W-L-K-A-F-Y-D-K-V-A-E-K-F-K-E-F-F-NH₂ |
| (SEQ ID NO:275) | Ac-E-W-L-K-A-F-Y-D-K-V-F-E-K-F-K-E-F-F-NH₂ |
| (SEQ ID NO:276) | AC-A-F-Y-D-K-V-A-E-K-L-K-E-A-F-NH₂ |
| (SEQ ID NO:277) | Ac-A-F-Y-D-K-V-A-E-K-F-K-E-A-F-NH₂ |
| (SEQ ID NO:278) | Ac-A-F-Y-D-K-V-A-E-K-F-K-E-A-F-NH₂ |
| (SEQ ID NO:279) | Ac-A-F-Y-D-K-F-F-E-K-F-K-E-F-F-NH₂ |
| (SEQ ID NO:280) | Ac-A-F-Y-D-K-F-F-E-K-F-K-E-F-F-NH₂ |
| (SEQ ID NO:281) | Ac-A-F-Y-D-K-V-A-E-K-F-K-E-A-F-NH₂ |
| (SEQ ID NO:282) | Ac-A-F-Y-D-K-V-A-E-K-L-K-E-F-F-NH₂ |
| (SEQ ID NO:283) | Ac-A-F-Y-D-K-V-F-E-K-F-K-E-A-F-NH₂ |
| (SEQ ID NO:284) | Ac-A-F-Y-D-K-V-F-E-K-L-K-E-F-F-NH₂ |
| (SEQ ID NO:285) | Ac-A-F-Y-D-K-V-A-E-K-F-K-E-F-F-NH₂ |
| (SEQ ID NO:286) | Ac-K-A-F-Y-D-K-V-F-E-K-F-K-E-F-NH₂ |
| (SEQ ID NO:287) | Ac-L-F-Y-E-K-V-L-E-K-F-K-E-A-F-NH₂ |
| (SEQ ID NO:288) | Ac-A-F-Y-D-K-V-A-E-K-F-K-E-A-F-NH₂ |
| (SEQ ID NO:289) | Ac-A-F-Y-D-K-V-A-E-K-L-K-E-F-F-NH₂ |
| (SEQ ID NO:290) | Ac-A-F-Y-D-K-V-F-E-K-F-K-E-A-F-NH₂ |
| (SEQ ID NO:291) | Ac-A-F-Y-D-K-V-F-E-K-L-K-E-F-F-NH₂ |
| (SEQ ID NO:292) | Ac-A-F-Y-D-K-V-A-E-K-F-K-E-F-F-NH₂ |
| (SEQ ID NO:293) | Ac-A-F-Y-D-K-V-F-E-K-F-K-E-F-F-NH₂ |
| (SEQ ID NO:294) | Ac-D-W-L-K-A-L-Y-D-K-V-A-E-K-L-K-E-A-L-NH₂ |
| (SEQ ID NO:295) | Ac-D-W-F-K-A-F-Y-E-K-V-A-E-K-L-K-E-F-F-NH₂ |
| (SEQ ID NO:296) | Ac-D-W-F-K-A-F-Y-E-K-F-F-E-K-F-K-E-F-F-NH₂ |
| (SEQ ID NO:297) | Ac-E-W-L-K-A-L-Y-E-K-V-A-E-K-L-K-E-A-L-NH₂ |
| (SEQ ID NO:298) | Ac-E-W-L-K-A-F-Y-E-K-V-A-E-K-L-K-E-A-F-NH₂ |
| (SEQ ID NO:299) | Ac-E-W-F-K-A-F-Y-E-K-V-A-E-K-L-K-E-F-F-NH₂ |
| (SEQ ID NO:300) | Ac-E-W-L-K-A-F-Y-E-K-V-F-E-K-F-K-E-F-F-NH₂ |
| (SEQ ID NO:301) | Ac-E-W-L-K-A-F-Y-E-K-F-F-E-K-F-K-E-F-F-NH₂ |
| (SEQ ID NO:302) | Ac-E-W-F-K-A-F-Y-E-K-F-F-E-K-F-K-E-F-F-NH₂ |
| (SEQ ID NO:303) | Ac-D-F-L-K-A-W-Y-D-K-V-A-E-K-L-K-E-A-W-NH₂ |
| (SEQ ID NO:304) | Ac-E-F-L-K-A-W-Y-E-K-V-A-E-K-L-K-E-A-W-NH₂ |
| (SEQ ID NO:305) | Ac-D-F-W-K-A-W-Y-D-K-V-A-E-K-L-K-E-W-W-NH₂ |
| (SEQ ID NO:306) | Ac-E-F-W-K-A-W-Y-E-K-V-A-E-K-L-K-E-W-W-NH₂ |
| (SEQ ID NO:307) | Ac-D-K-L-K-A-F-Y-D-K-V-F-E-W-A-K-E-A-F-NH₂ |
| (SEQ ID NO:308) | Ac-D-K-W-K-A-V-Y-D-K-F-A-E-A-F-K-E-F-L-NH₂ |
| (SEQ ID NO:309) | Ac-E-K-L-K-A-F-Y-E-K-V-F-E-W-A-K-E-A-F-NH₂ |
| (SEQ ID NO:310) | Ac-E-K-W-K-A-V-Y-E-K-F-A-E-A-F-K-E-F-L-NH₂ |
| (SEQ ID NO:311) | Ac-D-W-L-K-A-F-V-D-K-F-A-E-K-F-K-E-A-Y-NH₂ |
| (SEQ ID NO:312) | Ac-E-K-W-K-A-V-Y-E-K-F-A-E-A-F-K-E-F-L-NH₂ |
| (SEQ ID NO:313) | Ac-D-W-L-K-A-F-V-Y-D-K-V-F-K-L-K-E-F-F-NH₂ |
| (SEQ ID NO:314) | Ac-E-W-L-K-A-F-V-Y-E-K-V-F-K-L-K-E-F-F-NH₂ |
| (SEQ ID NO:315) | Ac-D-W-L-R-A-F-Y-D-K-V-A-E-K-L-K-E-A-F-NH₂ |
| (SEQ ID NO:316) | Ac-E-W-L-R-A-F-Y-E-K-V-A-E-K-L-K-E-A-F-NH₂ |
| (SEQ ID NO:317) | Ac-D-W-L-K-A-F-Y-D-R-V-A-E-K-L-K-E-A-F-NH₂ |
| (SEQ ID NO:318) | Ac-E-W-L-K-A-F-Y-E-R-V-A-E-K-L-K-E-A-F-NH₂ |
| (SEQ ID NO:319) | Ac-D-W-L-K-A-F-Y-D-K-V-A-E-R-L-K-E-A-F-NH₂ |
| (SEQ ID NO:320) | Ac-E-W-L-K-A-F-Y-E-K-V-A-E-R-L-K-E-A-F-NH₂ |
| (SEQ ID NO:321) | Ac-D-W-L-K-A-F-Y-D-K-V-A-E-K-L-R-E-A-F-NH₂ |
| (SEQ ID NO:322) | Ac-E-W-L-K-A-F-Y-E-K-V-A-E-K-L-R-E-A-F-NH₂ |
| (SEQ ID NO:323) | Ac-D-W-L-K-A-F-Y-D-R-V-A-E-R-L-K-E-A-F-NH₂ |
| (SEQ ID NO:324) | Ac-E-W-L-K-A-F-Y-E-R-V-A-E-R-L-K-E-A-F-NH₂ |
| (SEQ ID NO:325) | Ac-D-W-L-R-A-F-Y-D-K-V-A-E-K-L-R-E-A-F-NH₂ |
| (SEQ ID NO:326) | Ac-E-W-L-R-A-F-Y-E-K-V-A-E-K-L-R-E-A-F-NH₂ |
| (SEQ ID NO:327) | Ac-D-W-L-R-A-F-Y-D-R-V-A-E-K-L-K-E-A-F-NH₂ |
| (SEQ ID NO:328) | Ac-E-W-L-R-A-F-Y-E-R-V-A-E-K-L-K-E-A-F-NH₂ |
| (SEQ ID NO:329) | Ac-D-W-L-K-A-F-Y-D-K-V-A-E-R-L-R-E-A-F-NH₂ |
| (SEQ ID NO:330) | Ac-E-W-L-K-A-F-Y-E-K-V-A-E-R-L-R-E-A-F-NH₂ |
| (SEQ ID NO:331) | Ac-D-W-L-R-A-F-Y-D-K-V-A-E-R-L-K-E-A-F-NH₂ |
| (SEQ ID NO:332) | Ac-E-W-L-R-A-F-Y-E-K-V-A-E-R-L-K-E-A-F-NH₂ |

In some embodiments, an ApoA-I mimetic having the following sequence as described in U.S. Patent Application Publication No. 2006/0069030 is utilized: F-A-E-K-F-K-E-A-V-K-D-Y-F-A-K-F-W-D (SEQ ID NO: 333).

In some embodiments, an ApoA-I mimetic having the following sequence as described in U.S. Patent Application Publication No. 2009/0081293 is utilized:
DWFKAFYDKVAEKFKEAF (SEQ ID NO: 334); DWLKAFYDKVAEKLKEAF (SEQ ID NO: 335); PALEDLRQGLLPVLESFKVFLSALEEYTKKLNTQ (SEQ ID NO: 336).

Amphipathic lipids include, for example, any lipid molecule which has both a hydrophobic and a hydrophilic moiety. Examples include phospholipids or glycolipids. Examples of phospholipids which may be used in the sHDL-ML355 nanoparticles include but are not limited to dipalmitoylphosphatidylcholine (DPPC), dioleoyl-sn-glycero-3-phosphoethanolamine-N-[3-(2-pyridyldithio) propionate] (DOPE-PDP), 1,2-dipalmitoyl-*sn-*glycero-3-phosphothioethanol, 1,2-di-(9Z-octadecenoyl)-*sn*-glycero-3-phosphoethanolamine-N-[4-(p-maleimidophenyl)butyramide], 1,2-dihexadecanoyl-*sn*-glycero-3-phosphoethanolamine-N-[4-(p-maleimidophenyl)butyramide], 1,2-dihexadecanoyl-*sn-*glycero-3-phosphoethanolamine-N-[4-(p-maleimidomethyl)cyclohexane-carboxamide], 1,2-di-(9Z-octadecenoyl)-*sn*-glycero-3-phosphoethanolamine-N-[4-(p-maleimidomethyl)cyclohexane-carboxamide], phosphatidylcholine, phosphatidylinositol, phosphatidylserine, phosphatidylethanolamine, and combinations thereof.

In some embodiments, exemplary phospholipids include, but are not limited to, small alkyl chain phospholipids, egg phosphatidylcholine, soybean phosphatidylcholine, dipalmitoylphosphatidylcholine, dimyristoylphosphatidylcholine, distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, dioleoylphosphatidylcholine dioleophosphatidylethanolamine, dilauroylphosphatidylglycerol phosphatidylcholine, phosphatidylserine, phosphatidylethanolamine, phosphatidylinositol, phosphatidylglycerols, diphosphatidylglycerols such as dimyristoylphosphatidylglycerol, dipalmitoylphosphatidylglycerol, distearoylphosphatidylglycerol, dioleoylphosphatidylglycerol, dimyristoylphosphatidic acid, dipalmitoylphosphatidic acid, dimyristoylphosphatidylethanolamine, dipalmitoylphosphatidylethanolamine, dimyristoylphosphatidylserine, dipalmitoylphosphatidylserine, brain phosphatidylserine, brain sphingomyelin, egg sphingomyelin, milk sphingomyelin, palmitoyl sphingomyelin, phytosphingomyelin, dipalmitoylsphingomyelin, distearoylsphingomyelin, dipalmitoylphosphatidylglycerol salt, phosphatidic acid, galactocerebroside, gangliosides, cerebrosides, dilaurylphosphatidylcholine, (1,3)-D-mannosyl-(1,3)diglyceride, aminophenylglycoside, 3-cholesteryl-6'-(glycosylthio)hexyl ether glycolipids, and cholesterol and its derivatives. Phospholipid fractions including SM and palmitoylsphingomyelin can optionally include small quantities of any type of lipid, including but not limited to lysophospholipids, sphingomyelins other than palmitoylsphingomyelin, galactocerebroside, gangliosides, cerebrosides, glycerides, triglycerides, and cholesterol and its derivatives.

In some embodiments, the sHDL nanoparticles have a molar ratio of phospholipid/ HDL apolipoprotein from 2 to 250 (e.g., 10 to 200, 20 to 100, 20 to 50, 30 to 40).

The present invention is not limited to a particular manner of generating sHDL-ML355 nanoparticles. In some embodiments, for example, such sHDL-ML355 nanoparticles are formed by mixing an amphipathic lipid and the ML355 in a solvent. The solvent is then removed and the dried lipid mixture is hydrated with an aqueous buffer. HDL apolipoprotein is then added and the composition is mixed vigorously to effect the formation of the sHDL-ML355 nanoparticles.

In some embodiments, the sHDL-ML355 nanoparticles are prepared by co-lyophilization methods. For example, in some embodiments, lipids, ApoA mimetic peptides and ML355 will be dissolved (e.g., in glacial acetic acid) and lyophilized. The obtained powder will be hydrated in PBS (e.g., pH 7.4) and thermocycled above and below the phospholipid transition temperature to form drug-loaded sHDL.

Generally, the sHDL-ML355 nanoparticles so formed are spherical and have a diameter of from about 5 nm to about 20 nm (e.g., 4 - 22 nm, 6 - 18 nm, 8 - 15 nm, 8- 10 nm, etc.). In some embodiments, the sHDL-ML355 nanoparticles are subjected to size exclusion chromatography to yield a more homogeneous preparation.

In some embodiments, the sHDL-ML355 nanoparticles are prepared by a thin-film dispersion method. For example, in some embodiments, lipid (e.g., approximately 15 mg lipid) is dissolved in chloroform (e.g., approximately 2 ml chloroform) and mixed with ML355 stock DMSO solution (e.g., approximately 2.5 mg/mL drug stock DMSO solution). In some embodiments, organic solvent is evaporated and buffer (50 mM acetate buffer, pH 5.0) added into the lipid/drug mixture to hydrate the film by probe sonication in intervals (e.g., 30 second intervals) using an ultrasonic processor (e.g., a VibraCell ultrasonic processor (Sonics, Newtown, CT)). In some embodiments, apolipoprotein is dissolved in buffer and mixed with the lipid suspension. Next, in some embodiments, the mixture is incubated in water bath (e.g., 50°C water bath for 5 min) and cooled (e.g., cooled at room temperature for 5 min). In some embodiments, the water bath / cooling is repeated (e.g., cycled three times) to form sHDL-ML355 nanoparticles.

In some embodiments, the sHDL-ML355 nanoparticles are prepared by mixing (e.g., vortexing) (e.g., ultraturrexing) buffer with powder formulations of peptide, lipid and ML355. In some embodiments, the mixture is further incubated at or about the lipid phase transition temperature until sHDL-ML355 assembly is complete.

Generally, the sHDL-ML355 nanoparticles so formed are spherical and have a diameter of from about 5 nm to about 20 nm (e.g., 4 - 22 nm, 6 - 18 nm, 8 - 15 nm, 8- 10 nm, etc.). In some embodiments, the sHDL-ML355 nanoparticles are subjected to size exclusion chromatography to yield a more homogeneous preparation.

In some embodiments, the sHDL nanoparticles further encapsulate agents useful for determining the location of administered particles. Agents useful for this purpose include fluorescent tags, radionuclides and contrast agents.

Suitable imaging agents include, but are not limited to, fluorescent molecules such as those described by Molecular Probes (Handbook of fluorescent probes and research products), such as Rhodamine, fluorescein, Texas red, Acridine Orange, Alexa Fluor (various), Allophycocyanin, 7-aminoactinomycin D, BOBO-1, BODIPY (various), Calcien, Calcium Crimson, Calcium green, Calcium Orange, 6-carboxyrhodamine 6G, Cascade blue, Cascade yellow, DAPI, DiA, DID, Di1, DiO, DiR, ELF 97, Eosin, ER Tracker Blue-White, EthD-1, Ethidium bromide, Fluo-3, Fluo4, FM1-43, FM4-64, Fura-2, Fura Red, Hoechst 33258, Hoechst 33342, 7-hydroxy-4-methylcoumarin, Indo-1, JC-1, JC-9, JOE dye, Lissamine rhodamine B, Lucifer Yellow CH, LysoSensor Blue DND-167, LysoSensor Green, LysoSensor Yellow/Blu, Lysotracker Green FM, Magnesium Green, Marina Blue, Mitotracker Green FM, Mitotracker Orange CMTMRos, MitoTracker Red CMXRos, Monobromobimane, NBD amines, NeruoTrace 500/525 green, Nile red, Oregon Green, Pacific Blue. POP-1, Propidium iodide, Rhodamine 110, Rhodamine Red, R-Phycoerythrin, Resorfin, RH414, Rhod-2, Rhodamine Green, Rhodamine 123, ROX dye, Sodium Green, SYTO blue (various), SYTO green (Various), SYTO orange (various), SYTOX blue, SYTOX green, SYTOX orange, Tetramethylrhodamine B, TOT-1, TOT-3, X-rhod-1, YOYO-1, YOYO-3. In some embodiments, ceramides are provided as imaging agents. In some embodiments, S1P agonists are provided as imaging agents.

Additionally radionuclides can be used as imaging agents. Suitable radionuclides include, but are not limited to radioactive species of Fe(III), Fe(II), Cu(II), Mg(II), Ca(II), and Zn(I1) Indium, Gallium and Technetium. Other suitable contrast agents include metal ions generally used for chelation in paramagnetic T1-type MIR contrast agents, and include di- and tri-valent cations such as copper, chromium, iron, gadolinium, manganese, erbium, europium, dysprosium and holmium. Metal ions that can be chelated and used for radionuclide imaging, include, but are not limited to metals such as gallium, germanium, cobalt, calcium, indium, iridium, rubidium, yttrium, ruthenium, yttrium, technetium, rhenium, platinum, thallium and samarium. Additionally metal ions known to be useful in neutron-capture radiation therapy include boron and other metals with large nuclear cross-sections. Also suitable are metal ions useful in ultrasound contrast, and X-ray contrast compositions.

Examples of other suitable contrast agents include gases or gas emitting compounds, which are radioopaque.

In some embodiments, the sHDL-ML355 nanoparticles further encapsulate a targeting agent. In some embodiments, targeting agents are used to assist in delivery of the sHDL-ML355 nanoparticles to desired body regions (e.g., bodily regions affected by a cardiovascular related disorder). Examples of targeting agents include, but are not limited to, an antibody, receptor ligand, hormone, vitamin, and antigen, however, the present invention is not limited by the nature of the targeting agent. In some embodiments, the antibody is specific for a disease-specific antigen. In some embodiments, the receptor ligand includes, but is not limited to, a ligand for CFTR, EGFR, estrogen receptor, FGR2, folate receptor, IL-2 receptor, glycoprotein, and VEGFR. In some embodiments, the receptor ligand is folic acid.

In some embodiments, the sHDL-ML355 nanoparticles further encapsulate transgenes for delivery and expression to a target cell or tissue, *in vitro, ex vivo,* or *in vivo.* In such embodiments, rather than containing the actual protein, the sHDL-ML355 nanoparticles encapsulate an expression vector construct containing, for example, a heterologous DNA encoding a gene of interest and the various regulatory elements that facilitate the production of the particular protein of interest in the target cells.

In some embodiments, the gene is a therapeutic gene that is used, for example, to treat cardiovascular related disorders, to replace a defective gene, or a marker or reporter gene that is used for selection or monitoring purposes. In the context of a gene therapy vector, the gene may be a heterologous piece of DNA. The heterologous DNA may be derived from more than one source (i.e., a multigene construct or a fusion protein). Further, the heterologous DNA may include a regulatory sequence derived from one source and the gene derived from a different source. Tissue-specific promoters may be used to effect transcription in specific tissues or cells so as to reduce potential toxicity or undesirable effects to non-targeted tissues. The nucleic acid may be either cDNA or genomic DNA. The nucleic acid can encode any suitable therapeutic protein.

The nucleic acid may be an antisense nucleic acid. In such embodiments, the antisense nucleic acid may be incorporated into the nanoparticle of the present invention outside of the context of an expression vector.

In some embodiments, the sHDL-ML355 nanoparticles of the present invention may be delivered to local sites in a patient by a medical device. Medical devices that are suitable for use in the present invention include known devices for the localized delivery of therapeutic agents. Such devices include, but are not limited to, catheters such as injection catheters, balloon catheters, double balloon catheters, microporous balloon catheters, channel balloon catheters, infusion catheters, perfusion catheters, etc., which are, for example, coated with the therapeutic agents or through which the agents are administered; needle injection devices such as hypodermic needles and needle injection catheters; needleless injection devices such as jet injectors; coated stents, bifurcated stents, vascular grafts, stent grafts, etc.; and coated vaso-occlusive devices such as wire coils.

Exemplary devices are described in U.S. Pat. Nos. 5,935,114; 5,908,413; 5,792,105; 5,693,014; 5,674,192; 5,876,445; 5,913,894; 5,868,719; 5,851,228; 5,843,089; 5,800,519; 5,800,508; 5,800,391; 5,354,308; 5,755,722; 5,733,303; 5,866,561; 5,857,998; 5,843,003; and 5,933,145. Exemplary stents that are commercially available and may be used in the present application include the RADIUS (SCIMED LIFE SYSTEMS, Inc.), the SYMPHONY (Boston Scientific Corporation), the Wallstent (Schneider Inc.), the PRECEDENT II (Boston Scientific Corporation) and the NIR (Medinol Inc.). Such devices are delivered to and/or implanted at target locations within the body by known techniques.

In certain embodiments, the present invention provides compositions for use in methods of preventing, attenuating or treating thrombosis in a subject having or at risk for having conditions and symptoms caused by thrombosis, comprising administering to the subject such a composition comprising one or more sHDL-ML355 moieties. In some embodiments, administration of the composition results in, for example, reduction of platelet activity, reduction of platelet aggregation, prevention of thrombus formation, reduction of vessel occlusion, and reduction of platelet associated 12-LOX activity.

In some embodiments, the conditions and symptoms caused by thrombosis are related to a venous thrombosis. In some embodiments, the conditions and symptoms caused by thrombosis are related to an arterial thrombosis.

In some embodiments, thrombosis is a feature of an underlying disease or condition. Non-limiting examples of such disease or condition include acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, thromboembolic stroke, systemic embolism, ischemic stroke, venous thromboembolism, atrial fibrillation, non-valvular atrial fibrillation, atrial flutter, transient ischemic attacks, venous thrombosis, deep venous thrombosis, pulmonary embolus, coagulopathy, disseminated intravascular coagulation, thrombotic thrombocytopenic purpura, thromboanglitis obliterans, thrombotic disease associated with heparin-induced thrombocytopenia, thrombotic complications associated with extracorporeal circulation, thrombotic complications associated with instrumentation, thrombotic complications associated with the fitting of prosthetic devices, occlusive coronary thrombus formation resulting from either thrombolytic therapy or percutaneous transluminal coronary angioplasty, thrombus formation in the venous vasculature, disseminated intravascular coagulopathy, a condition wherein there is rapid consumption of coagulation factors and systemic coagulation which results in the formation of life-threatening thrombi occurring throughout the microvasculature leading to widespread organ failure, hemorrhagic stroke, renal dialysis, blood oxygenation, and cardiac catheterization.

In some embodiments, the conditions and symptoms caused by thrombosis are selected from the group consisting of embolic stroke, thrombotic stroke, venous thrombosis, deep venous thrombosis, acute coronary syndrome, and myocardial infarction.

In some embodiments for preventing, attenuating or treating a subject having or at risk for having conditions and symptoms caused by thrombosis, the composition comprising one or more sHDL-ML355 moeities is co-administered with one or more of the following therapeutic agents: heparin; tPA; anistreplase; streptokinase; urokinase; a coumadin; warfarin; idraparinux; fondaparinux; aspririn; an adenosine diphosphate receptor inhibitor; a phosphodiesterase inhibitor; a glycoprotein IIB/IIA inhibitor; an adenosine reuptake inhibitor; and a thromboxane receptor antagonist.

In such embodiments for preventing, attenuating, and/or treating thrombosis in a subject, the administering to the subject a therapeutically effective amount of a composition comprising one or more sHDL-ML355 moeities comprises a continuous infusion of the composition and/or a non-continuous infusion of the composition.

In some embodiments, the subject is a human being.

In certain embodiments, the present invention provides compsitions for use in methods of preventing, attenuating or treating a subject (e.g., a human subject) having a cardiovascular related disorder, comprising administering to the subject a therapeutically effective amount of such a composition comprising one or more sHDL-ML355 moieties.

In certain embodiments, the present invention provides compsitions for use in methods of preventing, attenuating or treating increased platelet activity in a subject (e.g., a human subject) having or at risk for having increased platelet activity, comprising administering to the subject such a composition comprising one or more sHDL-ML355 moieties.

In certain embodiments, the present invention provides compsitions for use in methods of preventing, attenuating or treating platelet aggregation in a subject (e.g., a human subject) having or at risk for having platelet aggregation, comprising administering to the subject such a composition comprising one or more sHDL-ML355 moieties.

In certain embodiments, the present invention provides compsitions for use in methods of preventing, attenuating or treating vessel occlusion (e.g., arterial and/or venous) in a subject (e.g., a human subject) having or at risk for having vessel occlusion, comprising administering to the subject such a composition comprising one or more sHDL-ML355 moieties.

In certain embodiments, the present invention provides compsitions for use in methods of preventing, attenuating or treating increased platelet associated 12-LOX activity in a subject (e.g., a human subject) having or at risk for having increased platelet associated 12-LOX activity, comprising administering to the subject such a composition comprising one or more sHDL-ML355 moieties.

The present application further discloses kits comprising sHDL-ML355 nanoparticles as described herein, which may be useful for illustrative purposes and to provide additional technical context for the disclosure. In some embodiments, the kits comprise one or more of the reagents and tools necessary to generate sHDL-ML355 nanoparticles, and methods of using such sHDL-ML355 nanoparticles.

The sHDL-ML355 nanoparticles of the present invention may be characterized for size and uniformity by any suitable analytical techniques. These include, but are not limited to, atomic force microscopy (AFM), electrospray-ionization mass spectroscopy, MALDI-TOF mass spectroscopy, ¹³C nuclear magentic resonance spectroscopy, high performance liquid chromatography (HPLC) size exclusion chromatography (SEC) (equipped with multi-angle laser light scattering, dual UV and refractive index detectors), capillary electrophoresis and get electrophoresis. These analytical methods assure the uniformity of the sHDL-ML355 nanoparticle population and are important in the production quality control for eventual use in in vivo applications.

In some embodiments, gel permeation chromatography (GPC), which can separate sHDL nanoparticles from liposomes and free ApoA-I mimetic peptide, is used to analyze the sHDL-ML355 nanoparticles. In some embodiments, the size distribution and zeta-potential is determined by dynamic light scattering (DLS) using, for example, a Malven Nanosizer instrument.

In some embodiments, the encapsulation efficiency of the ML355 will be determined by a desalting column method. Briefly, a sHDL-ML355 nanoparticle will be passed through a desalting column (cut off = 7000 Da) to remove any unencapsulated ML355, and an equal volume of a sHDL-ML355 nanoparticle that is not subject to desalting will be used as a comparison. All samples will be incubated with ethanol to break sHDL and subsequently analyzed by HPLC equipped with a C18 column³⁹. In some embodiments, an equation is used to calculate encapsulation efficiency. In some embodiments, the following equation is used to calculate the encapsulation efficiency: Encapsulation efficiency (%) = (the content of drug in sHDL passed through the desalting column)/(the content of ML355 in sHDL not passed through the desalting column) × 100%.

In some embodiments, to learn the release profile of ML355 from sHDL, sHDL-ML355 nanoparticles and free ML355 are placed into a dialysis bag (6-8kda), which will be put in 200 ml PBS (pH 7.4) containing 0.1% Tween 80⁴⁰. The release media will be put in a 37°C air bath shaker at 100 rpm. In some embodiments, at predetermined time points, 2 ml of the medium will be sampled and replaced with an equal volume of fresh release media. The amount of ML355 in the media will be quantified by reverse-phase HPLC.

In some embodiments, the sHDL-ML355 nanoparticles of the present invention are configured such that they are readily cleared from a subject (e.g., so that there is little to no detectable toxicity at efficacious doses).

Where clinical applications are contemplated, in some embodiments of the present invention, the sHDL-ML355 nanoparticles are prepared as part of a pharmaceutical composition in a form appropriate for the intended application. Generally, this entails preparing compositions that are essentially free of pyrogens, as well as other impurities that could be harmful to humans or animals. However, in some embodiments of the present invention, a straight sHDL-ML355 nanoparticle formulation may be administered using one or more of the routes described herein.

In preferred embodiments, the sHDL-ML355 nanoparticles are used in conjunction with appropriate salts and buffers to render delivery of the compositions in a stable manner to allow for uptake by target cells. Buffers also are employed when the sHDL-ML355 nanoparticles are introduced into a patient. Aqueous compositions comprise an effective amount of the sHDL-ML355 nanoparticles to cells dispersed in a pharmaceutically acceptable carrier or aqueous medium. Such compositions also are referred to as inocula. The phrase "pharmaceutically or pharmacologically acceptable" refer to molecular entities and compositions that do not produce adverse, allergic, or other untoward reactions when administered to an animal or a human. As used herein, "pharmaceutically acceptable carrier" includes any and all solvents, dispersion media, coatings, antibacterial and antifungal agents, isotonic and absorption delaying agents and the like. Except insofar as any conventional media or agent is incompatible with the vectors or cells of the present invention, its use in therapeutic compositions is contemplated. Supplementary active ingredients may also be incorporated into the compositions.

In some embodiments of the present invention, the active compositions include classic pharmaceutical preparations. Administration of these compositions according to the present invention is via any common route so long as the target tissue is available via that route. This includes oral, nasal, buccal, rectal, vaginal or topical. Alternatively, administration may be by orthotopic, intradermal, subcutaneous, intramuscular, intraperitoneal or intravenous injection.

The active sHDL-ML355 nanoparticles may also be administered parenterally or intraperitoneally or intratumorally. Solutions of the active compounds as free base or pharmacologically acceptable salts are prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

In some embodiments, a ML355 moiety is released from the sHDL-ML355 nanoparticles within a target cell (e.g., within a vascular region) (e.g., within a platelet).

The pharmaceutical forms suitable for injectable use include sterile aqueous solutions or dispersions and sterile powders for the extemporaneous preparation of sterile injectable solutions or dispersions. The carrier may be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial an antifungal agents, for example, parabens, chlorobutanol, phenol, sorbic acid, thimerosal, and the like. In many cases, it may be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active sHDL-ML355 nanoparticles in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, sHDL-ML355 nanoparticles are administered in a manner compatible with the dosage formulation and in such amount as is therapeutically effective. The formulations are easily administered in a variety of dosage forms such as injectable solutions, drug release capsules and the like. For parenteral administration in an aqueous solution, for example, the solution is suitably buffered, if necessary, and the liquid diluent first rendered isotonic with sufficient saline or glucose. These particular aqueous solutions are especially suitable for intravenous, intramuscular, subcutaneous and intraperitoneal administration. For example, one dosage could be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). In some embodiments of the present invention, the active particles or agents are formulated within a therapeutic mixture to comprise about 0.0001 to 1.0 milligrams, or about 0.001 to 0.1 milligrams, or about 0.1 to 1.0 or even about 10 milligrams per dose or so. Multiple doses may be administered.

Additional formulations that are suitable for other modes of administration include vaginal suppositories and pessaries. A rectal pessary or suppository may also be used. Suppositories are solid dosage forms of various weights and shapes, usually medicated, for insertion into the rectum, vagina or the urethra. After insertion, suppositories soften, melt or dissolve in the cavity fluids. In general, for suppositories, traditional binders and carriers may include, for example, polyalkylene glycols or triglycerides; such suppositories may be formed from mixtures containing the active ingredient in the range of 0.5% to 10%, preferably 1%-2%. Vaginal suppositories or pessaries are usually globular or oviform and weighing about 5 g each. Vaginal medications are available in a variety of physical forms, e.g., creams, gels or liquids, which depart from the classical concept of suppositories. The sHDL-ML355 nanoparticles also may be formulated as inhalants.

The present invention also includes compsitions for use in methods involving coadministration of the sHDL-ML355 nanoparticles as described herein with one or more additional active agents. Indeed, it is a further aspect of this invention to provide compsitions for use in methods for enhancing prior art therapies and/or pharmaceutical compositions by co-administering the sHDL-ML355 nanoparticles of this invention. In coadministration procedures, the agents may be administered concurrently or sequentially. In some embodiments, the sHDL-ML355 nanoparticles described herein are administered prior to the other active agent(s). The agent or agents to be co-administered depends on the type of condition being treated. For example, when the condition being treated is a cardiovascular related disorder, the additional agent includes angiotensin-converting enzyme (ACE) inhibitors (e.g., benazepril, enalapril, Lisinopril, perindopril, Ramipril), adenosine, alpha blockers (alpha adrenergic antagonist medications) (e.g., clonidine, guanabenz, labetalol, phenoxybenzamine, terazosin, doxazosin, guanfacine, methyldopa, prazosin), angtiotensin II receptor blockers (ARBs) (e.g., candesartan, irbesartan, olmesartan medoxomil, telmisartan, eprosartan, losartan, tasosartan, valsartan), antiocoagulants (e.g., heparin fondaparinux, warfarin, ardeparin, enoxaparin, reviparin, dalteparin, nadroparin, tinzaparin), antiplatelet agents (e.g., abciximab, clopidogrel, eptifibatide, ticlopidine, cilostazol, dipyridamole, sulfinpyrazone, tirofiban), beta blockers (e.g., acebutolol, betaxolol, carteolol, metoprolol, penbutolol, propranolol, atenolol, bisoprolol, esmolol, nadolol, pindolol, timolol), calcium channel blockers (e.g., amlopidine, felodipine, isradipine, nifedipine, verapamil, diltiazem, nicardipine, nimodipine, nisoldipine), diuretics, aldosterone blockers, loop diuretics (e.g., bumetanide, furosemide, ethacrynic acid, torsemide), potassium-sparing diuretics, thiazide diuretics (e.g., chlorothiazide, chlorthalidone, hydrochlorothiazide, hydroflumethiazide, methyclothiazide, metolazone, polythiazide, quinethazone, trichlormethiazide), inoptropics, bile acid sequestrants (e.g., cholestyramine, coletipol, colesevelam), fibrates (e.g., clofibrate, gemfibrozil, fenofibrate), statins (e.g., atorvastatinm, lovastatin, simvastatin, fluvastatin, pravastatin), selective cholesterol absorption inhibitors (e.g., ezetimibe), potassium channel blockers (e.g., amidarone, ibutilide, dofetilide), sodium channel blockers (e.g., disopyramide, mexiletine, procainamide, quinidine, flecainide, moricizine, propafenone), thrombolytic agents (e.g., alteplase, reteplase, tenecteplase, anistreplase, streptokinase, urokinase), vasoconstrictors, vasodilators (e.g., hydralazine, minoxidil, mecamylamine, isorbide dintrate, isorbide mononitrate, nitroglycerin). The additional agents to be co-administered can be any of the well-known agents in the art, including, but not limited to, those that are currently in clinical use.

### EXPERIMENTAL

The following examples are provided in order to demonstrate and further illustrate certain preferred embodiments and aspects of the present invention and are not to be construed as limiting the scope thereof. As used herein, the terms "we," "I," "our," or similar personal pronouns refer to the inventors.

### Example I.

This example describes the preparation and characterization of ML355-sHDL. ML355, a selective 12-LOX inhibitor, has an estimated solubility of < 5 µg/mL in pH 7.4 PBS (*43*). Its low water solubility necessitates the addition of solubilizing agents when administered orally and intravenously. Despite ML355's effective antithrombotic effects observed in previous animal studies (*42, 44*), sustained strong inhibition of thrombus formation in the laser-induced cremaster arteriole model required frequent dosing. This dosing regimen could be ascribed to non-specific delivery. To increase the site selectivity of the drug, we encapsulated ML355 into sHDL (illustrated in Fig. 2A), termed ML355-sHDL. sHDL is composed of ApoA1 mimetic peptide (22-amino acid peptide, 22A) and 1,2-dimyristoyl-sn-glycero-3-phosphocholine (DMPC), both of which are clinically validated biomaterials with a long history of safe use in humans (*28*). The discoidal structure of sHDL was retained upon ML355 loading as was observed by transmission electron microscopy (Fig. 2B). Dynamic light scattering (Fig. 2C) and gel permeation chromatography (Fig. 2D) revealed a homogeneous size distribution of 10.3 ± 1.6 nm and 10.6 ± 0.7 nm for both sHDL and ML355-sHDL, respectively, suggesting minimal impact of drug loading on the size and homogeneity of ML355-sHDL. The optimal formulation of ML355-sHDL was determined to be 20 mg/mL of DMPC, 10 mg/mL of 22A, and 0.5 mg/mL of ML355. Additionally, the solubility of ML355 was enhanced roughly 100-fold after sHDL encapsulation (*43*). Compared to the ML355 in solution, ML355 release from sHDL was investigated *in vitro* under physiological conditions (pH 7.4 PBS at 37 °C) and displayed a sustained release profile. Additionally, the presence of serum in the release medium had no significant effect on the ML355 release from ML355-sHDL, indicating the stability of ML355-sHDL in serum (Fig. 2E).

To assess the intercellular uptake of sHDL by platelets, washed mouse platelets were collected and prepared as described previously (*44, 45*). Washed mouse human platelets were incubated with DiO-sHDL (sHDL at 50 µg/mL and DiO at 2.5 µg/mL) for 5, 15, 30 and 60 minutes, respectively, and the uptake of DiO-sHDL by platelets was monitored by fluorescent microscopy. The Alexa Fluor^{®} 647 Anti-CD41 antibody was used to label the membrane of mouse platelets. The laser scanning confocal images clearly showed that sHDL was internalized by platelets after 5 minutes incubation. The fluorescence signal (Fig. 2F) was detected after 5 minutes incubation, and the signal reached a maximum after 15 minutes incubation. This suggested that sHDL was specifically internalized by mouse platelets and that uptake was saturated after 15 minutes incubation (30 minutes incubation did not further increase the fluorescent signal in mouse platelets). The sHDL uptake profile in mouse platelets was also quantitatively verified by flow cytometry (shown in Fig. 2G). In addition, our data (Fig. 3) also showed that pre-activated platelets were able to uptake sHDL to a similar extent compared to resting platelets. We next sought to determine the specific uptake of sHDL by platelets *in vivo* using flow cytometry analysis of whole blood. Both male and female C57BL/6J mice (n = 4) were dosed with DiO-sHDL (sHDL at 50 mg/kg and DiO at 2.5 mg/kg) via tail vein injection, and blood was collected at predetermined time points. Platelets were stained with CD41-PE antibody. Platelet uptake of sHDL over time is shown in Fig. 2H. The fluorescent signal of sHDL in platelets rapidly increased *in vivo* then gradually decreased after 15 minutes through 96 hours post-administration Fig. 2I. These results indicate that sHDL is internalized by platelets *in vivo* and is retained for up to 72 hours. Internalization of sHDL by platelets was further confirmed *in vivo* by injecting dual labeled Dil-488-sHDL (labeling the 22A peptide in sHDL with AlexaFluor 488 dye and the lipid bilayer in sHDL with cell-labeling fluorophore Dil) in mice. Our results showed that both lipid and peptide were internalized in consistent with our *in vitro* platelet uptake of sHDL. Interestingly, we observed that red blood cells and neutrophils were able to uptake sHDL *in vivo* in mice following sHDL treatment. However, the sHDL uptake by platelets are notably higher than in red blood cells and neutrophils in circulation. (see Fig. 4).

### Example II.

This example demonstrates that ML355-sHDL treatment inhibits human platelet aggregation *in vitro.*

To examine whether sHDL or ML355-sHDL modulates the platelet functions and inhibitory effects on platelet aggregation to a greater extent than that of ML355 alone, we performed *in vitro* studies on isolated, washed human platelets. Among these results shown in Fig. 5A, ML355-sHDL showed the strongest inhibition of platelet aggregation relative to control (0.4 ± 0.2% aggregation at 0.1 nM thrombin, *P* < 0.001, and 20.3 ± 2.4% aggregation at 0.25 nM thrombin, *P* < 0.01), followed by ML355 (5.6 ± 2.5% aggregation at 0.1 nM thrombin, *P* < 0.01, and 40.4 ± 6.8% aggregation at 0.25 nM thrombin, *P* < 0.01) and sHDL (20.8 ± 5.2% aggregation at 0.1 nM thrombin, P < 0.01, and 70.7 ± 9.1% aggregation at 0.25 nM thrombin, *P* < 0.05). At 0.5 nM thrombin, only ML355 (76.4 ± 4.7%, *P* < 0.05) and ML355-sHDL (56.4 ± 3.6%, *P* < 0.001) showed inhibitory effects on platelet aggregation relative to control, while sHDL did not significantly inhibit thrombin-induced platelet aggregation (81.2 ± 3.0%). In contrast, the inhibition of platelet aggregation at the highest concentration of thrombin (1 nM) was not observed for any of the treatments. Taken together, these data suggest that sHDL exerts an inhibitory effect on thrombin-induced platelet activation, and ML355 entrapment by sHDL (ML355-sHDL) exhibits a preferred inhibitory profile on platelet activation relative to either sHDL or ML355.

### Example III.

This example demonstrates that platelet aggregation was inhibited in mice treated with ML355-sHDL.

To determine the regulatory effect of sHDL on mouse platelet aggregation *ex vivo,* mice were intravenously treated with ML355, sHDL, or ML355-sHDL. After 24 hours, platelets were isolated from the blood and subjected to the aggregation assay. Similar to *in vitro* results, all groups effectively inhibited platelet aggregation compared to platelets from vehicle control-treated mice at both 0.1 nM and 0.25 nM thrombin (Fig. 5B). Among them, ML355-sHDL exhibited better inhibition of thrombin-induced platelet aggregation. Specifically, at 0.1 nM thrombin stimulation, platelets from mice treated with ML355-sHDL exhibited the least amount of aggregation (12.2 ± 4.1%, *P* < 0.001), platelets isolated from mice treated with ML355 exhibited 20.3 ± 3.5% aggregation (*P* < 0.001), and platelets isolated from mice treated with sHDL exhibited 43.3 ± 9.4% aggregation (*P* < 0.05); at 0.25 nM thrombin stimulation, platelets from mice treated with ML355-sHDL only exhibited 20.3 ± 3.5% aggregation (*P* < 0.001), platelets isolated from mice treated with ML355 showed 32.6 ± 6.6% aggregation (*P* < 0.01), and platelets isolated from mice treated with sHDL had 47.1 ± 5.5% aggregation (*P* < 0.05). Again, the inhibitory effects of ML355-sHDL, ML355, and sHDL on platelet aggregation were overcome by high concentrations of thrombin (0.5 nM), which is consistent with our *in vitro* platelet aggregation results and suggests that the normal hemostatic responses to acute bleeding injuries remain intact and unaffected with both ML355-sHDL and sHDL treatment.

### Example IV.

This example describes the pharmacokinetics of ML355-sHDL.

ML335 pharmacokinetics were improved by the incorporation of ML355 into sHDL. The plasma concentration of ML355 was determined by liquid chromatography-mass spectrometry (LC/MS) following intravenous administration of ML355-sHDL or ML355. The concentration curves in Fig. 6 show that ML355-sHDL (3 mg/kg, IV) has a longer circulation time compared to ML355 alone, suggesting that the encapsulation of ML355 into sHDL extends its blood circulation time.

### Example V.

This example describes an *in vivo* examination of sHDL's thrombus targeting ability.

sHDL's significant inhibition of platelet aggregation and long circulation time *in vivo* led us to investigate whether sHDL could specifically target thrombi. Here, we tested whether DiO-sHDL could accumulate at the site of a thrombus in mice using an endothelial damage-induced thrombus model (*44*). DiO-sHDL was administered through an IV, and after 24 hours, prior to thrombus formation, platelet marker DyLight 647-conjugated rat anti-mouse platelet GP1bβ antibody was administered. Following laser ablation and thrombus formation, we found that DiO-sHDL and DyLight 647-conjugated rat anti-mouse platelet GP1bβ antibody were co-localized within the newly formed thrombi, suggesting that sHDL may facilitate the targeted delivery of antithrombotic agents (Fig. 7A). In addition, the co-localization of sHDL within the thrombus was examined under confocal intravital microscopy and further evaluated using a 3D constructed model of the thrombus. As shown in Fig. 7B sHDL (green) mostly accumulated within the core regions of the thrombus which consists of densely packed platelets (red). Furthermore, our results also indicated that sHDL adhered to the injured endothelium surrounding the thrombus.

### Example V.

This example describes the effect of ML355-sHDL in a laser-induced cremaster arteriole thrombosis model.

Now that we have shown sHDL's ability to reduce platelet aggregation, improve ML355's circulation time, and target thrombi *in vivo,* we sought to determine ML355-sHDL's ability to inhibit thrombus formation *in vivo.* Using the laser injury cremaster arterial thrombosis model, we studied the dynamics of fluorescence-labeled platelet accumulation in growing thrombi. Thrombus formation was quantitatively assessed by real-time measurements of platelet recruitment at the site of injury in mice pretreated with different formulations 24 hours prior to vessel injury. As shown in representative images of thrombus formation in Fig. 8A and quantitative analysis of dynamic platelet recruitment within thrombi in Fig. 8B, all of the treatment conditions (ML355, sHDL and ML355-sHDL) exhibited varying levels of inhibition of thrombus formation. Notably, encapsulation of ML355 within sHDL (ML355-sHDL) exhibited synergetic inhibition on thrombus formation.

### Example VI.

This example describes the effect of ML355-sHDL in a FeCl₃-induced carotid artery thrombosis model.

In order to confirm the inhibitory effect of ML355, sHDL or ML355-sHDL treatment on thrombus formation and vessel occlusion in a large artery with severe injury, mice treated with ML355, sHDL or ML355-sHDL respectively, were studied using a FeCl₃-induced carotid artery thrombosis model. Following the vascular injury induced by FeCl₃ application, platelets started to adhere to the injury site in the carotid artery and quickly formed visible platelet aggregates resulting in the formation of stable thrombi. Fig. 9A. Thrombi were stable, grew to larger sizes and reached vessel occlusion. There was no reopening of the occluded vessel in the control group. Thrombus growth was delayed and unstable in mice treated with either ML355 or sHDL, which resulted in a delay in vessel occlusion time. Thrombus growth and vessel occlusion were severely impaired in mice treated with ML355-sHDL as compared to other groups, which proved consistent with our microvascular thrombosis model. (Mean vessel occlusion time in ML355, sHDL and ML355-sHDL were 11.0 ± 2.3 min (*P* < 0.01), 11.3 ± 1.3 min (*P* < 0.01), and 21.4 ± 6.4 min (*P* < 0.001), respectively, while mean vessel occlusion time in control mice was only 7.1 ± 1.2 min).

### Example VII.

This example describes evaluation of hemostasis *in vivo.*

Determination of phosphatidylserine exposure on platelets post administration of different ML355 formulation shown in (Fig. 10A) suggested that both ML355, sHDL and ML355-sHDL treatment did not increase platelet phosphatidylserine exposure as it is comparable to the levels on resting platelets, indicating that sHDL treatment is not likely to cause platelet activation or platelet clearance. This result was further supported by our data showing that sHDL had no significant effect on platelet counts in mice (fig. 11). We also examined the effect of sHDL on coagulation and hemostatic clot formation using thromboelastography (TEG) and tail bleeding assays. Our results from the TEG analysis of whole blood collected from mice treated by different formulations (Fig. 10, B to F) showed that sHDL treatment in mice did not significantly alter the hemostatic parameters of clotting, indicating that ML355, sHDL and ML355-sHDL treatment did not compromise the coagulation. Furthermore, our study showed that sHDL, ML355, and ML355-sHDL did not prolong tail bleeding time and had no effect on blood loss evaluated by measuring hemoglobin (Fig. 10, G and H). Overall, all of the treatment conditions (ML355, sHDL and ML355-sHDL) treatment show inhibited thrombus formation without impairing hemostasis in mice.

### Example VIII.

This example provides a discussion related to Examples I-VII.

sHDL infusion has been previously demonstrated as an effective approach to inhibit platelet activation and arterial thrombosis in both mice (*33, 38*) and diabetic patients (*40*). The novelty of our approach is to utilize sHDL as a delivery vehicle for antithrombotic agents in order to enable the delivery of higher levels of the drug to sites of injury or inflammation while avoiding the off-target accumulation, thus achieving improved antithrombotic efficacy through synergistic effects without compromising hemostasis. Through this proof-of-concept study, we hope to further strengthen the antithrombotic application of sHDL. In addition to its proven antithrombotic effect, sHDL is a biomimetic nanoparticle used to mimic the *in vivo* biological activity of endogenous HDL, which is well recognized as protective in cardiovascular and chronic inflammatory diseases. We have previously loaded various therapeutics into sHDL for atherosclerosis and cancer applications (*29, 46, 47*). Thus, development of sHDL as an antithrombotic drug delivery vehicle is a sensible approach to improve the therapeutic potential of these drug molecules. ML355, a selective 12-LOX inhibitor, was previously assessed as a promising anti-platelet therapeutic *in vivo* (*44*). Despite efficient thrombus inhibition of orally administrated ML355 in multiple thrombosis models in our previous studies, the targeted delivery of ML355 may further enhance its efficacy and decrease any unforeseen off-target effects.

In the experiments described in Examples I-VII, we developed and investigated the effect of sHDL on platelet function *in vitro* and thrombus formation *in vivo* using mouse models of thrombosis and hemostasis. Furthermore, we encapsulated ML355 in our nano-based delivery system for targeted drug delivery at the site of vascular injury to enhance the drug's therapeutic efficacy in ordre to explore the potential application of advantages of sHDL in thrombotic disease. Preparation and characterization of ML355-sHDL showed that ML355 was encapsulated in sHDL, which displayed a typical discoidal structure and uniform distribution with nanoscale size. ML355-sHDL exhibited sustained release of ML355. Both *in vitro* human platelet uptake and *ex vivo* mouse platelet uptake studies clearly demonstrated that platelets specifically endocytosed sHDL. Moreover, sHDL and platelets were co-localized in our endothelial injury-induced thrombi in mice. Specific mechanisms of sHDL uptake and accumulation in thrombi have yet to be identified. Possible mechanisms for platelet uptake of sHDL could be mediated by the following, but are not limited to: 1) passive uptake of sHDL by platelets in blood, which is the fundamental principle for non-specific nanoparticle-directed drug delivery; 2) active internalization mediated by some unidentified proteins expressed on the surface of platelets, like scavenger receptors or glycoproteins; and 3) bonding of sHDL to the other components involved in thrombus formation, like von Willebrand Factor.

In addition, we found that sHDL exhibited a significant antiplatelet effect itself, inhibiting thrombin-induced platelet aggregation, which was consistent with previous reports (*35, 40*). Moreover, the encapsulation of ML355 within sHDL (ML355-sHDL) showed improved antiplatelet effects by combining the antiplatelet effects of ML355 and sHDL. Finally, a laser injury-induced cremaster arteriole thrombus was employed to investigate the antithrombotic efficacy of ML355-sHDL *in vivo.* The inhibition of thrombus formation was observed in mice treated with either ML355 or sHDL. The antithrombotic effects of sHDL here are consistent with the previous finding (*35*). While, the antithrombotic effects of sHDL *in vivo* appear to be due to the direct inhibitory effects on platelet activation (*32, 48*), other possible mechanisms, including, modulation of vascular endothelial cell function (*49*), cholesterol efflux (*34*, *50*), and prevention of von Willebrand factor self-association and subsequent platelet adhesion may play additional roles in its effect in the vessel (*33*). ML355-sHDL exhibits stronger thrombotic inhibition compared to ML355 and sHDL alone. The stronger thrombotic effect of ML355-sHDL is partially due to the combination of ML355 and sHDL working together, creating a synergistic effect. Additionally, by encapsulating ML355 in sHDL, sHDL limits wide drug exposure in the blood, delivering more of the drug into platelets that can then accumulate within the thrombus, thus enhancing the drug's therapeutic index. Finally, tail vein bleeding results suggested that ML355-sHDL effectively inhibits thrombosis without impairing hemostasis.

Taken together, the results presented here demonstrate the utility of targeting 12-LOX in the platelet with ML355 delivered by sHDL for the prevention of platelet activation and thrombus formation and warrant further development of ML355-sHDL for clinical translation. sHDL not only exerts its own antithrombotic effects, it additionally functions as an effective delivery vehicle for antithrombotic agents such as ML355.

Antiplatelet drugs, either administered as a mono- or polytherapy, are the first-choice therapy for the clinical treatment of cardiovascular disease and prevention of arterial thrombotic events. Current treatment options in clinical use have been limited to primarily cyclooxygenase-1, the ADP receptor (P2Y₁₂), and integrin receptor (αIIbβ3). Despite effectively reduced morbidity and mortality in the clinic, there are limitations associated with oral and intravenous administration of the currently approved antiplatelet agents, including an increased risk of bleeding, and delayed onset of action due to the requirement for *in vivo* conversion (like thienopyridines), irreversible inhibition (like thienopyridines) and delayed offset, as well as suboptimal platelet inhibition due to poor target specificity (*51*). Therefore, developing safer antiplatelet agents to rapidly and potently curtail thrombotic-associated events without increasing the risk of bleeding events in the gut and brain remains an unmet clinical need. Recent advances have identified a number of newer platelet pharmacological targets, including targeting surface receptors (glycoproteins and G protein-coupled receptors), oxygenases, and phosphodiesterases (*2*). However, most of those novel antiplatelet drugs are still in preclinical or early clinical stages of development. Among them, our preclinical studies have previously demonstrated the potential therapeutic benefits of selectively targeting 12-LOX with ML355 without notably impairing hemostasis (*44*). While oral administration is always preferred, some agents have low or variable bioavailability and large patient to patient variability in pharmacokinetics, which could lead to side effects especially when administered in an acute disease setting. Our current study shows that the preferential uptake of sHDL by platelets has served as a means of direct platelet targeting strategy, thus enhancing the antiplatelet effects of ML355. Importantly, ML355-sHDL showed favorable synergistic antithrombotic effects without increasing the bleeding risk in addition to a targeted delivery to the site of platelet-rich thrombi. In summary, delivering antiplatelet agents using sHDL as a vehicle may be a promising approach for the prevention of thrombotic events associated with cardiovascular disease such as heart attacks and strokes and may improve clinical outcomes.

### Example IX.

This example describes the materials and methods utilized in Examples I-VII.

### Materials

ApoA1 mimetic peptide 22A, PVLDLFRELLNELLEALKQKLK (SEQ ID NO: 4), was synthesized by Genscript Inc. (Piscataway, NJ). Peptide purity was determined to be >95% by reverse phase HPLC. Phospholipid 1,2-dimyristoyl-sn-glycero-3-phosphocholine (DMPC) was purchased from NOF America Corporation. ML355 was synthesized by the NIH Molecular Libraries Program, Bethesda, MD. For the washed human platelet aggregation assay, ML355 was dissolved in DMSO as a stock solution at 10 mM. For animal studies, ML355 was dissolved in a vehicle (5% DMSO, 10% Solutol, 20% PEG300, 65% PBS) and administrated to animals via intravenous injection immediately. All other materials were obtained from commercial sources.

### Preparation of washed human platelets

All studies involving human subjects have been reviewed and approved by the University of Michigan Institutional Review Board. Written informed consent was obtained from all healthy donors prior to the blood draws. Platelets were isolated as described previously (*44, 45*).

### Experimental Animals

All experimental procedures in this study were approved by the Institutional Animal Care and Use Committee at the University of Michigan. Both male and female C57BL/6 wild-type mice (10-12 weeks old) were purchased from Jackson Laboratories and were housed at 22 ± 1 °C in a 12:12 h light-dark cycle at the University of Michigan.

### Preparation and characterization of ML355-sHDL

ML355-sHDL was prepared using a lyophilization method that we previously developed (29). Briefly, DMPC and ApoA1 mimetic 22A peptide were dissolved in acetic acid, and ML355 was dissolved in dimethyl sulfoxide (DMSO). The solution was mixed at a 20:10:0.5 weight ratio and freeze dried for 24 hours. The lyophilized powder was hydrated in PBS and cycled between 55 °C and 4 °C (3 minutes for each cycle, and 3 thermal cycles) to obtain ML355-sHDL. The pH of the ML355-sHDL was adjusted to 7.4 by NaOH. The solution was passed through sterile filters (0.22 µm) and stored frozen at -20 °C until use. The final concentrations for 22A, DMPC, and ML355 were determined by liquid chromatography/mass spectrometry (LC/MS) to be 20, 10, and 0.5 mg/mL, respectively. *In vitro* release of ML355 from sHDL was investigated using Float-A-Lyzer G2 dialysis devices with 3,000 Da molecular weight cut-off (Spectrum) (*52, 53*). Briefly, 1 mL of ML355-sHDL was introduced into the dialysis membrane tube and then incubated in 50 mL of PBS with constant stirring (PBS buffer at 37 °C) that contained 0.5% sodium dodecyl sulfate to increase the solubility of ML355 in the external PBS media to maintain sink conditions. Additionally, ML355 (in DMSO) was tested in the above release medium to obtain a free drug release profile. In order to test the effect of serum on ML355 release from sHDL, 10% FBS was added with ML355-sHDL and further incubated in PBS buffer supplemented with 10% FBS. At predetermined intervals, buffer was drawn and replaced with an equal volume of fresh media. The concentration of ML355 was measured by HPLC (*43*). For observation of sHDL uptake by platelet *in vitro,* DiO (ThermoFisher) was encapsulated into sHDL to obtain DiO-sHDL using the same method above, and the final DiO-sHDL formulation contained 10 mg/mL of 22A peptide and 0.5 mg/mL of DiO. For investigation of biodistribution of sHDL in different major blood cell types *in vivo,* Dil-488-sHDL was prepared by dual labeling the 22A peptide in sHDL with AlexaFluor 488 dye using Invitrogen protein labeling kit (A10235) and labeling the lipid bilayer in sHDL with cell-labeling fluorophore DiI (V22889) following the manufacturer's instructions.

### sHDL uptake by washed mouse platelets

Washed mouse platelets were resuspended in Tyrode's buffer at 3×10⁶ platelets/mL. The mouse platelet suspension was incubated with DiO-sHDL (sHDL at 50 µg/mL and DiO at 2.5 µg/mL) for predetermined durations (5, 15, 30 and 60 minutes) at 37°C. After incubation, mouse platelets were washed with Tyrode's buffer twice and fixed with 4% paraformaldehyde followed by staining with Alexa Fluor 647 conjugated anti-mouse CD41 antibody (BioLegend#133934) before imaging with a confocal microscope (Nikon A1). For quantitative measurement of sHDL uptake by mouse platelets, after incubation with DiO-sHDL for different time points, mouse platelets were washed and mean fluorescent intensity of DiO in platelets were quantitatively analyzed by flow cytometry (ZE5^{™} Cell Analyzer, Bio-Rad) (*54*). Washed mouse platelets were treated with protease-activated receptor 4-activating peptide (PAR4-AP) (AYPGKF; AnaSpec, Fremont, CA, USA) to induce platelet activation, then incubated with DiO-sHDL as described above and sHDL uptake by activated platelets was determined and compared to unstimulated resting platelets.

### sHDL distribution in major blood cells in vivo

Both male and female C57BL/6J mice (n = 4) were intravenously dosed with Dil-488-sHDL (Dil at 0.5 mg/kg and Alexa Fluor 488 at 0.5 mg/kg). At different time points (from 5 minutes up to 24 hours), whole blood was collected and mean fluorescent intensity of both lipid tracer Dil and peptide tracer Alexa Fluor 488 in each cell type were analyzed by flow cytometry. Briefly, 5 µL of heparinized whole blood was collected and labeled with platelet marker Alexa Fluor^{®} 647 anti-mouse CD41 antibody (BioLegend #133934), red blood cell marker Alexa Fluor^{®} 647 anti-mouse TER-119 antibody (BioLegend #116218) and neutrophils marker Alexa Fluor^{®} 647 anti-mouse Ly-6G Antibody (BioLegend #116218), followed by flow cytometry as described previously (55).

### In vitro washed human platelet aggregation test

Human platelets were prepared and aggregation was assayed as described previously (*44, 45, 56*). Platelets were incubated with DMSO (equivalent volume to dissolve ML355) and treated with control, ML355 (10 µM), sHDL (100 µg/mL), or ML355-sHDL (sHDL at 100 µg/mL and ML355 at 10 µM) for 15 minutes. In addition, untreated platelets were included as control. Platelet aggregation was induced by various doses of thrombin (0.1-1 nM) as reported in previous studies (*44*). Aggregation was measured in response to thrombin with a lumi-aggregometer (Model 700D; Chrono-Log) under stirring conditions at 1100 rpm at 37 °C.

### Ex vivo washed mice platelet activation inhibition test

Male and female C57BL/6J mice were divided into four groups (n = 4) and intravenously dosed with the following formulations: 1) control vehicle (equivalent volume); 2) ML355 (1.5 mg/kg); 3) sHDL (50 mg/kg); or 4) ML355-sHDL (sHDL at 50 mg/kg and ML355 at 1.5 mg/kg). 24 hours after administration, mice were sacrificed under terminal anesthesia. Blood was drawn from the inferior vena cava using a syringe containing sodium citrate. Mouse platelet preparation was performed as previously described (*44, 45*). Murine platelets were resuspended at 3×10⁸ platelets/mL in Tyrode's buffer. Mouse platelet aggregation was induced by various doses of thrombin (0.1-0.5 nM) and measured with a lumi-aggregometer using the same method described above.

### Pharmacokinetic study

Male and female C57BL/6J mice were divided into two groups (n = 4) and dosed with the following formulations: intravenous administration of ML355 (3 mg/kg), or intravenous administration of ML355-sHDL (sHDL at 100 mg/kg and ML355 at 3 mg/kg). Plasma drug concentration was determined at different time points (0.25, 2, 8, 24 and 48 hours) and assessed by LC/MS analysis as previously described (*43*).

### In vivo thrombus targeting property of sHDL

Male C57BL/6J mice were chosen in this section due to the growing thrombus induced by the laser injury cremaster arterial thrombosis model. Male C57BL/6J mice (n = 3) were intravenously dosed with DiO-sHDL (sHDL at 50 mg/kg and DiO at 2.5 mg/kg). 24 hours after administration, mice were anesthetized by an intraperitoneal injection of ketamine/xylazine (100 mg/kg and 10 mg/kg, respectively) and the cremaster arteriole was externalized. The cremaster muscle was prepared and perfused with preheated bicarbonate-buffered saline throughout the experiment. DyLight 647-conjugated rat anti-mouse platelet GP1bβ antibody (0.1 µg/g; X649; EMFRET Analytics) was administered by jugular vein cannula prior to vascular injury. Multiple independent thrombi (8-10 thrombi per mouse) were induced in the arterioles (30-50 µm diameter) using a laser ablation system (Ablate! photoablation system; Intelligent Imaging Innovations). Images of thrombus formation at the site of injured arterioles were acquired in real-time by a Zeiss Axio Examiner Z1 fluorescent microscope equipped with a 63X water-immersion objective and a high-speed sCMOS camera. Furthermore, thrombus composition was examined under confocal intravital microscopy as described (*44, 56*).

### Laser-induced cremaster arteriole thrombosis model

Male C57BL/6J mice (n = 3) were intravenously dosed with the following treatments: 1) saline control (equivalent volume); 2) ML355 (1.5 mg/kg); 3) sHDL (50 mg/kg); or 4) ML355-sHDL (sHDL at 50 mg/kg and ML355 at 1.5 mg/kg). 24 hours post-administration, mice were anesthetized and surgically prepared as above described. DyLight 647-conjugated rat anti-mouse platelet GP1bβ antibody (0.1 µg/g; X649; EMFRET Analytics) was administered by jugular vein cannula prior to vascular injury. Multiple independent thrombi (8-10) were induced in the arterioles (30-50 µm diameter) in each mouse (3 mice per group) by a laser ablation system. All captured images were analyzed for change in fluorescent intensity over time of thrombus formation by subtracting fluorescent background defined on an uninjured section of the vessel using the Slidebook program. To monitor and compare dynamic thrombus formation among different treatment groups, the relative fluorescent intensity of Alexa Flour 647-labled platelets (recruited within thrombus) was plotted using the mean fluorescence at each time point. Data were evaluated for significance with two-way ANOVA and Mann-Whitney test for nonparametric data using Prism 6 software (Graphpad, La Jolla, CA, USA).

### FeCl₃-induced carotid artery thrombosis model

Male and female C57BL/6J mice (n = 6) were intravenously injected with vehicle control (equivalent volume of saline), ML355 (1.5 mg/kg), sHDL (50 mg/kg) or ML355-sHDL (sHDL at 50 mg/kg and ML355 at 1.5 mg/kg), respectively. 24 hours post-administration, mice were anesthetized as described above and tail veins were injected with a DyLight 488 anti-GPlb (1 µg/g; Emfret, Elbelstadt, Germany) to label circulating resting platelets in mice prior to intravital microscopy imaging. The mice were placed on a heating pad and the right common carotid artery was prepared under the dissecting microscope. Then, the mice were placed on the microscopic stage and blood flow in the carotid artery was visualized under 10X air objective using a Zeiss Axio Examiner Z1 upright fluorescent microscopy. Carotid artery injury was induced by topically placing a 10% FeCl₃ saturated Whatman paper for 3 minutes under recording. Images of platelet adhesion and the dynamics of thrombus formation were recorded for 30 minutes using a high-speed sCMOS camera using Slidebook 6.0. Vessel occlusion was defined by formation of an occlusive thrombus and cease of blood flow for 1 minutes or 30 minutes was taken as the vessel occlusion time if the carotid artery failed to occlude during the recording.

### Phosphatidylserine exposure on platelets

Male and female C57BL/6J mice (n = 6) were intravenously dosed with the following treatments: 1) Saline control (equivalent volume); 2) ML355 (1.5 mg/kg); 3) sHDL (50 mg/kg); or 4) ML355-sHDL (sHDL at 50 mg/kg and ML355 at 1.5 mg/kg). At different time points post administration (1, 6 and 24 hours), the phosphatidylserine-exposure over time course in platelets from different groups were quantified by flow cytometry. Briefly, whole blood was collected from the saphenous vein and incubated with PE anti-mouse CD41 Antibody (BioLegend#133906) for platelet labeling and Annexin V, Alexa Fluor^{™} 647 conjugate (phosphatidylserine-exposure) (Thermo Fisher Scientific). The mean fluorescent intensity of Annexin in platelets was quantified by flow cytometry. In addition, blood collected from mice prior to treatment was taken as resting platelets (negative control) and blood stimulated with PAR4-AP (200µM) was used as activated platelets (positive control).

### Mouse tail bleeding assay

Male and female C57BL/6J mice (n = 6) were intravenously dosed with the following treatments: 1) vehicle control (equivalent volume); 2) ML355 (1.5 mg/kg); 3) sHDL (50 mg/kg); or 4) ML355-sHDL (sHDL at 50 mg/kg and ML355 at 1.5 mg/kg). The tail bleeding assay was performed post 24 hour of treatment as previously described (*44, 56*). Briefly, mice were anesthetized as described above and placed on a heating pad. Five millimeters of tail tip was excised, and the tails were immediately immersed in 14 mL of sterile saline at 37 °C. Bleeding time was recorded as the cessation of blood flow from the tail for at least a minutes using a stopwatch. The amount of blood loss from tail tip was quantified by measuring hemoglobin using Drabkin's reagent (Sigma). Briefly, blood samples were pelleted at 500g for 10 minutes at room temperature, and the pellet was resuspended in 5 mL Drabkin's Reagent and incubated at room temperature for 15 minutes. Amount of hemoglobin lost was quantified by comparing the absorbance of the samples at 540 nm using SpectraMax i3 microplate reader (Molecular Devices LLC., San Jose, CA) to a standard curve of bovine hemoglobin in Drabkin's reagent.

### Coagulation tests by thromboelastography

Male and female C57BL/6J mice (n = 6) were intravenously injected with vehicle control (equivalent volume of saline), ML355 (1.5 mg/kg), sHDL (50 mg/kg) or ML355-sHDL (sHDL at 50 mg/kg and ML355 at 1.5 mg/kg), respectively. 24 hours after administration, mice were anesthetized by intraperitoneal injection of ketamine/xylazine mixture as described above. Whole blood was collected via vena cava using a 25G syringe containing sodium citrate 3.8% (blood to citrate volume ratio is 1:9). 340 µL citrated blood was mixed with 20 µL CaCl₂ (0.2 mol/L), and viscoelastic properties of whole blood clot formation was studied under low shear stress using a Heamoscope TEG 5000 Thrombelastograph Hemostasis Analyzer (Haemonetics Corp., Braintree, Massachusetts, USA) according to the manufacturer's instructions. Major coagulation parameters including R time (time to formation of the initial fibrin threads), Alpha angle (the rapidity with which the clot forms), K time (the time until the clot reaches a certain strength) and maximum amplitude (clot's maximum strength) were analyzed and compared among different groups.

### Platelet counts in mouse whole blood

Male and female C57BL/6J mice (n = 6) were intravenously dosed with the following treatments: 1) vehicle control (equivalent volume of saline); 2) ML355 (1.5 mg/kg); 3) sHDL (50 mg/kg); or 4) ML355-sHDL (sHDL at 50 mg/kg and ML355 at 1.5 mg/kg). 24 hours post administration, mice were anesthetized as described above. Blood sample was collected from the saphenous vein. Complete blood counts were performed using a Hemavet 950 analyzer (Drew Scientific Inc., Oxford, CT, USA).

### Statistical Analysis

Unpaired, paired two-tailed student t-tests, and two-way analysis of variance (ANOVA) were used to compare between experimental groups with Prism 6.0 software (GraphPad). Where appropriate, the statistical test used is contained in the figure legend. Data represents mean values ± standard deviation (SD). Differences were considered significant when **P* < 0.05, ***P* < 0.01, and ****P* < 0.001.

### REFERENCES

1. N. Mackman, Triggers, targets and treatments for thrombosis. Nature 451, 914-918 (2008).
2. J. Yeung, W. Li, M. Holinstat, Platelet signaling and disease: targeted therapy for thrombosis and other related diseases. Pharmacol. Rev. 70, 526-548 (2018).
3. R. Abbate, G. Cioni, I. Ricci, M. Miranda, A. M. Gori, Thrombosis and acute coronary syndrome. Thromb. Res. 129, 235-240 (2012).
4. P. D. Dunne, S. B. Laursen, L. Laine, H. R. Dalton, J. H. Ngu, M. Schultz, A. Rahman, A. Anderloni, I. A. Murray, A. J. Stanley, Previous use of antithrombotic agents reduces mortality and length of hospital stay in patients with high-risk upper gastrointestinal bleeding. Clin. Gastroenterol. Hepatol. 17, 440-447. e442 (2019).
5. J. W. Eikelboom, J. Hirsh, F. A. Spencer, T. P. Baglin, J. I. Weitz, Antiplatelet drugs: antithrombotic therapy and prevention of thrombosis: American College of Chest Physicians evidence-based clinical practice guidelines. Chest 141, e89S-e119S (2012).
6. J. L. Mega, T. Simon, Pharmacology of antithrombotic drugs: an assessment of oral antiplatelet and anticoagulant treatments. The Lancet 386, 281-291 (2015).
7. J. I. Weitz, J. W. Eikelboom, M. M. Samama, New antithrombotic drugs: antithrombotic therapy and prevention of thrombosis: American college of chest physicians evidence-based clinical practice guidelines. Chest 141, e120S-e151S (2012).
8. R. D. Watson, B. S. Chin, G. Y. Lip, Antithrombotic therapy in acute coronary syndromes. BMJ 325, 1348-1351 (2002).
9. G. N. Levine, M. N. Ali, A. I. Schafer, Antithrombotic therapy in patients with acute coronary syndromes. Arch. Intern. Med. 161, 937-948 (2001).
10. W. Ageno, A. S. Gallus, A. Wittkowsky, M. Crowther, E. M. Hylek, G. Palareti, Oral anticoagulant therapy: antithrombotic therapy and prevention of thrombosis: American College of Chest Physicians evidence-based clinical practice guidelines. Chest 141, e44S-e88S (2012).
11. E. J. Benjamin, P. Muntner, M. S. Bittencourt, C. W. Callaway, A. P. Carson, A. M. Chamberlain, A. R. Chang, S. Cheng, S. R. Das, F. N. Delling, DjousseLuc , M. S. V. Elkind, J. F. Ferguson, M. Fornage, L. C. Jordan, S. S. Khan, B. M. Kissela, K. L. Knutson, T. W. Kwan, D. T. Lackland, T. T. Lewis, J. H. Lichtman, C. T. Longenecker, M. S. Loop, P. L. Lutsey, S. S. Martin, K. Matsushita, A. E. Moran, M. E. Mussolino, M. O'Flaherty, A. Pandey, A. M. Perak, W. D. Rosamond, G. A. Roth, U. K. A. Sampson, G. M. Satou, E. B. Schroeder, S. H. Shah, N. L. Spartano, A. Stokes, D. L. Tirschwell, C. W. Tsao, M. P. Turakhia, L. B. VanWagner, J. T. Wilkins, S. S. Wong, S. S. Virani, Heart disease and stroke statistics-2019 update: a report from the American Heart Association. Circulation 139, e56-e528 (2019).
12. A. M. Flores, J. Ye, K.-U. Jarr, N. Hosseini-Nassab, B. R. Smith, N. J. Leeper, Nanoparticle therapy for vascular diseases. Arterioscler. Thromb. Vasc. Biol. 39, 635-646 (2019).
13. M. Varna, M. Juenet, R. Bayles, M. Mazighi, C. Chauvierre, D. Letourneur, Nanomedicine as a strategy to fight thrombotic diseases. Future science OA 1, FSO46 (2015).
14. Y.-H. Ma, S.-Y. Wu, T. Wu, Y.-J. Chang, M.-Y. Hua, J.-P. Chen, Magnetically targeted thrombolysis with recombinant tissue plasminogen activator bound to polyacrylic acid-coated nanoparticles. Biomaterials 30, 3343-3351 (2009).
15. J. R. McCarthy, I. Y. Sazonova, S. S. Erdem, T. Hara, B. D. Thompson, P. Patel, I. Botnaru, C. P. Lin, G. L. Reed, R. Weissleder, F. A. Jaffer, Multifunctional nanoagent for thrombus-targeted fibrinolytic therapy. Nanomedicine 7, 1017-1028 (2012).
16. H. Kawata, Y. Uesugi, T. Soeda, Y. Takemoto, J.-H. Sung, K. Umaki, K. Kato, K. Ogiwara, K. Nogami, K. Ishigami, M. Horii, S. Uemura, M. Shima, Y. Tabata, Y. Saito, A new drug delivery system for intravenous coronary thrombolysis with thrombus targeting and stealth activity recoverable by ultrasound. J. Am. Coll. Cardiol. 60, 2550-2557 (2012).
17. N. Korin, M. Kanapathipillai, B. D. Matthews, M. Crescente, A. Brill, T. Mammoto, K. Ghosh, S. Jurek, S. A. Bencherif, D. Bhatta, A. U. Coskun, C. L. Feldman, D. D. Wagner, D. E. Ingber, Shear-activated nanotherapeutics for drug targeting to obstructed blood vessels. Science 337, 738-742 (2012).
18. H.-j. Jin, H. Zhang, M.-I. Sun, B.-g. Zhang, J.-w. Zhang, Urokinase-coated chitosan nanoparticles for thrombolytic therapy: preparation and pharmacodynamics in vivo. J. Thromb. Thrombolysis 36, 458-468 (2013).
19. J. Xu, X. Wang, H. Yin, X. Cao, Q. Hu, W. Lv, Q. Xu, Z. Gu, H. Xin, Sequentially Site-Specific Delivery of Thrombolytics and Neuroprotectant for Enhanced Treatment of Ischemic Stroke. ACS nano 13, 8577-8588 (2019).
20. Z. Wei, G. Xin, H. Wang, H. Zheng, C. Ji, J. Gu, L. Ma, C. Qin, Z. Xing, H. Niu, W. Huang, The diosgenin prodrug nanoparticles with pH-responsive as a drug delivery system uniquely prevents thrombosis without increased bleeding risk. Nanomedicine 14, 673-684 (2018).
21. S. Jin, Y. Wang, H. Zhu, Y. Wang, S. Zhao, M. Zhao, J. Liu, J. Wu, W. Gao, S. Peng, Nanosized aspirin-Arg-Gly-Asp-Val: delivery of aspirin to thrombus by the target carrier Arg-Gly-Asp-Val tetrapeptide. ACS nano 7, 7664-7673 (2013).
22. Y. Chen, G. Cui, M. Zhao, C. Wang, K. Qian, S. Morris-Natschke, K.-H. Lee, S. Peng, Synthesis, nano-scale assembly, and in vivo anti-thrombotic activity of novel short peptides containing L-Arg and L-Asp or L-Glu. Bioorg. Med. Chem. 16, 5914-5925 (2008).
23. R. U. Palekar, A. P. Jallouk, J. W. Myerson, H. Pan, S. A. Wickline, Inhibition of thrombin with PPACK-nanoparticles restores disrupted endothelial barriers and attenuates thrombotic risk in experimental atherosclerosis. Arterioscler. Thromb. Vasc. Biol. 36, 446-455 (2016).
24. A. C. Tang, M.-Y. Chang, Z. C. Tang, H.-J. Li, G.-L. Hwang, P. C. Hsieh, Treatment of acute thromboembolism in mice using heparin-conjugated carbon nanocapsules. ACS nano 6, 6099-6107 (2012).
25. J. Zhou, D. Guo, Y. Zhang, W. Wu, H. Ran, Z. Wang, Construction and evaluation of Fe3O4-based PLGA nanoparticles carrying rtPA used in the detection of thrombosis and in targeted thrombolysis. ACS Appl. Mater. Interfaces. 6, 5566-5576 (2014).
26. F. Bi, J. Zhang, Y. Su, Y.-C. Tang, J.-N. Liu, Chemical conjugation of urokinase to magnetic nanoparticles for targeted thrombolysis. Biomaterials 30, 5125-5130 (2009).
27. H. He, L. Liu, E. E. Morin, M. Liu, A. Schwendeman, Survey of Clinical Translation of Cancer Nanomedicines-Lessons Learned from Successes and Failures. Acc. Chem. Res. 52, 2445-2461 (2019).
28. R. Kuai, D. Li, Y. E. Chen, J. J. Moon, A. Schwendeman, High-density lipoproteins: nature's multifunctional nanoparticles. ACS nano 10, 3015-3041 (2016).
29. Y. Guo, W. Yuan, B. Yu, R. Kuai, W. Hu, E. E. Morin, M. T. Garcia-Barrio, J. Zhang, J. J. Moon, A. Schwendeman, Y. E. Chen, Synthetic high-density lipoprotein-mediated targeted delivery of liver X receptors agonist promotes atherosclerosis regression. EBioMedicine 28, 225-233 (2018).
30. Q. Song, M. Huang, L. Yao, X. Wang, X. Gu, J. Chen, J. Chen, J. Huang, Q. Hu, T. Kang, Z. Rong, H. Qi, G. Zheng, H. Chen, X. Gao, Lipoprotein-based nanoparticles rescue the memory loss of mice with Alzheimer's disease by accelerating the clearance of amyloid-beta. ACS nano 8, 2345-2359 (2014).
31. D. J. Rader, Apolipoprotein Al infusion therapies for coronary disease: two outs in the ninth inning and swinging for the fences. JAMA Cardiol. 3, 799-801 (2018).
32. P. Barter, The role of HDL-cholesterol in preventing atherosclerotic disease. Eur. Heart J. 7, F4-F8 (2005).
33. D. W. Chung, J. Chen, M. Ling, X. Fu, T. Blevins, S. Parsons, J. Le, J. Harris, T. R. Martin, B. A. Konkle, Y. Zheng, J. A. López, High-density lipoprotein modulates thrombosis by preventing von Willebrand factor self-association and subsequent platelet adhesion. Blood 127, 637-645 (2016).
34. A. J. Murphy, N. Bijl, L. Yvan-Charvet, C. B. Welch, N. Bhagwat, A. Reheman, Y. Wang, J. A. Shaw, R. L. Levine, H. Ni, A. R. Tall, N. Wang, Cholesterol efflux in megakaryocyte progenitors suppresses platelet production and thrombocytosis. Nat. Med. 19, 586-594 (2013).
35. D. Li, S. Weng, B. Yang, D. S. Zander, T. Saldeen, W. W. Nichols, S. Khan, J. L. Mehta, Inhibition of arterial thrombus formation by ApoA1 Milano. Arterioscler. Thromb. Vasc. Biol. 19, 378-383 (1999).
36. P. G. Lerch, M. O. Spycher, J. E. Doran, Reconstituted high density lipoprotein (rHDL) modulates platelet activity in vitro and ex vivo. Thromb. Haemost. 80, 316-320 (1998).
37. D. Pajkrt, P. G. Lerch, T. van der Poll, M. Levi, M. Illi, J. E. Doran, B. Arnet, A. van den Ende, J. W. ten Cate, S. J. van Deventer, Differential effects of reconstituted high-density lipoprotein on coagulation, fibrinolysis and platelet activation during human endotoxemia. Thromb. Haemost. 77, 303-307 (1997).
38. D. Li, S. Weng, B. Yang, D. S. Zander, T. Saldeen, W. W. Nichols, S. Khan, J. L. Mehta, Inhibition of arterial thrombus formation by ApoA1 Milano. Arterioscler. Thromb. Vasc. Biol. 19, 378-383 (1999).
39. J.-R. Nofer, M. Walter, B. Kehrel, S. Wierwille, M. Tepel, U. Seedorf, G. Assmann, HDL3-mediated inhibition of thrombin-induced platelet aggregation and fibrinogen binding occurs via decreased production of phosphoinositide-derived second messengers 1, 2-diacylglycerol and inositol 1, 4, 5-tris-phosphate. Arterioscler. Thromb. Vasc. Biol. 18, 861-869 (1998).
40. A. C. Calkin, B. G. Drew, A. Ono, S. J. Duffy, M. V. Gordon, S. M. Schoenwaelder, D. Sviridov, M. E. Cooper, B. A. Kingwell, S. P. Jackson, Reconstituted high-density lipoprotein attenuates platelet function in individuals with type 2 diabetes mellitus by promoting cholesterol efflux. Circulation 120, 2095-2104 (2009).
41. K. Ma, A. Xiao, S. H. Park, L. Glenn, L. Jackson, T. Barot, J. R. Weaver, D. A. Taylor-Fishwick, D. K. Luci, D. J. Maloney, R. G. Mirmira, Y. Imai, J. L. Nadler, 12-lipoxygenase inhibitor improves functions of cytokine-treated human islets and type 2 diabetic islets. J. Clin. Endocrinol. Metab. 102, 2789-2797 (2017).
42. X.-J. Zhang, X. Cheng, Z.-Z. Yan, J. Fang, X. Wang, W. Wang, Z.-Y. Liu, L.-J. Shen, P. Zhang, P.-X. Wang, R. Liao, Y.-X. Ji, J.-Y. Wang, S. Tian, X.-Y. Zhu, Y. Zhang, R.-F. Tian, L. Wang, X.-L. Ma, Z. Huang, Z.-G. She, H. Li, An ALOX12-12-HETE-GPR31 signaling axis is a key mediator of hepatic ischemia-reperfusion injury. Nat. Med. 24, 73-83 (2018).
43. D. K. Luci, J. B. Jameson, A. Yasgar, G. Diaz, N. Joshi, A. Kantz, K. Markham, S. Perry, N. Kuhn, J. Yeung, E. H. Kerns, L. Schultz, M. Holinstat, J. L. Nadler, D. A. Taylor-Fishwick, A. Jadhav, A. Simeonov, T. R. Holman, D. J. Maloney, Synthesis and structure-activity relationship studies of 4-((2-hydroxy-3-methoxybenzyl) amino) benzenesulfonamide derivatives as potent and selective inhibitors of 12-lipoxygenase. J. Med. Chem. 57, 495-506 (2014).
44. R. Adili, B. E. Tourdot, K. Mast, J. Yeung, J. C. Freedman, A. Green, D. K. Luci, A. Jadhav, A. Simeonov, D. J. Maloney, T. R. Holman, M. Holinstat, First selective 12-LOX inhibitor, ML355, impairs thrombus formation and vessel occlusion in vivo with minimal effects on hemostasis. Arterioscler. Thromb. Vasc. Biol. 37, 1828-1839 (2017).
45. J. Yeung, B. E. Tourdot, P. Fernandez-Perez, J. Vesci, J. Ren, C. J. Smyrniotis, D. K. Luci, A. Jadhav, A. Simeonov, D. J. Maloney, T. R. Holman, S. E. McKenzie, M. Holinstat, Platelet 12-LOX is essential for FcγRIIa-mediated platelet activation. Blood 124, 2271-2279 (2014).
46. P. Kadiyala, D. Li, F. M. Nuñez, D. Altshuler, R. Doherty, R. Kuai, M. Yu, N. Kamran, M. Edwards, J. J. Moon, P. R. Lowenstein, M. G. Castro, A. Schwendeman, High-Density Lipoprotein-Mimicking Nanodiscs for Chemo-immunotherapy against Glioblastoma Multiforme. ACS nano 13, 1365-1384 (2019).
47. R. Kuai, L. J. Ochyl, K. S. Bahjat, A. Schwendeman, J. J. Moon, Designer vaccine nanodiscs for personalized cancer immunotherapy. Nat. Mater. 16, 489-496 (2017).
48. L. Badimon, G. Vilahur, LDL-cholesterol versus HDL-cholesterol in the atherosclerotic plaque: inflammatory resolution versus thrombotic chaos. Ann. N. Y. Acad. Sci. 1254, 18-32 (2012).
49. M. Ruiz, C. Frej, A. Holmér, L. J. Guo, S. Tran, B. Dahlbäck, High-density lipoprotein-associated apolipoprotein M limits endothelial inflammation by delivering sphingosine-1-phosphate to the sphingosine-1-phosphate receptor 1. Arterioscler. Thromb. Vasc. Biol. 37, 118-129 (2017).
50. S. Badrnya, A. Assinger, I. Volf, Native high density lipoproteins (HDL) interfere with platelet activation induced by oxidized low density lipoproteins (OxLDL). Int. J. Mol. Sci. 14, 10107-10121 (2013).
51. P. E. van der Meijden, J. W. Heemskerk, Platelet biology and functions: new concepts and clinical perspectives. Nat Rev Cardiol. 16, 166-179 (2019).
52. Y.-C. Wang, X.-Q. Liu, T.-M. Sun, M.-H. Xiong, J. Wang, Functionalized micelles from block copolymer of polyphosphoester and poly (ε-caprolactone) for receptor-mediated drug delivery. J. Control. Release 128, 32-40 (2008).
53. Z. Meng, L. Meng, K. Wang, J. Li, X. Cao, J. Wu, Y. Hu, Enhanced hepatic targeting, biodistribution and antifibrotic efficacy of tanshinone IIA loaded globin nanoparticles. Eur. J. Pharm. Sci. 73, 35-43 (2015).
54. S. Novakowski, K. Jiang, G. Prakash, C. Kastrup, Delivery of mRNA to platelets using lipid nanoparticles. Sci. Rep. 9, 552 (2019).
55. Z. Liu, N. Zhang, B. Shao, S. R. Panicker, J. Fu, R. P. McEver, Replacing the promoter of the murine gene encoding P-selectin with the human promoter confers human-like basal and inducible expression in mice. J. Biol. Chem. 291, 1441-1447 (2016).
56. R. Adili, E. M. Voigt, J. L. Bormann, K. N. Foss, L. J. Hurley, E. S. Meyer, A. J. Veldman, K. A. Mast, J. L. West, S. W. Whiteheart, M. Holinstat, M. k. Larson, In vivo modeling of docosahexaenoic acid and eicosapentaenoic acid-mediated inhibition of both platelet function and accumulation in arterial thrombi. Platelets 30, 271-279 (2019).

The foregoing embodiments are to be considered in all respects illustrative rather than limiting the invention described herein. Scope of the invention is thus indicated by the appended claims rather than by the foregoing description.

## Claims

1. A composition comprising one or more synthetic HDL nanoparticle (sHDL) associated with (e.g., complexed, conjugated, encapsulated, absorbed, adsorbed, admixed) N-2-benzothiazolyl-4-[[(2-hydroxy-3-methoxyphenyl)methyl]amino]-benzenesulfonamide (ML355) (sHDL-ML355) moieties, wherein the sHDL-ML355 comprises a mixture of at least one lipid component, at least one HDL apolipoprotein component, and ML355,
wherein the HDL apolipoprotein component is selected from the group consisting of apolipoprotein A-I (apo A-I), apolipoprotein A-II (apo A-II), apolipoprotein A-II xxx (apo A-II-xxx), apolipoprotein A4 (apo A4), apolipoprotein Cs (apo Cs), apolipoprotein E (apo E), apolipoprotein A-I milano (apo A-I-milano), apolipoprotein A-I paris (apo A-I-paris), apolipoprotein M (apo M), an HDL apolipoprotein mimetic, preproapoliprotein, preproApoA-I, proApoA I, preproApoA-II, proApoA II, preproApoA-IV, proApoA-IV, ApoA-V, preproApoE, proApoE, preproApoA I_{Milano}, proApoA-I_{Milano}, preproApoA-I_{Paris}, proApoA-I_{Paris}, and mixtures thereof, and
wherein the ApoA-I mimetic is described by any of SEQ ID NOs: 1-336 and WDRVKDLATVYVDVLKDSGRDYVSQF (SEQ ID NO: 337), LKLLDNWDSVTSTFSKLREOL (SEQ ID NO: 338), PVTOEFWDNLEKETEGLROEMS (SEQ ID NO: 339), KDLEEVKAKVQ (SEQ ID NO: 340), KDLEEVKAKVO (SEQ ID NO: 341), PYLDDFQKKWQEEMELYRQKVE (SEQ ID NO: 342), PLRAELQEGARQKLHELOEKLS (SEQ ID NO: 343), PLGEEMRDRARAHVDALRTHLA (SEQ ID NO: 344), PYSDELRQRLAARLEALKENGG (SEQ ID NO: 345), ARLAEYHAKATEHLSTLSEKAK (SEQ ID NO: 346), PALEDLROGLL (SEQ ID NO: 347), PVLESFKVSFLSALEEYTKKLN (SEQ ID NO: 348), PVLESFVSFLSALEEYTKKLN (SEQ ID NO: 349), PVLESFKVSFLSALEEYTKKLN (SEQ ID NO: 350), TVLLLTICSLEGALVRRQAKEPCV QTVTDYGKDLME (SEQ ID NO: 351), KVKSPELOAEAKSYFEKSKE (SEQ ID NO: 352), VLTLALVAVAGARAEVSADOVATV (SEQ ID NO: 353), NNAKEAVEHLOKSELTOOLNAL (SEQ ID NO: 354), LPVLVWLSIVLEGPAPAOGTPDVSS (SEQ ID NO: 355), LPVLVVVLSIVLEGPAPAQGTPDVSS (SEQ ID NO: 356), ALDKLKEFGNTLEDKARELIS (SEQ ID NO: 357), VVALLALLASARASEAEDASLL (SEQ ID NO: 358), HLRKLRKRLLRDADDLQKRLAVYOA (SEQ ID NO: 359), AQAWGERLRARMEEMGSRTRDR (SEQ ID NO: 360), LDEVKEQVAEVRAKLEEQAQ (SEQ ID NO: 361), DWLKAFYDKVAEKLKEAF (SEQ ID NO: 362), DWLKAFYDKVAEKLKEAFPDWAKAAYDKAAEKAKEAA (SEQ ID NO: 363), PVLDLFRELLNELLEALKQKL (SEQ ID NO: 364), PVLDLFRELLNELLEALKQKLA (SEQ ID NO: 365), PVLDLFRELLNELLEALKQKLK (SEQ ID NO: 366), PVLDLFRELLNELLEALKQKLA (SEQ ID NO: 367), PVLDLFRELLNELLEALKKLLK (SEQ ID NO: 368), PVLDLFRELLNELLEALKKLLA (SEQ ID NO: 369), and PLLDLFRELLNELLEALKKLLA (SEQ ID NO: 370)..

2. . The composition of Claim 1, wherein the sHDL-ML355 is configured for sustained release of the ML355 over a 24 hour period.

3. . The composition of Claim 1, wherein upon administration to a subject (e.g., a human subject) the sHDL-ML355 is capable of inhibiting platelet aggregation, inhibiting thrombosis formation, inhibiting vessel occlusion, and/or inhibiting platelet associated 12-LOX activity.

4. . The composition of Claim 1, wherein the molar ratio of the HDL apolipoprotein component to the lipid component is about 2:1 to 200:1, or wherein the lipid component comprises neutral phospholipids, negatively charged phospholipids, positively charged phospholipids, or a combination thereof.

5. . The composition of Claim 1, wherein the lipid component comprises a combination of one or any combination of sphingomyelin (SM), D-erythrose-sphingomyelin, D-erythrose dihydrosphingomyelin, palmitoylsphingomyelin, lysophospholipids, galactocerebroside, gangliosides, cerebrosides, glycerides, triglycerides, diglycerides, small alkyl chain phospholipids, phosphatidylcholine, egg phosphatidylcholine, soybean phosphatidylcholine, dipalmitoylphosphatidylcholine (DPPC), dimyristoylphosphatidylcholine, 1-palmitoyl-2-oleoylphosphatidylcholine (POPC), 1,2-dimyristoyl-sn-glycero-3-phosphatidylcholine (DMPC), 1,2-distearoyl-sn-glycero-3-phosphatidylcholine (DSPC), distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, dioleoylphosphatidylcholine dioleophosphatidylethanolamine, dilauroylphosphatidylglycerol phosphatidylcholine, phosphatidylserine, phosphatidylethanolamine, phosphatidylinositol, phosphatidylglycerols, diphosphatidylglycerols such as dimyristoylphosphatidylglycerol, dipalmitoylphosphatidylglycerol, distearoylphosphatidylglycerol, dioleoylphosphatidylglycerol, dimyristoylphosphatidic acid, dipalmitoylphosphatidic acid, dimyristoylphosphatidylethanolamine, dipalmitoylphosphatidylethanolamine, ceramides, a phosphatidylserine, dimyristoylphosphatidylserine, dipalmitoylphosphatidylserine, brain phosphatidylserine, brain sphingomyelin, egg sphingomyelin, milk sphingomyelin, palmitoyl sphingomyelin, phytosphingomyelin, dipalmitoylsphingomyelin, distearoylsphingomyelin, dipalmitoylphosphatidylglycerol salt, phosphatidic acid, galactocerebroside, gangliosides, cerebrosides, dilaurylphosphatidylcholine, (1,3)-D-mannosyl-(1,3)diglyceride, aminophenylglycoside, 3-cholesteryl-6'-(glycosylthio)hexyl ether glycolipids, and cholesterol and its derivatives, lyso-phosphotydyl choline, lyso-sphingomyelin, dioleoyl-sn-glycero-3-phosphoethanolamine-N-[3-(2-pyridyldithio) propionate] (DOPE-PDP), 1,2-dipalmitoyl-*sn-*glycero-3-phosphothioethanol, 1,2-di-(9Z-octadecenoyl)-*sn*-glycero-3-phosphoethanolamine-N-[4-(p-maleimidophenyl)butyramide], 1,2-dihexadecanoyl-*sn*-glycero-3-phosphoethanolamine-N-[4-(p-maleimidophenyl)butyramide], 1,2-dihexadecanoyl-*sn-*glycero-3-phosphoethanolamine-N-[4-(p-maleimidomethyl)cyclohexane-carboxamide], 1,2-di-(9Z-octadecenoyl)-*sn*-glycero-3-phosphoethanolamine-N-[4-(p-maleimidomethyl)cyclohexane-carboxamide], Lyso phoshphatidic acid, Lyso phosphatidylcholine, OA-NO₂ (nitrated oleic acid 9- and 10- nitro-*cis*-octedecenolic acids), LNO₂ (nitrated linoleic Acid 9-, 10-, 12-and 13-nitro-*cis*-octedecadienoic acids), AA-NO₂ (nitrated Arachidonic Acid 5-, 6-, 8-, 9-, 11-, 12-, 14,-and 15-nitro-cis-eicosatetraenoic acids), CLNO₂ (nitrated cholesteryl linoleate cholestaryl-9-, 10-, 12- and 13-nitro-*cis-*octedecadiencates), fatty acid, omega-3 polyunsaturated fatty acids, hexadecatrienoic acid (HTA; 16:3 (n-3); all-cis-7,10,13-hexadecatrienoic acid), α-Linolenic acid (ALA; 18:3 (n-3); all-cis-9,12,15-octadecatrienoic acid), stearidonic acid (SDA; 18:4 (n-3); all-cis-6,9,12,15-octadecatetraenoic acid), eicosatrienoic acid (ETE; 20:3 (n-3); all-cis-11,14,17-eicosatrienoic acid), eicosatetraenoic acid (ETA; 20:4 (n-3); all-cis-8,11,14,17-eicosatetraenoic acid), eicosapentaenoic acid (EPA; 20:5 (n-3); all-cis-5,8,11,14,17-eicosapentaenoic acid), heneicosapentaenoic acid (HPA; 21:5 (n-3); all-cis-6,9,12,15,18-heneicosapentaenoic acid); docosapentaenoic acid (DPA; clupanodonic acid; 22:5 (n-3); all-cis-7,10,13,16,19-docosapentaenoic acid), docosahexaenoic acid (DHA; 22:6 (n-3); all-cis-4,7,10,13,16,19-docosahexaenoic acid), tetracosapentaenoic acid; 24:5 (n-3); all-cis-9,12,15,18,21-tetracosapentaenoic acid), tetracosahexaenoic acid (Nisinic acid; 24:6 (n-3), all-cis-6,9,12,15,18,21-tetracosahexaenoic acid), sphingosine-1-phosphate analogs, sphingosine-1-phosphate antagonists, sphingosine-1-phosphate agonists, sphingosine-1-phosphate receptor agonists, sphingosine-1-phosphate receptor antagonists, and sphingosine-1-phosphate receptor analogs.

6. . A composition according to any of claims 1 to 5 for use in a method of preventing, attenuating or treating thrombosis in a subject (e.g., a human subject) having or at risk for having conditions and symptoms caused by thrombosis, comprising administering to the subject such a composition.

7. . The composition for use of Claim 6, wherein administration of the composition results in, for example, reduction of platelet activity, reduction of platelet aggregation, prevention of thrombus formation, reduction of vessel occlusion, and reduction of platelet associated 12-LOX activity.

8. . The composition for use of Claim 6, wherein the conditions and symptoms caused by thrombosis are related to a venous thrombosis and/or an arterial thrombosis.

9. . The composition for use of Claim 6, wherein the thrombosis is related to one or more of the following conditions: acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, thromboembolic stroke, systemic embolism, ischemic stroke, venous thromboembolism, atrial fibrillation, non-valvular atrial fibrillation, atrial flutter, transient ischemic attacks, venous thrombosis, deep venous thrombosis, pulmonary embolus, coagulopathy, disseminated intravascular coagulation, thrombotic thrombocytopenic purpura, thromboanglitis obliterans, thrombotic disease associated with heparin-induced thrombocytopenia, thrombotic complications associated with extracorporeal circulation, thrombotic complications associated with instrumentation, thrombotic complications associated with the fitting of prosthetic devices, occlusive coronary thrombus formation resulting from either thrombolytic therapy or percutaneous transluminal coronary angioplasty, thrombus formation in the venous vasculature, disseminated intravascular coagulopathy, a condition wherein there is rapid consumption of coagulation factors and systemic coagulation which results in the formation of life-threatening thrombi occurring throughout the microvasculature leading to widespread organ failure, hemorrhagic stroke, renal dialysis, blood oxygenation, and cardiac catheterization.

10. . The composition for use of Claim 6, wherein the conditions and symptoms caused by thrombosis are selected from the group consisting of embolic stroke, thrombotic stroke, venous thrombosis, deep venous thrombosis, acute coronary syndrome, and myocardial infarction.

11. . The composition for use of Claim 6, wherein the composition is co-administered with one or more of the following therapeutic agents: heparin; tPA; anistreplase; streptokinase; urokinase; a coumadin; warfarin; idraparinux; fondaparinux; aspririn; an adenosine diphosphate receptor inhibitor; a phosphodiesterase inhibitor; a glycoprotein IIB/IIA inhibitor; an adenosine reuptake inhibitor; and a thromboxane receptor antagonist.

12. . A composition according to any of claims 1 to 5 for use in a method of preventing, attenuating or treating a subject (e.g., a human subject) having a cardiovascular related disorder, comprising administering to the subject a therapeutically effective amount of such a composition.

13. . A composition according to any of claims 1 to 5 for use in a method of preventing, attenuating or treating increased platelet activity in a subject (e.g., a human subject) having or at risk for having increased platelet activity, or of preventing, attenuating or treating platelet aggregation in a subject (e.g., a human subject) having or at risk for having platelet aggregation, or of preventing, attenuating or treating vessel occlusion (e.g., arterial and/or venous) in a subject (e.g., a human subject) having or at risk for having vessel occlusion, or of preventing, attenuating or treating increased platelet associated 12-LOX activity in a subject (e.g., a human subject) having or at risk for having increased platelet associated 12-LOX activity, comprising administering to the subject such a composition.

## Patentansprüche

1. Zusammensetzung, umfassend ein oder mehrere Einheiten synthetischer HDL-Nanopartikel (sHDL) assoziiert mit (z. B. komplexiert, konjugiert, verkapselt, absorbiert, adsorbiert, vermischt) N-2-Benzothiazolyl-4-[[(2-hydroxy-3-methoxyphenyl)methyl]amino]benzolsulfonamid (ML355) (sHDL-ML355), wobei das sHDL-ML355 eine Mischung aus zumindest einer Lipidkomponente, zumindest einer HDL-Apolipoprotein-Komponente und ML355 umfasst,
wobei die HDL-Apolipoprotein-Komponente ausgewählt ist aus der Gruppe bestehend aus Apolipoprotein A-I (Apo A-I), Apolipoprotein A-II (Apo A-II), Apolipoprotein A-II xxx (Apo A-II-xxx), Apolipoprotein A4 (Apo A4), Apolipoprotein Cs (Apo Cs), Apolipoprotein E (Apo E), Apolipoprotein A-I Milano (Apo A-I-Milano), Apolipoprotein A-I Paris (Apo A-I-Paris), Apolipoprotein M (Apo M), einem HDL-Apolipoprotein-Mimetikum, Preproapoliprotein, PreproApoA-I, ProApoA I, PreproApoA-II, ProApoA II, PreproApoA-IV, ProApoA-IV, ApoA-V, PreproApoE, ProApoE, PreproApoA I_{Milano}, ProApoA-I_{Milano}, PreproApoA-I_{Paris}, ProApoA-I_{Paris} und Mischungen davon, und
wobei das ApoA-I-Mimetikum beschrieben ist durch beliebige von SEQ ID NOs: 1-336 und WDRVKDLATVYVDVLKDSGRDYVSQF (SEQ ID NO: 337), LKLLDNWDSVTSTFSKLREOL (SEQ ID NO: 338), PVTOEFWDNLEKETEGLROEMS (SEQ ID NO: 339), KDLEEVKAKVQ (SEQ ID NO: 340), KDLEEVKAKVO (SEQ ID NO: 341), PYLDDFQKKWQEEMELYRQKVE (SEQ ID NO: 342), PLRAELQEGARQKLHELOEKLS (SEQ ID NO: 343), PLGEEMRDRARAHVDALRTHLA (SEQ ID NO: 344), PYSDELRQRLAARLEALKENGG (SEQ ID NO: 345), ARLAEYHAKATEHLSTLSEKAK (SEQ ID NO: 346), PALEDLROGLL (SEQ ID NO: 347), PVLESFKVSFLSALEEYTKKLN (SEQ ID NO: 348), PVLESFVSFLSALEEYTKKLN (SEQ ID NO: 349), PVLESFKVSFLSALEEYTKKLN (SEQ ID NO: 350), TVLLLTICSLEGALVRRQAKEPCV QTVTDYGKDLME (SEQ ID NO: 351), KVKSPELOAEAKSYFEKSKE (SEQ ID NO: 352), VLTLALVAVAGARAEVSADOVATV (SEQ ID NO: 353), NNAKEAVEHLOKSELTOOLNAL (SEQ ID NO: 354), LPVLVWLSIVLEGPAPAOGTPDVSS (SEQ ID NO: 355), LPVLVVVLSIVLEGPAPAQGTPDVSS (SEQ ID NO: 356), ALDKLKEFGNTLEDKARELIS (SEQ ID NO: 357), VVALLALLASARASEAEDASLL (SEQ ID NO: 358), HLRKLRKRLLRDADDLQKRLAVYOA (SEQ ID NO: 359), AQAWGERLRARMEEMGSRTRDR (SEQ ID NO: 360), LDEVKEQVAEVRAKLEEQAQ (SEQ ID NO: 361), DWLKAFYDKVAEKLKEAF (SEQ ID NO: 362), DWLKAFYDKVAEKLKEAFPDWAKAAYDKAAEKAKEAA SEQ ID NO: 363), PVLDLFRELLNELLEALKQKL (SEQ ID NO: 364), PVLDLFRELLNELLEALKQKLA (SEQ ID NO: 365), PVLDLFRELLNELLEALKQKLK (SEQ ID NO: 366), PVLDLFRELLNELLEALKQKLA (SEQ ID NO: 367), PVLDLFRELLNELLEALKKLLK (SEQ ID NO: 368), PVLDLFRELLNELLEALKKLLA (SEQ ID NO: 369) und PLLDLFRELLNELLEALKKLLA (SEQ ID NO: 370).

2. Zusammensetzung nach Anspruch 1, wobei das sHDL-ML355 für verzögerte Freisetzung des ML355 über einen Zeitraum von 24 Stunden konfiguriert ist.

3. Zusammensetzung nach Anspruch 1, wobei bei Verabreichung an ein Subjekt (z. B. ein menschliches Subjekt) das sHDL-ML355 in der Lage ist, Thrombozytenaggregation zu hemmen, Thrombosebildung zu hemmen, Gefäßverschluss zu hemmen und/oder Thrombozyten-assoziierte 12-LOX-Aktivität zu hemmen.

4. Zusammensetzung nach Anspruch 1, wobei das Molverhältnis der HDL-Apolipoprotein-Komponente zu der Lipidkomponente etwa 2:1 bis 200:1 ist, oder wobei die Lipidkomponente neutrale Phospholipide, negativ geladene Phospholipide, positiv geladene Phospholipide oder eine Kombination davon umfasst.

5. Zusammensetzung nach Anspruch 1, wobei die Lipidkomponente eine Kombination aus einem oder eine beliebige Kombination aus Sphingomyelin (SM), D-Erythrose-sphingomyelin, D-Erythrose-dihydrosphingomyelin, Palmitoylsphingomyelin, Lysophospholipiden, Galactocerebrosid, Gangliosiden, Cerebrosiden, Glyceriden, Triglyceriden, Diglyceriden, kleinen Alkylkettenphospholipiden, Phosphatidylcholin, Eiphosphatidylcholin, Sojabohnenphosphatidylcholin, Dipalmitoylphosphatidylcholin (DPPC), Dimyristoylphosphatidylcholin, 1-Palmitoyl-2-oleoyl-phosphatidylcholin (POPC), 1,2-Dimyristoyl-sn-glycero-3-phosphatidylcholin (DMPC), 1,2-Distearoyl-sn-glycero-3-phosphatidylcholin (DSPC), Distearoylphosphatidylcholin-1-myristoyl-2-palmitoylphosphatidylcholin, 1-Palmitoyl-2-myristoylphosphatidylcholin, 1-Palmitoyl-2-stearoylphosphatidylcholin, 1-Stearoyl-2-palmitoylphosphatidylcholin, Dioleoylphosphatidylcholin-dioleophosphatidylethanolamin, Dilauroylphosphatidylglycerinphosphatidylcholin, Phosphatidylserin, Phosphatidylethanolamin, Phosphatidylinositol, Phosphatidylglycerinen, Diphosphatidylglycerinen wie Dimyristoylphosphatidylglycerin, Dipalmitoylphosphatidylglycerin, Distearoylphosphatidylglycerin, Dioleoylphosphatidylglycerin, Dimyristoylphosphatidinsäure, Dipalmitoylphosphatidinsäure, Dimyristoylphosphatidylethanolamin, Dipalmitoylphosphatidylethanolamin, Ceramiden, einem Phosphatidylserin, Dimyristoylphosphatidylserin, Dipalmitoylphosphatidylserin, Gehirnphosphatidylserin, Gehirnsphingomyelin, Eisphingomyelin, Milchsphingomyelin, Palmitoylsphingomyelin, Phytosphingomyelin, Dipalmitoylsphingomyelin, Distearoylsphingomyelin, Dipalmitoylphosphatidylglycerinsalz, Phosphatidinsäure, Galactocerebrosid, Gangliosiden, Cerebrosiden, Dilaurylphosphatidylcholin, (1,3)-D-Mannosyl-(1,3)diglycerid, Aminophenylglycosid, 3-Cholesteryl-6'-(glycosylthio)hexyletherglycolipiden und Cholesterin und seinen Derivaten, Lyso-Phosphotydylcholin, Lyso-Sphingomyelin, Dioleoyl-sn-glycero-3-phosphoethanolamin-N-[3-(2-pyridyldithio)propionat] (DOPE-PDP), 1,2-Dipalmitoyl-*sn*-glycero-3-phosphothioethanol, 1,2-di-(9Z-Octadecenoyl)-*sn*-glycero-3-phosphoethanolamin-N-[4-(p-maleimidophenyl)butyramid], 1,2-Dihexadecanoyl-*sn*-glycero-3-phosphoethanolamin-N-[4-(p-maleimidophenyl)butyramid], 1,2-Dihexadecanoyl-*sn*-glycero-3-phosphoethanolamin-N-[4-(p-maleimidomethyl)cyclohexan-carboxamid], 1,2-di-(9Z-Octadecenoyl)-*sn*-glycero-3-phosphoethanolamin-N-[4-(p-maleimidomethyl)cyclohexan-carboxamid], Lysophosphatidsäure, Lysophosphatidylcholin, OA-NO₂ (nitrierte Ölsäure-9- und 10-nitro-*cis*-Octedecenolsäuren), LNO₂ (nitrierte Linolsäure-9-, 10-, 12- und 13-nitro-*cis*octedecadienoinsäuren), AA-NO₂ (nitrierte Arachidoninsäure-5-, 6-, 8-, 9-, 11-, 12-, 14- und 15-nitro-cis-Eicosatetraenoinsäuren), CLNO₂ (nitrierte Cholesteryllinoleatcholestaryl-9-, 10-, 12- und 13-nitro-*cis*-Octedecadiencate), Fettsäure, polyungesättigten Omega-3- Fettsäuren, Hexadecatrienoinsäure (HTA; 16:3 (n-3); all-cis-7,10,13-Hexadecatrienoinsäure), α-Linolensäure (ALA; 18:3 (n-3); all-cis-9,12,15-Octadecatriensäure), Stearidonsäure (SDA; 18:4 (n-3); all-cis-6,9,12,15-Octadecatetraensäure), Eicosatrienoinsäure (ETE; 20:3 (n-3); all-cis-11,14,17-Eicosatrienoinsäure), Eicosatetraenoinsäure (ETA; 20:4 (n-3); all-cis-8,11,14,17-Eicosatetraenoinsäure), Eicosapentaenoinsäure (EPA; 20:5 (n-3); all-cis-5,8,11,14,17-Eicosapentaenoinsäure), Heneicosapentaenoinsäure (HPA; 21:5 (n-3); all-cis-6,9,12,15,18-Heneicosapentaenoinsäure); Docosapentaenoinsäure (DPA; Clupanodonsäure; 22:5 (n-3); all-cis-7,10,13,16,19-Docosapentaenoinsäure), Docosahexaenoinsäure (DHA; 22:6 (n-3); all-cis-4,7,10,13,16,19-Docosahexaenoinsäure), Tetracosapentaenoinsäure; 24:5 (n-3); all-cis-9,12,15,18,21-Tetracosapentaenoinsäure), Tetracosahexaenoinsäure (Nisininsäure; 24:6 (n-3), all-cis-6,9,12,15,18,21-Tetracosahexaenoinsäure), Sphingosin-1-phosphat-Analoga, Sphingosin-1-Phosphat-Antagonisten, Sphingosin-1-phosphat-Agonisten, Sphingosin-1-phosphat-Rezeptor-Agonisten, Sphingosin-1-phosphat-Rezeptor-Antagonisten und Sphingosin-1-phosphat-Rezeptor-Analoga umfasst.

6. Zusammensetzung nach einem der Ansprüche 1 bis 5 zur Verwendung in einem Verfahren zum Verhindern, Abschwächen oder Behandeln von Thrombose bei einem Subjekt (z. B. einem menschlichen Subjekt) mit Zuständen und Symptomen, die durch Thrombose verursacht werden, oder bei dem das Risiko besteht, solche Zustände und Symptome aufzuweisen, umfassend Verabreichen einer solchen Zusammensetzung an das Subjekt.

7. Zusammensetzung zur Verwendung nach Anspruch 6, wobei Verabreichung der Zusammensetzung zum Beispiel in Reduzierung von Thrombozytenaktivität, Reduzierung von Thrombozytenaggregation, Verhinderung von Thrombusbildung, Reduzierung von Gefäßverschluss und Reduzierung von Thrombozyten-assoziierter 12-LOX-Aktivität resultiert.

8. Zusammensetzung zur Verwendung nach Anspruch 6, wobei die Zustände und Symptome, die durch Thrombose verursacht werden, mit einer venösen Thrombose und/oder einer arteriellen Thrombose zusammenhängen.

9. Zusammensetzung zur Verwendung nach Anspruch 6, wobei die Thrombose mit einem oder mehreren der folgenden Zustände zusammenhängt: akutem Koronarsyndrom, Myokardinfarkt, instabiler Angina, refraktärer Angina, okklusivem koronaren Thrombus, der nach thrombolytischer Therapie oder nach koronarer Angioplastie auftritt, einem thrombotisch vermittelten zerebrovaskulären Syndrom, embolischem Schlaganfall, thrombotischem Schlaganfall, thromboembolischem Schlaganfall, systemischem Embolismus, ischämischem Schlaganfall, venösem Thromboembolismus, Vorhofflimmern, nicht valvulärem Vorhofflimmern, Vorhofflattern, transienten ischämischen Attacken, venöser Thrombose, tiefer venöser Thrombose, pulmonalem Embolus, Koagulopathie, disseminierter intravaskulärer Koagulation, thrombotischer thrombozytopenischer Purpura, Thromboanglitis obliterans, thrombotischer Krankheit assoziiert mit Heparin-induzierter Thrombozytopenie, thrombotischen Komplikationen assoziiert mit extrakorporaler Zirkulation, thrombotischen Komplikationen assoziiert mit Instrumentierung, thrombotischen Komplikationen assoziiert mit der Anbringung von prothetischen Vorrichtungen, okklusiver koronarer Thrombusbildung resultierend aus entweder thrombolytischer Therapie oder perkutaner transluminaler koronarer Angioplastie, Thrombusbildung in der venösen Vaskulatur, verbreiteter intravaskulärer Koagulopathie, einem Zustand, wobei es schnellen Verbrauch von Koagulationsfaktoren und systemischer Koagulation gibt, was in der Bildung von lebensbedrohlichen Thromben resultiert, die in der gesamten Mikrovaskulatur auftreten, was zu weitverbreitetem Organversagen führt, hämorrhagischem Schlaganfall, Nierendialyse, Sauerstoffanreicherung des Blutes und Herzkatheterisierung.

10. Zusammensetzung zur Verwendung nach Anspruch 6, wobei die Zustände und Symptome, die durch Thrombose verursacht werden, ausgewählt sind aus der Gruppe bestehend aus embolischem Schlaganfall, thrombotischem Schlaganfall, venöser Thrombose, tiefer venöser Thrombose, akutem Koronarsyndrom und Myokardinfarkt.

11. Zusammensetzung zur Verwendung nach Anspruch 6, wobei die Zusammensetzung mit einem oder mehreren der folgenden therapeutischen Mittel co-verabreicht wird: Heparin; tPA; Anistreplase; Streptokinase; Urokinase; einem Coumadin; Warfarin; Idraparinux; Fondaparinux; Aspirin; einem Adenosindiphosphat-Rezeptor-Hemmer; einem Phosphodiesterase-Hemmer; einem Glykoprotein-IIB/IIA-Hemmer; einem Adenosin-Wiederaufnahme-Hemmer; und einem Thromboxan-Rezeptor-Antagonisten.

12. Zusammensetzung nach einem der Ansprüche 1 bis 5 zur Verwendung in einem Verfahren zum Verhindern, Abschwächen oder Behandeln eines Subjekts (z. B. eines menschlichen Subjekts) mit einer Herz-Kreislauf-Erkrankung, umfassend Verabreichen einer therapeutisch wirksamen Menge einer solchen Zusammensetzung an das Subjekt.

13. Zusammensetzung nach einem der Ansprüche 1 bis 5 zur Verwendung in einem Verfahren zum Verhindern, Abschwächen oder Behandeln von erhöhter Thrombozytenaktivität bei einem Subjekt (z. B. einem menschlichen Subjekt) mit erhöhter Thrombozytenaktivität oder bei dem das Risiko besteht, eine solche aufzuweisen, oder zum Verhindern, Abschwächen oder Behandeln von Thrombozytenaggregation bei einem Subjekt (z. B. einem menschlichen Subjekt) mit Thrombozytenaggregation oder bei dem das Risiko besteht, eine solche aufzuweisen, oder zum Verhindern, Abschwächen oder Behandeln von Gefäßverschluss (z. B. arteriell und/oder venös) bei einem Subjekt (z. B. einem menschlichen Subjekt) mit Gefäßverschluss oder bei dem das Risiko besteht, einen solchen aufzuweisen, oder zum Verhindern, Abschwächen oder Behandeln von erhöhter Thrombozyten-assoziierter 12-LOX-Aktivität bei einem Subjekt (z. B. einem menschlichen Subjekt) mit erhöhter Thrombozyten-assoziierter 12-LOX-Aktivität oder bei dem das Risiko besteht, eine solche aufzuweisen, umfassend Verabreichen einer solchen Zusammensetzung an das Subjekt.

## Revendications

1. Composition comprenant une ou plusieurs nanoparticules synthétiques de HDL (sHDL) associées à des fractions de N-2-benzothiazolyl-4-[[(2-hydroxy-3-méthoxyphényl)méthyl]amino]-benzènesulfonamide (ML355) (sHDL-ML355) (par exemple, complexées, conjuguées, encapsulées, absorbées, adsorbées, mélangées), dans laquelle le sHDL-ML355 comprend un mélange d'au moins un composant lipidique, d'au moins un composant apolipoprotéine HDL et de ML355,
dans lequel le composant apolipoprotéine HDL est choisi dans le groupe constitué de l'apolipoprotéine A-I (apo A-I), de l'apolipoprotéine A-II (apo A-II), de l'apolipoprotéine A-II xxx (apo A-II-xxx), de l'apolipoprotéine A4 (apo A4), de l'apolipoprotéine Cs (apo Cs), de l'apolipoprotéine E (apo E), de l'apolipoprotéine A-I milano (apo A-I-milano), de l'apolipoprotéine A-I paris (apo A-I-paris), de l'apolipoprotéine M (apo M), d'un mimétique de l'apoIipoprotéine HDL, de la préproapoliprotéine, de la préproApoA-I, de la proApoA I, de la préproApoA-II, de la proApoA II, de la préproApoA-IV, de la proApoA-IV, de l'ApoA-V, de la préproApoE, de la proApoE, de la préproApoA I_{Milano}, de la proApoA-I_{Milano}, de la préproApoA-I_{Paris}, de la proApoA-I_{Paris}, et de mélanges de ceux-ci, et
dans lequel le mimétique ApoA-I est décrit par l'une quelconque des SEQ ID N° : 1-336 et WDRVKDLATVYVDVLKDSGRDYVSQF (SEQ ID N° : 337), LKLLDNWDSVTSTFSKLREOL (SEQ ID N° : 338), PVTOEFWDNLEKETEGLROEMS (SEQ ID N° : 339), KDLEEVKAKVQ (SEQ ID N° : 340), KDLEEVKAKVO (SEQ ID N° : 341), PYLDDFQKKWQEEMELYRQKVE (SEQ ID N° : 342), PLRAELQEGARQKLHELOEKLS (SEQ ID N° : 343), PLGEEMRDRARAHVDALRTHLA (SEQ ID N° : 344), PYSDELRQRLAARLEALKENGG (SEQ ID N° : 345), ARLAEYHAKATEHLSTLSEKAK (SEQ ID N° : 346), PALEDLROGLL (SEQ ID N° : 347), PVLESFKVSFLSALEEYTKKLN (SEQ ID N° : 348), PVLESFVSFLSALEEYTKKLN (SEQ ID N° : 349), PVLESFKVSFLSALEEYTKKLN (SEQ ID N° : 350), TVLLLTICSLEGALVRRQAKEPCV QTVTDYGKDLME (SEQ ID N° : 351), KVKSPELOAEAKSYFEKSKE (SEQ ID N° : 352), VLTLALVAVAGARAEVSADOVATV (SEQ ID N° : 353), NNAKEAVEHLOKSELTOOLNAL (SEQ ID N° : 354), LPVLVWLSIVLEGPAPAOGTPDVSS (SEQ ID N° : 355), LPVLVVVLSIVLEGPAPAQGTPDVSS (SEQ ID N° : 356), ALDKLKEFGNTLEDKARELIS (SEQ ID N° : 357), VVALLALLASARASEAEDASLL (SEQ ID N° : 358), HLRKLRKRLLRDADDLQKRLAVYOA (SEQ ID N° : 359), AQAWGERLRARMEEMGSRTRDR (SEQ ID N° : 360), LDEVKEQVAEVRAKLEEQAQ (SEQ ID N° : 361), DWLKAFYDKVAEKLKEAF (SEQ ID N° : 362), DWLKAFYDKVAEKLKEAFPDWAKAAYDKAAEKAKEAA SEQ ID N° : 363), PVLDLFRELLNELLEALKQKL (SEQ ID N° : 364), PVLDLFRELLNELLEALKQKLA (SEQ ID N° : 365), PVLDLFRELLNELLEALKQKLK (SEQ ID N° : 366), PVLDLFRELLNELLEALKQKLA (SEQ ID N° : 367), PVLDLFRELLNELLEALKKLLK (SEQ ID N° : 368), PVLDLFRELLNELLEALKKLLA (SEQ ID N° : 369) et PLLDLFRELLNELLEALKKLLA (SEQ ID N° : 370).

2. Composition de la revendication 1, dans laquelle le sHDL-ML355 est configuré pour une libération prolongée du ML355 sur une période de 24 heures.

3. Composition de la revendication 1, dans laquelle, lors de l'administration à un sujet (par exemple, un sujet humain), le sHDL-ML355 est capable d'inhiber l'agrégation plaquettaire, d'inhiber la formation de thrombose, d'inhiber l'occlusion vasculaire et/ou d'inhiber l'activité 12-LOX associée aux plaquettes.

4. Composition de la revendication 1, dans laquelle le rapport molaire du composant apolipoprotéine HDL au composant lipidique est d'environ 2:1 à 200:1, ou dans laquelle le composant lipidique comprend des phospholipides neutres, des phospholipides chargés négativement, des phospholipides chargés positivement, ou une combinaison de ceux-ci.

5. Composition de la revendication 1, dans laquelle le composant lipidique comprend une combinaison d'une ou de toute combinaison de sphingomyéline (SM), D-érythrose-sphingomyéline, D-érythrose dihydrosphingomyéline, palmitoylsphingomyéline, lysophospholipides, galactocérébroside, gangliosides, cérébrosides, glycérides, triglycérides, diglycérides, phospholipides à petites chaînes alkyles, phosphatidylcholine, phosphatidylcholine d'œuf, phosphatidylcholine de soja, dipalmitoylphosphatidylcholine (DPPC), dimyristoylphosphatidylcholine, 1-palmitoyl-2-oléoyl-phosphatidylcholine (POPC), 1,2-dimyristoyl-sn-glycéro-3-phosphatidylcholine (DMPC), 1,2- distéaroyl-sn-glycéro-3-phosphatidylcholine (DSPC), distéaroylphosphatidylcholine 1-myristoyl-2-palmitoylphosphatidylcholine, 1-palmitoyl-2-myristoylphosphatidylcholine, 1-palmitoyl-2-stéaroylphosphatidylcholine, 1-stéaroyl-2-palmitoylphosphatidylcholine, dioléoylphosphatidylcholine dioléophosphatidyléthanolamine, dilauroylphosphatidylglycérol phosphatidylcholine, phosphatidylsérine, phosphatidyléthanolamine, phosphatidylinositol, phosphatidylglycérols, diphosphatidylglycérols tels que dimyristoylphosphatidylglycérol, dipalmitoylphosphatidylglycérol, distéaroylphosphatidylglycérol, dioléoylphosphatidylglycérol, acide dimyristoylphosphatidique, acide dipalmitoylphosphatidique, dimyristoylphosphatidyléthanolamine, dipalmitoylphosphatidyléthanolamine, céramides, une phosphatidylsérine, dimyristoylphosphatidylsérine, dipalmitoylphosphatidylsérine, phosphatidylsérine cérébrale, sphingomyéline cérébrale, sphingomyéline d'œuf, sphingomyéline de lait, palmitoyl sphingomyéline, phytosphingomyéline, dipalmitoylsphingomyéline, distéaroylsphingomyéline, sel de dipalmitoylphosphatidylglycérol, acide phosphatidique, galactocérébroside, gangliosides, cérébrosides, dilaurylphosphatidylcholine, (1,3)-D-mannosyl-(1,3)diglycéride, aminophénylglycoside, 3-cholestéryl-6'-(glycosylthio)hexyl éther glycolipides, et cholesterol et ses dérivés, lyso-phosphotydylcholine, lyso-sphingomyéline, dioléoyl-sn-glycéro-3-phosphoéthanolamine-N-[3-(2-pyridyldithio) propionate] (DOPE-PDP), 1,2-dipalmitoyl-sn-glycéro-3-phosphothioéthanol, 1,2-di-(9Z-octadécénoyl)-*sn*-glycéro-3-phosphoéthanolamine-N-[4-(p-maléimidophényl)butyramide], 1,2-dihexadécanoyl-*sn*-glycéro-3-phosphoéthanolamine-N-[4-(p-maléimidophényl)butyramide], 1,2-dihexadécanoyl-*sn*-glycéro-3-phosphoéthanolamine-N-[4-(p-maléimidométhyl)cyclohexane-carboxamide], 1,2-di-(9Z-octadécénoyl)-*sn*-glycéro-3-phosphoéthanolamine-N-[4-(p-maléimidométhyl)cyclohexane-carboxamide], acide lysophoshphatidique, lysophosphatidylcholine, OA-NO₂ (acide oléique nitré acides 9- et 10-nitro-cis-octédécénoliques), LNO₂ (acide linoléique nitré acides 9-, 10-, 12- et 13-nitro-*cis-*octédécadiénoïques), AA-NO₂ (acide arachidonique nitré acides 5-, 6-, 8-, 9-, 11-, 12-, 14 et 15-nitro-*cis*-eicosatétraenoïques), CLNO₂ (linoléate de cholestéryl nitré cholestaryl-9-, 10-, 12- et 13-nitro-*cis*-octadécadiènes), acide gras, acides gras polyinsaturés oméga-3, acide hexadécatriénoïque (HTA ; 16:3 (n-3) ; acide tout-cis-7,10,13-hexadécatriénoïque), acide α-Linolénique (ALA ; 18:3 (n-3); acide tout-cis-9,12,15-octadécatriénoïque), acide stéaridonique (SDA ; 18:4 (n-3) ; acide tout-cis-6,9,12,15-octadécatétraénoïque), acide eicosatriénoïque (ETE ; 20:3 (n-3) ; acide tout-cis-11,14,17-eicosatriénoïque), acide eicosatétraénoïque (ETA ; 20:4 (n-3) ; acide tout-cis-8,11,14,17-eicosatétraénoïque), acide eicosapentaénoïque (EPA ; 20:5 (n-3) ; acide tout-cis-5,8,11,14,17-eicosapentaénoïque), acide hénéicosapentaénoïque (HPA ; 21:5 (n-3) ; acide tout-cis-6,9,12,15,18-hénéicosapentaénoïque) ; acide docosapentaénoïque (DPA ; acide clupanodonique ; 22:5 (n-3) ; acide tout-cis-7,10,13,16,19-docosapentaénoïque), acide docosahexaénoïque (DHA ; 22:6 (n-3) ; acide tout-cis-4,7,10,13,16,19-docosahexaénoïque), acide tétracosapentaénoïque ; 24:5 (n-3) ; acide tout-cis-9,12,15,18,21-tétracosapentaénoïque), acide tétracosahexaénoïque (acide nisinique ; 24:6 (n-3), acide tout-cis-6,9,12,15,18,21-tétracosahexaénoïque), analogues de sphingosine-1-phosphate, antagonistes de sphingosine-1-phosphate, agonistes de sphingosine-1-phosphate, agonistes de récepteurs de sphingosine-1-phosphate, antagonistes de récepteurs de sphingosine-1-phosphate et analogues de récepteurs de sphingosine-1- phosphate.

6. Composition selon l'une quelconque des revendications 1 à 5, destinée à être utilisée dans un procédé de prévention, d'atténuation ou de traitement de la thrombose chez un sujet (par exemple, un sujet humain) présentant ou risquant de présenter des états et des symptômes causés par la thrombose, comprenant l'administration au sujet d'une telle composition.

7. Composition destinée à être utilisée selon la revendication 6, dans laquelle l'administration de la composition entraîne, par exemple, la réduction de l'activité plaquettaire, la réduction de l'agrégation plaquettaire, la prévention de la formation de thrombus, la réduction de l'occlusion vasculaire et la réduction de l'activité 12-LOX associée aux plaquettes.

8. Composition destinée à être utilisée selon la revendication 6, dans laquelle les états et les symptômes causés par la thrombose sont liés à une thrombose veineuse et/ou à une thrombose artérielle.

9. Composition destinée à être utilisée selon la revendication 6, dans laquelle la thrombose est liée à un ou plusieurs des états suivants : syndrome coronarien aigu, infarctus du myocarde, angine instable, angine réfractaire, thrombus coronarien occlusif survenant après une thérapie thrombolytique ou après une angioplastie coronarienne, syndrome cérébrovasculaire à médiation thrombotique, accident vasculaire cérébral embolique, accident vasculaire cérébral thrombotique, accident vasculaire cérébral thromboembolique, embolie systémique, accident vasculaire ischémique, thromboembolie veineuse, fibrillation auriculaire, fibrillation auriculaire non valvulaire, flutter auriculaire, accidents ischémiques transitoires, thrombose veineuse, thrombose veineuse profonde, embolie pulmonaire, coagulopathie, coagulation intravasculaire disséminée, purpura thrombotique thrombocytopénique, thromboangéite oblitérante, maladie thrombotique associée à la thrombocytopénie induite par l'héparine, complications thrombotiques associées à la circulation extracorporelle, complications thrombotiques associées à l'instrumentation, complications thrombotiques associées à l'adaptation de prothèses, formation de thrombus coronaire occlusif résultant soit d'une thérapie thrombolytique, soit d'une angioplastie coronarienne transluminale percutanée, formation de thrombus dans le système vasculaire veineux, coagulopathie intravasculaire disséminée, état **caractérisé par** une consommation rapide de facteurs de coagulation et une coagulation systémique entraînant la formation de thrombus potentiellement mortels dans le système microvasculaire ce qui conduit à une défaillance généralisée des organes, à un accident vasculaire cérébral hémorragique, à une dialyse rénale, à une oxygénation du sang et à un cathétérisme cardiaque.

10. Composition destinée à être utilisée selon la revendication 6, dans laquelle les états et les symptômes causés par la thrombose sont choisis dans le groupe constitué de l'accident vasculaire cérébral embolique, l'accident vasculaire cérébral thrombotique, la thrombose veineuse, la thrombose veineuse profonde, le syndrome coronarien aigu et l'infarctus du myocarde.

11. Composition destinée à être utilisée selon la revendication 6, ladite composition étant co-administrée avec un ou plusieurs des agents thérapeutiques suivants : héparine ; tPA ; anistreplase ; streptokinase ; urokinase ; une coumadine ; warfarine ; idraparinux ; fondaparinux ; aspirine ; un inhibiteur du récepteur de l'adénosine diphosphate ; un inhibiteur de la phosphodiestérase ; un inhibiteur de la glycoprotéine IIB/IIA ; un inhibiteur de la recapture de l'adénosine ; et un antagoniste du récepteur du thromboxane.

12. Composition selon l'une quelconque des revendications 1 à 5, destinée à être utilisée dans un procédé de prévention, d'atténuation ou de traitement d'un sujet (par exemple, un sujet humain) présentant un trouble cardiovasculaire, comprenant l'administration au sujet d'une quantité thérapeutiquement efficace d'une telle composition.

13. Composition selon l'une quelconque des revendications 1 à 5, destinée à être utilisée dans un procédé de prévention, d'atténuation ou de traitement de l'activité plaquettaire accrue chez un sujet (par exemple, un sujet humain) présentant ou risquant de présenter une activité plaquettaire accrue, ou de prévention, d'atténuation ou de traitement de l'agrégation plaquettaire chez un sujet (par exemple, un sujet humain) présentant ou risquant de présenter une agrégation plaquettaire, ou de prévention, d'atténuation ou de traitement de l'occlusion vasculaire (par exemple, artérielle et/ou veineuse) chez un sujet (par exemple, un sujet humain) présentant ou risquant de présenter une occlusion vasculaire, ou de prévention, d'atténuation ou de traitement de l'activité 12-LOX associée aux plaquettes accrue chez un sujet (par exemple, un sujet humain) présentant ou risquant de présenter une activité 12-LOX associée aux plaquettes accrue, comprenant l'administration au sujet d'une telle composition.
